# EUROPEAN PATENT SPECIFICATION

(11) **EP 0 931 149 B1**
(45) Date of publication and mention of the grant of the patent: **05.12.2007**
(21) Application number: 97919143.4
(22) Date of filing: 11.09.1997
(51) Int. Cl.: C12N 15/19, C07K 14/715, A61K 38/17, C07K 16/18, A01K 67/027

(54) **A NOVEL HAEMOPOIETIN RECEPTOR AND GENETIC SEQUENCES ENCODING SAME**
HEMOPOTEIN-REZEPTOR UND DIESE KODIERENDE GENETISCHE SEQUENZEN
NOUVEAU RECEPTEUR D'HEMATOPOIETINE ET SEQUENCES GENETIQUES CODANT CE DERNIER

(30) Priority: 11.09.1996 AU PE224696
(43) Date of publication of application: 28.07.1999
(73) Proprietor: Amrad Operations Pty.,Ltd., Richmond, VIC 3121 (AU)
(72) Inventor: HILTON, Douglas, James, Warrandyte, VIC 3113 (AU); NICOLA, Nicos, Antony, Mont Albert, VIC 3127 (AU); FARLEY, Alison, North Fitzroy, VIC 3068 (AU); WILLSON, Tracy, North Balwyn, VIC 3104 (AU); ZHANG, Jian-Guo, Hoppers Crossing, VIC 3029 (AU); ALEXANDER, Warren, Moonee Ponds, VIC 3039 (AU); RAKAR, Steven, Avondale Heights, VIC 3034 (AU); FABRI, Louis, Mill Park, VIC 3082 (AU); KOJIMA, Tetsuo, Tokyo 144 (JP); MAEDA, Masatsugu, Tsukuba-shi, Ibaraki-ken 305 (JP); KIKUCHI, Yasufumi, Tsuchiura-shi, Ibaraki-ken 300 (JP); NASH, Andrew, Northcote, VIC 3070 (AU)
(74) Representative: Jones, Elizabeth Louise
(86) International application number: PCT/GB1997/002479
(87) International publication number: WO 1998/011225

(56) References cited:
- WO-A-96/07737
- WO-A-96/08510
- WO-A-97/12037
- WO-A-97/15663
- WO-A-97/25425
- DATABASE EMEST12 embl SEQ ID MM77631 Acc.No:W66776, 15 June 1996 "Mus musculus cDNA me17b11.r1 similar to PIR:B38252 granulocyte colony-stimulating factor receptor precursor" XP002055540 cited in the application & MARRA ET AL.: "The WahU-HHMI mouse EST project" .,
- ROBB ET AL.: "Structural analysis of the gene encoding the murine Interleukin-11 receptor alpha-chain and a related locus" JOURNAL OF BIOLOGICAL CHEMISTRY, vol. 271, no. 23, 7 June 1996, MD US, pages 13754-13761, XP002055539

## Description

The present invention relates generally to a novel haemopoietin receptor or derivatives thereof and to genetic sequences encoding same. Interaction between the novel receptor of the present invention and a ligand facilitates proliferation, differentiation and survival of a wide variety of cells. The novel receptor and its derivatives and the genetic sequences encoding same of the present invention are useful in the development of a wide range of agonists, antagonists, therapeutics and diagnostic reagents based on ligand interaction with its receptor.

Bibliographic details of the publications numerically referred to in this specification are collected at the end of the description. Sequence Identity Numbers (SEQ ID NOs.) for the nucleotide and amino acid sequences referred to in the specification are defined following the bibliography.

Throughout this specification and the claims which follow, unless the context requires otherwise, the word "comprise", or variations such as "comprises" or "comprising"; will be understood to imply the inclusion of a stated integer or group of integers but not the exclusion of any other integer or group of integers.

The rapidly increasing sophistication of recombinant DNA techniques is greatly facilitating research into the medical and allied health fields. Cytokine research is of particular importance, especially as these molecules regulate the proliferation, differentiation and function of a wide variety of cells. Administration of recombinant cytokines or regulating cytokine function and/or synthesis is becoming increasingly the focus of medical research into the treatment of a range of disease conditions.

Despite the discovery of a range of cytokines and other secreted regulators of cell function, comparatively few cytokines are directly used or targeted in therapeutic regimens. One reason for this is the pleiotropic nature of many cytokines. For example, interleukin (IL)-11 is a functionally pleiotropic molecule (1,2), initially characterized by its ability to stimulate proliferation of the IL-6-dependent plasmacytoma cell line, T11 65 (3). Other biological actions of IL-11 include induction of multipotential haemopoietin progenitor cell proliferation (4,5,6), enhancement of megakaryocyte and platelet formation (7,8,9,10), stimulation of acute phase protein synthesis (11) and inhibition of adipocyte lipoprotein lipase activity (12, 13).

Other important cytokines in the IL-11 group include IL-6, leukaemia inhibitory factor (LIF), oncostatin M (OSM) and CNTF. All these cytokines exhibit pleiotropic properties with significant activities in proliferation, differentiation and survival of cells. Members of the haemopoietin receptor family are defined by the presence of a conserved amino acid domain in their extracellular region. However, despite the low level of amino acid sequence conservation between other haemopoietin receptor domains of different receptors, they are all predicted to assume a similar tertiary structure, centred around two fibronectin-type III repeats (18,19).

The size of the haemopoietin receptor family has now become extensive and includes the cell surface receptors for may cytokines including interleukin-2 (IL-2), IL-3, IL-4, IL-5, IL-6, IL-7, IL-9, IL-11, IL-12, IL-13, IL-15, granulocyte colony stimulating factor (G-CSF), granulocyte-macrophage-CSF (GM-CSF), erythropoietin, thrombopoietin, leptin, leukaemia inhibitory factor, oncostatin-M, ciliary neurotrophic factor, cardiotrophin, growth hormone and prolactin. Although most of the members of the haemopoietin receptor family act as classic cell surface receptors, binding their cognate ligand at the cell surface and initiating intracellular signal transduction, some receptors are also produced in naturally occuuring soluble forms. These soluble receptors can either act as cytokine antagonists, by binding to cytokines and inhibiting productive interactions with cell surface receptors (eg LIF binding protein; (20) or as agonists, binding to cytokine and potentiating interaction with cell surface receptor components (eg soluble interleukin-6 receptor a-chain; (21). Still other members of the family appear to be produced only as secreted proteins, with no evidence of a cell surface form. In this regard, the IL-12 p40 subunit is a useful example. The cytokine IL-12 is secreted as a heterodimer composed of a p35 subunit which shows similarity to cytokines such as IL-6 (22) and a p40 subunit which shares similarity with the IL-6 receptor a-chain (23). In this case the soluble receptor acts as part of the cytokine itself and essential to formation of an active protein. In addition to acting as cytokines (eg IL-12p40), cytokine agonists (eg IL-6 receptor a-chain) or cytokine antagonists (LIF binding protein), members of the haemopoietin receptor have been useful in the discovery of small molecule cytokine mimetics. For example, the discovery of peptide mimetics of two commercially valuable cytokines, erythropoietin and thrombopoietin, centred on the selection of peptides capable of binding to soluble versions of the erythropoietin and thrombopoietin receptors (24,25). Due to the importance and multifactorial nature of these cytokines, there is a need to identify receptors, including both cell bound and soluble, for pleiotropic cytokines. Identification of such receptors permits the identification of pleiotropic cytokines and the development of a range of therapeutic and diagnostic agents.

Accordingly, the present invention relates to a nucleic acid molecule comprising a sequence of nucleotides encoding or complementary to a sequence encoding a novel haemopoietin receptor or a derivative thereof.

More particularly, the present invention concerns a nucleic acid molecule,comprising a sequence of nucleotides encoding or complementary to a sequence encoding a novel haemopoietin receptor or a derivative thereof having the motif:
Trp Ser Xaa Trp Ser [SEQ ID NO:1],
wherein Xaa is any amino acid and is preferably Asp or Glu.

Even more particularly, the present invention concerns a nucleic acid molecule comprising a sequence of nucleotides encoding or complementary to a sequence encoding a novel haemopoietin receptor or a derivative thereof, said receptor comprising the motif:
Trp Ser Xaa Trp Ser [SEQ ID NO;1]
wherein Xaa is any amino acid and is preferably Asp or Glu, said nucleic acid molecule is identifiable by hybridisation to said molecule under low stringency conditions at 42°C with
5' (A/G) CTCCA (A/G) TC (A/G) CTCCA 3' [SEQ ID NO:7] and
5' (A/G)CTCCA(C/T)TC(A/G)CTCCA 3' [SEQ ID NO:8].

Accordingly, the present invention provides an isolated nucleic acid molecule comprising a sequence of nucleotides as set forth in SEQ ID NO:12 or a nucleotide sequence having at least 60% similarity to the nucleotide sequence set forth in SEQ ID NO:12 or a nucleotide sequence capable of hybridising thereto under high stringency conditions at 42°C and wherein said nucleotide sequence encodes a novel haemopoietin receptor

In a related embodiment the present invention provides an isolated nucleic acid molecule comprising a sequence of nucleotides as set forth in SEQ ID NO:14 or a nucleotide sequence having at least 60% similarity to the nucleotide sequence set forth in SEQ ID NO:14 or a nucleotide sequence capable of hybridising thereto under high stringency conditions at 42°C and wherein said nucleotide sequence encodes a novel haemopoietin receptor.

In another related embodiment, the present invention provides an isolated nucleic acid molecule comprising a sequence of nucleotides as set forth in SEQ ID NO:16 or a nucleotide sequence having at least 60% similarity to the nucleotide sequence set forth in SEQ ID NO:16 or a nucleotide sequence capable of hybridising thereto under high stringency conditions at 42°C and wherein said nucleotide sequence encodes a novel haemopoietin receptor.

In a further related embodiment, the present invention provides an isolated nucleic acid molecule comprising a sequence of nucleotides as set forth in SEQ ID NO:18 or a nucleotide sequence having at least 60% similarity to the nucleotide sequence set forth in SEQ ID NO:18 or a nucleotide sequence capable of hybridising thereto under high stringency conditions at 42°C and wherein said nucleotide sequence encodes a novel haemopoietin receptor.

In yet a further related embodiment, the present invention provides an isolated nucleic acid molecule comprising a sequence of nucleotides as set forth in SEQ ID NO:24 or a nucleotide sequence having at least 60% similarity to the nucleotide sequence set forth in SEQ ID NO:24 or a nucleotide sequence capable of hybridising thereto under high stringency conditions at 42°C and wherein said nucleotide sequence encodes a novel haemopoietin receptor.

Still yet a further embodiment of the present invention is directed to a sequence of nucleotides as set forth in SEQ ID NO:28 or a nucleotide sequence having at least 60% similarity to the nucleotide sequence set forth in SEQ ID NO:28 or a nucleotide sequence capable of hybridising thereto under high stringency conditions at 42°C and wherein said nucleotide sequence encodes a novel haemopoietin receptor.

In still yet another embodiment, the present invention provides an isolated nucleic acid molecule comprising a sequence of nucleotides set forth in SEQ ID NO:38 or a nucleotide sequence having at least 60% similarity to the nucleotide sequence set forth in SEQ ID NO:38 or a nucleotide sequence capable of hybridising thereto under high stringency conditions at 42°C and wherein said nucleotide sequence encodes a novel haemopoietin receptor.

The term "receptor" is used in its broadest sense and includes any molecule capable of binding, associating or otherwise interacting with a ligand. Generally, the interaction will have a signalling effect although the present invention is not necessarily so limited. For example, the "receptor" may be in soluble form, often referred to as a cytokine binding protein. A receptor may be deemed a receptor notwithstanding that its ligand or ligands has or have not been identified.

Preferably, the novel receptor is derived from a mammal or a species of bird. Particularly, preferred mammals include humans, primates, laboratory test animals (e.g. mice, rats, rabbits, guinea pigs), livestock animals (e.g. sheep, horses, pigs, cows), companion animals (e.g. dogs, cats) or captive wild animals (e.g. deer, foxes, kangaroos). Although the present invention is exemplified with respect to mice, the scope of the subject invention extends to all animals and in particular humans.

The present invention is predicated in part on an ability to identify members of the haemopoietin receptor family with limited sequence similarity. Based on this approach, a genetic sequence has been identified in accordance with the present invention which encodes a novel receptor. The expressed genetic sequence is referred to herein as "NR6". Different forms of NR6 are referred to as, for example, NR6.1, NR6.2 and NR6.3.
The nucleotide and corresponding amino acid sequences for these molecules are represented in SEQ ID NOs:12, 14 and 16, respectively.

Preferred human and murine nucleic acid sequences for NR6 or its derivatives include sequences from brain, liver, kidney, neonatal, embryonic, cancer or tumour-derived tissues.

Reference herein to a low stringency at 42°C includes and encompasses from at least about 1% v/v to at least about 15% v/v formamide and from at least about 1M to at least about 2M salt for hybridisation, and at least about 1M to at least about 2M salt for washing conditions. Alternative stringency conditions may be applied where necessary, such as medium stringency, which includes and encompasses from at least about 16% v/v to at least about 30% v/v formamide and from at least about 0.5M to at least about 0.9M salt for hybridisation, and at least about 0.5M to at least about 0.9M salt for washing conditions, or high stringency, which includes and encompasses from at least about 31% v/v to at least about 50% v/v formamide and from at least about 0.01M to at least about 0.15M salt for hybridisation, and at least about 0.01M to at least about 0.15M salt for washing conditions.

The nucleic acid molecules contemplated by the present invention are generally in isolated form and are preferably cDNA or genomic DNA molecules. In a particularly preferred embodiment, the nucleic acid molecules are in vectors and most preferably expression vectors to enable expression in a suitable host cell. Particularly useful host cells include prokaryotic cells, mammalian cells, yeast cells and insect cells. The cells may also be in the form of a cell line.

Accordingly, another aspect of the present invention provides an expression vector comprising a nucleic acid molecule encoding the novel haempoietin receptor as hereinbefore described, said expression vector capable of expression in a selected host cell.

A method for cloning a nucleotide sequence encoding NR6 or a derivative thereof can be carried out, said method comprising searching a nucleotide data base for a sequence which encodes the amino acid sequence set forth in SEQ ID NO:1, designing one or more oligonucleotide primers based on the nucleotide sequence located in the search, screening a nucleic acid library with said one or more oligonucleotides and obtaining a clone therefrom which encodes said NR6 or part thereof.

Once a novel nucleotide sequence in obtained as indicated above encoding NR6, oligonucleotides may be designed which bind cDNA clones with high stringency. Direct colony hybridisation may be employed or PCR amplification may be used. The use of oligonucleotide primers which bind under conditions of high stringency ensures rapid cloning of a molecule encoding the novel NR6 and less time is required in screening out cloning artefacts. However, depending on the primers used, low or medium stringency conditions may also be employed.

Alternatively, a library may be screened directly such as using oligonucleotides set forth in SEQ ID NO:7 or SEQ ID NO:8 or a mixture of both oligonucleotides may be used. In addition, one or more of oligonucleotides defined in SEQ ID NO:2 to 11 may also be used.

Preferably, the nucleic acid library is a cDNA, genomic, cDNA expression or mRNA library.

Preferably, the nucleic acid library is a cDNA expression library.

Preferably, the nucleotide data base is of human or murine origin and of brain, liver, kidney, neo-natal tissue, embryonic tissue, tumour or cancer tissue origin.

Preferred percentage similarities to the reference nucleotide sequences include at least about 70%, more preferably at least about 80%, still more preferably at least about 90% and even more preferably at least about 95% or above.

Another aspect of the present invention provides an isolated nucleic acid molecule comprising or having at least about 50% similarity to SEQ ID NO:13 or a fragment of a sequence of nucleotides encoding a novel haemopoietin receptor having an amino acid sequence as set forth in SEQ ID NO:13 encoding a haemopoietin receptor.

Still yet another aspect of the present invention provides an isolated nucleic acid molecule comprising a or having at least about 50% similarity to SEQ ID NO:15 or a fragment of sequence of nucleotides encoding a novel haemopoietin receptor having an amino acid sequence as set forth in SEQ ID NO:15 encoding a haemopoietin receptor.

Even yet another aspect of the present invention provides an isolated nucleic acid molecule comprising or having at least about 50% similarity to SEQ ID NO:17, or a fragment of a sequence of nucleotides encoding a novel haemopoietin receptor or derivative thereof having an amino acid sequence as set forth in SEQ ID NO:17 encoding a haemopoietin receptor.

A further aspect of the present invention provides an isolated nucleic acid molecule comprising or having at least about 50% similarity to SEQ ID NO:19, or a fragment of a sequence of nucleotides encoding a novel haemopoietin receptor or derivative thereof having an amino acid sequence as set forth in SEQ ID NO:19 encoding a haemopoietin receptor.

Even yet a another aspect of the present invention provides an isolated nucleic acid molecule comprising or having at least about 50% similarity to SEQ ID NO:25, or a fragment of a sequence of nucleotides encoding a novel haemopoietin receptor or derivative thereof having an amino acid sequence as set forth in SEQ ID NO:25 encoding a haemopoietin receptor.

Another aspect of the present invention provides an isolated nucleic acid molecule comprising or having at least about 50% similarity to SEQ ID NO.29 or a fragment of a sequence of nucleotides encoding a novel haemopoietin receptor or derivative thereof having an amino acid sequence as set forth in one or more of SEQ ID NOs:29 encoding a haemopoietin receptor.

Preferably, the percentage amino acid similarity is at least about 60%, more preferably at least about 70%, even more preferably at least about 80-85% and still even more preferably at least about 90-95% or greater.

Derivatives of the NR6 polypeptide which are components, parts, fragments, homologues or analogues of the novel haemopoietin receptors are preferably encoded by all or part of a nucleotide sequences substantially set forth in SEQ ID NO:12 or 14 or 16 or 18 or 25 or 20 or 24 or 28 or 38 or a molecule having at least about 60% nucleotide similarity to all or part thereof or a molecule capable of hybridising to the nucleotide sequence set forth in SEQ ID NO:12 or 14 or 16 or 18 or 20 or 24 or 28 or 38 or a complementary form thereof. The NR6 molecule may be glycosylated or non-glycosylated. When in glycosylated form, the glycosylation may be substantially the same as naturally occurring haemopoietin receptor or may be a modified form of glycosylation. Altered or differential glycosylation states may or may not affect binding activity of the novel receptor.

The NR6 haemopoietin receptor may be in soluble form or may be expressed on a cell surface or conjugated or fused to a solid support or another molecule.

Derivatives of NR6 include fragments, parts, portions, mutants, homologues and analogues of the NR6 polypeptide and corresponding genetic sequence. Derivatives also include single or multiple amino acid substitutions, deletions and/or additions to NR6 or single or multiple nucleotide substitutions, deletions and/or additions to the genetic sequence encoding NR6. "Additions" to amino acid sequences or nucleotide sequences include fusions with other peptides, polypeptides or proteins or fusions to nucleotide sequences. Derivatives of NR6 include functional derivatives or NR6 immunologically interactive derivatives.

Analogues of NR6 include, but are not limited to, modification to side chains, incorporating of unnatural amino acids and/or their derivatives during peptide, polypeptide or protein synthesis and the use of crosslinkers and other methods which impose conformational constraints on the proteinaceous molecule or their analogues.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS) ; acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation via O-acylisourea formation followed by subsequent derivitisation, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 1.

These types of modifications may be important to stabilise NR6 if administered to an individual or for use as a diagnostic reagent.

Crosslinkers can be used, for example, to stabilise 3D conformations, using homo-bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of Cα and N α-methylamino acids, introduction of double bonds between Cα and Cβ atoms of amino acids and the formation of cyclic peptides or analogues by introducing covalent bonds such as forming an amide bond between the N and C termini, between two side chains or between a side chain and the N or C terminus.

**TABLE 1**

| Non-conventional amino acid | Code | Non-conventional amino acid | Code |
|---|---|---|---|
| aminobutyric acid | Abu | L-N-methylalanine | Nmala |
| Amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | | ChexaL-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| Dα-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| y-aminobutyric acid | Gabu | N-(p-hydroxyphenyl)glycine | Nhtyr |
| L-t-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-t-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Morn |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) carbamylmethyl) glycine | Nnbhm | N-(N-(3,3-dilphenylpropyl) carbamylmethyl)glycine | Nnbhe |
| 1-carboxy-1-(2,2-diphenyl-ethylamino)cyclopropane | Nmbc | | |

Chemical analogues of NR6 may be capable of acting as antagonists or agonists of NR6 or which can act as functional analogues of NR6. Chemical analogues may not necessarily be derived from NR6 but may share certain conformational similarities. Alternatively, chemical analogues may be specifically designed to mimic certain physiochemical properties of NR6. Chemical analogues may be chemically synthesised or may be detected following, for example, natural product screening.

The identification of NR6 permits the generation of a range of therapeutic molecules capable of modulating expression of NR6 or modulating the activity of NR6. Modulators include agonists and antagonists of NR6 expression.

Antagonists of NR6 expression include antisense molecules, ribozymes and co-suppression molecules. Agonists include molecules which increase promoter ability or interfere with negative regulatory mechanisms. Agonists of NR6 include molecules which overcome any negative regulatory mechanism. Antagonists of NR6 include antibodies and inhibitor peptide fragments.

Other derivatives include a range of glycosylation variants from a completely unglycosylated molecule to a modified glycosylated molecule. Altered glycosylation patterns may result from expression of recombinant molecules in different host cells.

A method for modulating expression of NR6 in a subject such as a human or mouse can be developed, said method comprising contacting the genetic sequence encoding NR6 with an effective amount of a modulator of NR6 expression for a time and under conditions sufficient to up-regulate or down-regulate or otherwise modulate expression of NR6. Modulating NR6 expression provides a means of modulating NR6-ligand interaction or NR6 stimulation of cell activities.

A method of modulating activity of NR6 in a human can be developed, said method comprising administering to said mammal a modulating effective amount of a molecule for a time and under conditions sufficient to increase or decrease NR6 activity. The molecule may be a proteinaceous molecule or a chemical entity and may also be a derivative of NR6 or its ligand or a chemical analogue or truncation mutant of NR6 or its ligand.

The present invention, therefore, contemplates a pharmaceutical composition comprising NR6 of the invention and one or more pharmaceutically acceptable carriers and/or diluents. These components are referred to as the "active ingredients".

The pharmaceutical forms suitable for injectable use include sterile aqueous solutions (where water soluble) and sterile powders for the extemporaneous preparation of sterile injectable solutions. It must be stable under the conditions of manufacture and storage and must be preserved against the contaminating action of microorganisms such as bacteria and fungi. The carrier can be a solvent or dilution medium comprising, for example, water, ethanol, polyol (for example, glycerol, propylene glycol and liquid polyethylene glycol, and the like), suitable mixtures thereof, and vegetable oils. The proper fluidity can be maintained, for example, by the use of superfactants. The preventions of the action of microorganisms can be brought about by various antibacterial and antifungal agents, for example, parabens, chlorobutanol, phenol, sorbic acid, thirmerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients are suitably protected they may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like.
Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations according to the present invention are prepared so that an oral dosage unit form contains between about 0.1 ug and 2000 mg of active compound. Alternative dosage amounts include from about 1 µg to about 1000 mg and from about 10 µg to about 500 mg.

The tablets, troches, pills, capsules and the like may also contain the components as listed hereafter: A binder such as gum, acacia, corn starch or gelatin; excipients such as dicalcium phosphate; a disintegrating agent such as corn starch, potato starch, alginic acid and the like; a lubricant such as magnesium stearate; and a sweetening agent such a sucrose, lactose or saccharin may be added or a flavouring agent such as peppermint, oil of wintergreen, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

The present invention also extends to forms suitable for topical application such as creams, lotions and gels as well as a range of "paints" which are applied to skin and through which the active ingredients are absorbed.

Pharmaceutically acceptable carriers and/or diluents include any and all solvents, dispersion media, coatings, antibacterial and antifungal agents, isotonic and absorption delaying agents and the like. The use of such media and agents for pharmaceutical active substances is well known in the art and except insofar as any conventional media or agent is incompatible with the active ingredient, their use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed.
A unit dosage form can, for example, contain the principal active compound in amounts ranging from 0.5 µg to about 2000 mg. Expressed in proportions, the active compound is generally present in from about 0.5 µg to about 2000 mg/ml of carrier. In the case of compositions containing supplementary active ingredients, the dosages are determined by reference to the usual dose and manner of administration of the said ingredients.

Dosages may also be expressed per body weight of the recipient. For example, from about 10 ng to about 1000 mg/kg body weight, from about 100 ng to about 500 mg/kg body weight and for about 1 *µ*g to above 250 mg/kg body weight may be administered.

The pharmaceutical composition may also comprise genetic molecules such as a vector capable of transfecting target cells where the vector carries a nucleic acid molecule capable of modulating NR6 expression or NR6 activity. The vector may, for example, be a viral vector.

Still another aspect of the present invention is directed to antibodies to NR6 of the invention. Such antibodies may be monoclonal or polyclonal and may be selected from naturally occurring antibodies to NR6 or may be specifically raised to NR6 In the case of the latter, NR6 may first need to be associated with a carrier molecule. The antibodies and/or recombinant NR6 of the present invention are particularly useful as therapeutic or diagnostic agents. For example, NR6 antibodies or antibodies to its ligand may act as antagonists.

For example, NR6 and its derivatives can be used to screen for naturally occurring antibodies to NR6. These may occur, for example in some autoimmune diseases. Alternatively, specific antibodies can be used to screen for NR6. Techniques for such assays are well known in the art and include, for example, sandwich assays and ELISA. Knowledge of NR6 levels may be important for diagnosis of certain cancers or a predisposition to cancers or for monitoring certain therapeutic protocols.

Antibodies to NR6 of the present invention may be monoclonal or polyclonal. Alternatively, fragments of antibodies may be used such as Fab fragments. Furthermore, the present invention extends to recombinant and synthetic antibodies and to antibody hybrids. A "synthetic antibody" is considered herein to include fragments and hybrids of antibodies. The antibodies of this aspect of the present invention are particularly useful for immunotherapy and may also be used as a diagnostic tool for assessing apoptosis or monitoring the program of a therapeutic regimen.

For example, specific antibodies can be used to screen for NR6 proteins. The latter would be important, for example, as a means for screening for levels of NR6 in a cell extract or other biological fluid or purifying NR6 made by recombinant means from culture supernatant fluid. Techniques for the assays contemplated herein are known in the art and include, for example, sandwich assays and ELISA.

It is within the scope of this invention to include any second antibodies (monoclonal, polyclonal or fragments of antibodies or synthetic antibodies) directed to the first mentioned antibodies discussed above. Both the first and second antibodies may be used in detection assays or a first antibody may be used with a commercially available anti-immunoglobulin antibody. An antibody as contemplated herein includes any antibody specific to any region of NR6.

Both polyclonal and monoclonal antibodies are obtainable by immunization with the enzyme or protein and either type is utilizable for immunoassays. The methods of obtaining both types of sera are well known in the art. Polyclonal sera are less preferred but are relatively easily prepared by injection of a suitable laboratory animal with an effective amount of NR6, or antigenic parts thereof, collecting serum from the animal, and isolating specific sera by any of the known immunoadsorbent techniques. Although antibodies produced by this method are utilizable in virtually any type of immunoassay, they are generally less favoured because of the potential heterogeneity of the product.

The use of monoclonal antibodies in an immunoassay is particularly preferred because of the ability to produce them in large quantities and the homogeneity of the product. The preparation of hybridoma cell lines for monoclonal antibody production derived by fusing an immortal cell line and lymphocytes sensitized against the immunogenic preparation can be done by techniques which are well known to those who are skilled in the art.

Another aspect of the present invention contemplates a method for detecting NR6 in a biological sample from a subj ect said method comprising contacting said biological sample with an antibody specific for NR6 of the invention for a time and under conditions sufficient for an antibody-NR6 complex to form, and then detecting said complex.
The presence of NR6 may be accomplished in a number of ways such as by Western blotting and ELISA procedures.
A wide range of immunoassay techniques are available as can be seen by reference to US Patent Nos. 4,016,043, 4, 424,279 and 4,018,653. These, of course, includes both single-site and two-site or "sandwich" assays of the non-competitive types, as well as in the traditional competitive binding assays. These assays also include direct binding of a labelled antibody to a target.

Sandwich assays are among the most useful and commonly used assays and are favoured for use in the present invention. A number of variations of the sandwich assay technique exist, and all are intended to be encompassed by the present invention. Briefly, in a typical forward assay, an unlabelled antibody is immobilized on a solid substrate and the sample to be tested brought into contact with the bound molecule. After a suitable period of incubation, for a period of time sufficient to allow formation of an antibody; antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of hapten. Variations on the forward assay include a simultaneous assay, in which both sample and labelled antibody are added simultaneously to the bound antibody. These techniques are well known to those skilled in the art, including any minor variations as will be readily apparent. In accordance with the present invention, the sample is one which might contain NR6 including cell extract, tissue biopsy or possibly serum, saliva, mucosal secretions, lymph, tissue fluid and respiratory fluid. The sample is, therefore, generally a biological sample comprising biological fluid but also extends to fermentation fluid and supernatant fluid such as from a cell culture.

In the typical forward sandwich assay, a first antibody having specificity for the NR6 or antigenic parts thereof, is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. from about room temperature to about 37°C) to allow binding of any subunit present in the antibody. Following the incubation period, the antibody subunit solid phase is washed and dried and incubated with a second antibody specific for a portion of the hapten. The second antibody is linked to a reporter molecule which is used to indicate the binding of the second antibody to the hapten.

An alternative method involves immobilizing the target molecules in the biological sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.

In another alternative method, the NR6 ligand is immobilised to a solid support and a biological sample containing NR6 brought into contact with its immobilised ligand. Binding between NR5 and its ligand can then be determined using an antibody to NR6 which itself may be labelled with a reporter molecule or a further anti-immunoglobulin antibody labelled with a reporter molecule could be used to detect antibody bound to NR6.

By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.
In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable colour change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of hapten which was present in the sample. "Reporter molecule" also extends to use of cell agglutination or inhibition of agglutination such as red blood cells on latex beads, and the like.

Alternately, fluorescent compounds, such as fluorescein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic colour visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength the fluorescence observed indicates the presence of the hapten of interest. Immunofluorescene and EIA techniques are both very well established in the art and are particularly preferred for the present method. However, other reporter molecules, such as radioisotope, chemiluminescent or bioluminescent molecules, may also be employed.

Genetic assays can be performed such as involving PCR analysis to detect the NR6 gene or its derivatives. Alternative methods or methods used in conjunction include direct nucleotide sequencing or mutation scanning such as single stranded conformational polymorphisms analysis (SSCP) as specific oligonucleotide hybridisation, as methods such as direct protein truncation tests.

The nucleic acid molecules of the present invention may be DNA or RNA. When the nucleic acid molecule is in a DNA form, it may be genomic DNA or cDNA. RNA forms of the nucleic acid molecules of the present invention are generally mRNA.

Although the nucleic acid molecules of the present invention are generally in isolated form, they may be integrated into or ligated to or otherwise fused or associated with other genetic molecules such as vector molecules and in particular expression vector molecules. Vectors and expression vectors are generally capable of replication and, if applicable, expression in one or both of a prokaryotic cell or a eukaryotic cell. Preferably, prokaryotic cells include *E. coli,* Bacillus sp and Pseudomonas *sp.* Preferred eukaryotic cells include yeast, fungal, mammalian and insert cells.

Accordingly, another aspect of the present invention contemplates a genetic construct comprising a vector portion and a mammalian and more particularly a human NR6 gene portion, which NR6 gene portion is capable of encoding an NR6 polypeptide or a functional or immunologically interactive derivative thereof.

Preferably, the NR6 gene portion of the genetic construct is operably linked to a promoter on the vector such that said promoter is capable of directing expression of said NR6 gene portion in an appropriate cell.

In addition, the NR6 gene portion of the genetic construct may comprise all or part of the gene fused to another genetic sequence such as a nucleotide sequence encoding maltose binding protein or glutathione-S-transferase or part thereof.

The present invention extends to such genetic constructs and to prokaryotic or eukaryotic cells comprising same.

Derivatives of NR6 including mutants, part, fragments, portions, homologues and analogues or their encoding genetic sequence including single or multiple nucleotide or amino acid substitutions, additions and/or deletions to the naturally occurring nucleotide or amino acid sequence are also described.

NR6 may be important for the proliferation, differentiation and survival of a diverse array of cell types. Accordingly, it is proposed that NR6 or its functional derivatives be used to regulate development, maintenance or regeneration in an array of different cells and tissues *in vitro* and *in vivo.* For example, NR6 is contemplated to be useful in modulating neuronal proliferation, differentation and survival.

Soluble NR6 polypeptides are also contemplated to be useful in the treatment of a range of diseases, injuries or abnormalities.

Membrane bound or soluble NR6 may be used in vitro on nerve cells or tissues to modulate proliferation, differentiation or survival, for example, in grafting procedures or transplantation.

As stated above, the NR6 of the present invention or its functional derivatives may be provided in a pharmaceutical composition comprising the NR6 together with one or more pharmaceutically acceptable carriers and/or diluents. In addition, the present invention contemplates a method of treatment comprising the administration of an effective amount of a NR6 of the present invention. The present invention also extends to antagonists and agonists of NR6s and their use in therapeutic compositions and methodologies.

A further aspect of the present invention contemplates the use of NR6 of the invention in the manufacture of a medicament for the treatment of NR6 mediated conditions defective or deficient. A ligand for NR6 is described preferably, in isolated or recombinant form or a derivative of said ligand.

Knockout non-human animals such as mice or other murine species for the NR6 gene including homozygous and heterozygous knockout animals can be produced. Such animals provide a particularly useful live *in vivo* model for studying the effects of NR6 as well as screening for agents capable of acting as agonists or antagonists of NR6.

A non-human transgenic animal comprising a mutation in at least one allele of the gene encoding NR6 can be produced. Additionally, a (non human) transgenic animal comprising a mutation in two alleles of the gene encoding NR6 can be produced. The transgenic animal may be a murine animal such as a mousse or rat.

The present invention is further described by the following non-limiting Figures and Examples.

In the Figures:
**Figure 1** is a diagrammatic representation showing expansion of sequenced region of the mouse NR6 gene indicating splicing patterns seen in the three forms of NR6 cDNA, NR6.1, NR6.2 and NR6.3.
**Figure 2** is a representation of the nucleotide sequence of the mouse NR6 gene, containing exons encoding the cDNA from nucleotide 148 encoding D50 of the cDNAs shown in SEQ ID NOs:12 and 14 to the end of the 3' untranslated region shared by both NR6.1, NR6.2 and NR6.3. In this figure, this region encompasses nucleotides g1182 to g6617. This sequence is also defined in SEQ ID NO:28.
**Figure 3** is a representation of the nucleotide sequence of the mouse genomic NR6 gene with additional 5' sequences. The coding exons of NR6 span approximately 11kb of the mouse genome. There are 9 coding exons separated by 8 introns:

| | | |
|---|---|---|
| exon1 | at least 239nt | intron1 5195nt |
| exon 2 | 282nt | intron2 214nt |
| exon3 | 130nt | Intron3 107nt |
| exon4 | 170nt | intron4 1372nt |
| exon5 | 158nt | intron5 68nt |
| exon6 | 169nt | intron6 2020nt |
| exon6 | 188nt | intron7 104nt |
| exon8 | 43nt | intron8 181nt |
| exon9 | 252nt | |

Exon 1 encoding the signal sequence, exon 2 the Ig-like domain, exons 3 to 6 the hemopoietin domain. Exons 7, 8 and 9 are alternatively spliced.

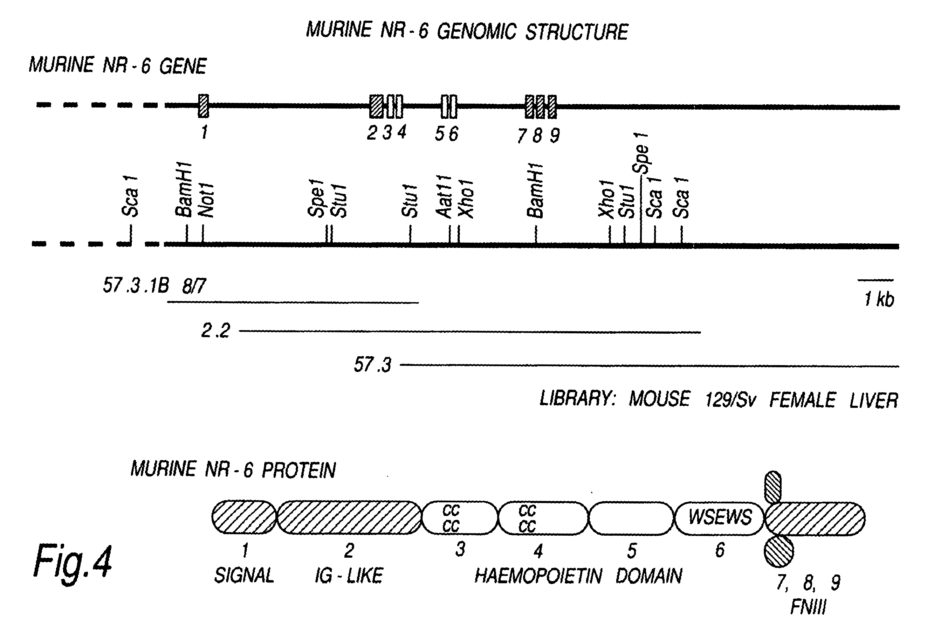

**Figure 4** is a diagrammatic representation showing the genomic structure of murine NR-6.

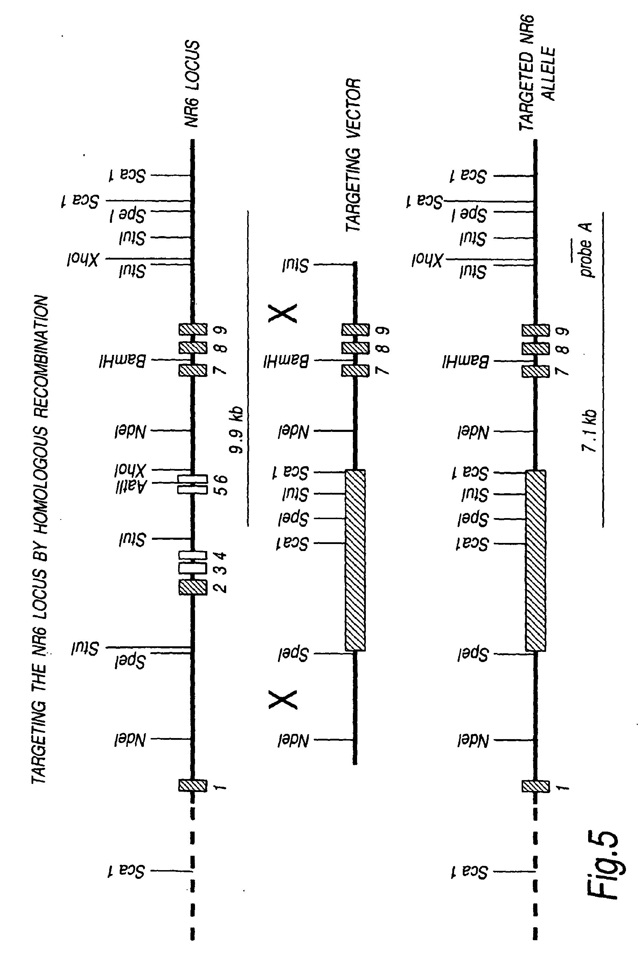

**Figure 5** is a diagrammatic representation showing targetting of the NR6 locus by homologous recombination.

Single and three letter abbreviations for amino acid residues used in the specification are summarised in Table 2:

**TABLE 2**

| Amino Acid | Three-letter Abbreviation | One-letter Symbol |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any residue | Xaa | X |

**TABLE 3**

| **SUMMARY OF SEQ ID NO.** | |
|---|---|
| Sequence | SEQ ID NO. |
| Amino acid sequence WSXWS | 1 |
| Oligonucleotide primers and probes listed in Example 1 | 2-11 |
| Nucleotide sequence of NR6.1¹ | 12 |
| Amino acid sequence of NR6.1 | 13 |
| Nucleotide sequence of NR6.2² | 14 |
| Amino acid sequence of NR6.2 | 15 |
| Nucleotide sequence of NR6.3³ | 16 |
| Amino acid sequence of NR6.3 | 17 |
| Nucleotide sequence of products generated by 5' RACE of brain cDNA using NR6 specific primers⁴ | 18 |
| Amino acid sequence of SEQ ID NO:18 | 19 |
| Nucleotide sequence unique to 5' RACE of brain cDNA | 20 |
| Amino acid sequence for SEQ ID NO:20 | 21 |
| Unspliced murine NR6 nucleotide sequence | 22 |
| PCR product for human NR6 | 23 |
| Nucleotide sequence of clone HFK-66 encoding human NR6 | 24 |
| Amino acid sequence of SEQ ID NO:24 | 25 |
| Oligonucleotide sequences UP1 and LP1, respectively | 26-27 |
| Genomic nucleotide sequence of murine NR6 | 28 |
| Amino acid sequence of SEQ ID NO:28 | 29 |
| Murine NR6.1 oligonucleotide primers | 30, 31 |
| Murine IL-3 signal sequence | 32 |
| Linker sequence for mouse IL-3 signal sequence and FLAG epitope | 33-35 |
| Genomic nucleotide sequence of murine NR6 containing additonal 5' sequence | 38 |
| Oligonucleotide 2199 and 2200, respectively | 36, 37 |
| N-terminal region of NR6 | 39 |

| | |
|---|---|
| ¹The polyadenylation signal AATAAATAAA is at nucleotide position 1451 to 1460; NR6.1 (SEQ ID NO:12) and NR6.2 (SEQ ID NO:14) are identical to nucleotide 1223 encoding Q407, the represents the end of an exon. NR6.1 splices out an exon present only in NR6.2 and uses a different reading frame for the final exon which is shared with NR6.2; this corresponds to amino acids VLPAKL at amino acid residue positions 403-413. The region of 3'-untranslated DNA shared by NR6.1, NR6.2 and NR6.3 is from nucleotide 1240 to 1475. The WSXWS motif is at amino acid residues 330 to 334. ²The polyadenylation signal AATAAA is at nucleotide positions 1494 to 1503. The WSXWS motif is at amino acid residues 330 to 334. NR6.1 and NR6.2 are identical to nucleotide 1223 encoding Q407 which represents the end of an exon. NR6.2 splices in an exon beginning at amino acid residue D408, nucleotide 1224 and ends at residue G422, nucleotide 1264. The region of 3' untranslated DNA shared by NR6.1, NR6.2 and NR6.3 is from nucleotide position 1283 to 1517. ³The nucleotide and amino acid numbering corresponds to SEQ ID NO:12 and 14. The WSXWS motif is at amino acid residues 330 to 334. The polyadenylation signal AATAAATAAA is from nucleotide 1781 to 1780. NR6.1, NR6.2 and NR6.3 are identical to nucleotide 1223 encoding Q407, this represents the end of an exon. NR6.3 fails to splice from this position and, therefore, translation continues through the intron, giving rise to the C-terminal protein region from amino acid residues 408 to 461. The region of 3' untranslated DNA shared by NR6.1, NR6.2 and NR6.3 is from nucleotide 1469 to 1804. ⁴The nucleotide sequence is identical to NR6.1, NR6.2 and NR6.3 from nucleotide C151, the first nucleotide for Pro51. The numbering from this nucleotide is the same as for SEQ ID NO:14 and 16. The 5' of this point is unique to the products generated by 5' RACE not being found in NR6.1, NR6.2 and NR6.3 and is represented in SEQ ID NOs:20 and 21. ⁵Structure of the murine genomic NR6 locus. The coding exons of NR6 span approximately 11kb of the mouse genome. There are 9 coding exons separated by 3 introns: exon 1 at least 239nt intron1 5195nt exon 2 282nt intron2 214nt exon 3 130nt intron3 107nt exon 4 170nt intron 4 1372nt exon 5 158nt intron5 68nt exon 6 169nt intron6 2020nt exon 7 188nt intron7 104nt exon 8 43nt intron8 181nt exon 9 252nt | |

Exon 1 encodes the signal sequence, exon 2 the Ig-like domain, exons 3 to 6 the hemopoietin domain. Exons 7, 8 and 9 are alternatively spliced.

The NRG molecules of the present invention have a range of utilities referred to in the subject specification. Additional utilities include:

### 1. Identification of molecules that interact with NR6.

These may include :
a) a corresponding ligand using standard orphan receptor techniques (26),
b) monoclonal antibodies that act either as receptors antagonists or agonists,
c) mimetic or antagonistic peptides isolated using phage display technology (27,28),
d) small molecule natural products that act either as antagonists or agonists.

### 2. Development of diagnostics to detect deletions/rearrangements in the NR6 gene.

The NR6 knock-out mice studies described herein provide a useful model for this utility. There are also applications in the field of reproduction. For example, people can be tested for their NR6 status. NR6 +/- carriers might be expected to give rise to offspring with developmental problems.

### EXAMPLE 1

### Oligonucleotides

| | |
|---|---|
| M116: | 5' ACTCGCTCCAGATTCCCGCCTTTT 3' [SEQ ID NO:2] |
| M108: | 5' TCCCGCCTTTTTCGACCCATAGAT 3' [SEQ ID NO:3] |
| M159: | 5' GGTACTTGGCTTGGAAGAGGAAAT 3' [SEQ ID NO:4] |
| M242: | 5' CGGCTCACGTGCACGTCGGGTGGG 3' [SEQ ID NO:5] |
| M112: | 5' AGCTGCTGTTAAAGGGCTTCTC 3' [SEQ ID NO:6] |
| WSDWS | 5' (A/G)CTCCA(A/G)TC(A/G)CTCCA 3' [SEQ ID NO:7] |
| WSEWS | 5' (A/G)CTCCA(C/T)TC(A/G)CTCCA 3' [SEQ ID NO:8] |
| 1944 | 5' AAGTGTGACCATCATGTGGAC 3' [SEQ ID NO:9] |
| 2106 | 5' GGAGGTGTTAAGGAGGCG 3' [SEQ ID NO:10] |
| 2120 | 5' ATGCCCGCGGGTCGCCCG 3' [SEQ ID NO:11] |

### EXAMPLE 2

### Isolation of initial NR6 cDNA clones using oligonucleotides designed against the conserved WSXWS motif found in members of the haemopoietin receptor family

(i) A commercial adult mouse testis cDNA library cloned into the UNI-ZAP bacteriophage (Stratagene, CA, USA; Catalogue numbers 937 308) was used to infect Escherichia coli of the strain LE392. Infected bacteria were grown on twenty 150 mm agar plates, to give approximately 50,000 plaques per plate. Plaques were then transferred to duplicate 150 mm diameter nylon membranes (Colony/Plaque Screen, NEN Research Products, MA, USA), bacteria were lysed and the DNA was denatured and fixed by autoclaving at 100°C for 1 min with dry exhaust. The filters were rinsed twice in 0.1%(w/v) sodium dodecyl sulfate (SDS), 0.1 x SSC (SSC is 150 mM sodium chloride, 15 mM sodium citrate dihydrate) at room temperature and pre-hybridized overnight at 42°C in 6 x SSC containing 2 mg/ml bovine serum albumin, 2 mg/ml Ficoll, 2 mg/ml polyvinylpyrrolidone, 100 mM ATP, 10 mg/ml tRNA, 2 mM sodium pyrophosphate, 2 mg/ml salmon sperm DNA, 0.1% (w/v) SDS and 200 mg/ml sodium azide. The pre-hybridisation buffer was removed. 1.2 µg of the degenerate oligonucleotides for hybridization (WSDWS; Example 1) were phosphorylated with T4 polynucleotide kinase using 960 mCi of Y³²P-ATP (Bresatec, S.A., Australia). Unincorporated ATP was separated from the labelled oligonucleotide using a pre-packed gel filtration column (NAP-5; Pharmacia, Uppsala, Sweden). Filters were hybridized overnight at 42°C in 80 ml of the prehybridisation buffer containing 0.1%(w/v) SDS, rather than NP40, and 10⁶ - 10⁷ cpm/ml of labelled oligonucleotide. Filters were briefly rinsed twice at room temperature in 6 x SSC, 0.1%(v/v) SDS, twice for 30 min at 45°C in a shaking waterbath containing 1.5 1 of the same buffer and then briefly in 6 x SSC at room temperature. Filters were then blotted dry and exposed to autoradiographic film at -70°C using intensifying screens, for 7 - 14 days prior to development. Plaques that appeared positive on orientated duplicate filters were picked, eluted in 1 ml of 100 mM NaCl, 10 mM MgCl₂, 10 mM Tris.HCl pH7.4 containing 0.5%(w/v) gelatin and 0.5% (v/v) chloroform and stored at 4°C. After 2 days LE392 cells were infected with the eluate from the primary plugs and replated for the secondary screen. This process was repeated until hybridizing plaques were pure.
   Once purified, positive cDNAs were excised from the ZAP II bacteriophage according to the manufacturer's instructions (Stratagene, CA, USA) and cloned into the plasmid pBluescript. A CsCl purified preparation of the DNA was made and this was sequenced on both strands. Sequencing was performed using an Applied Biosystems automated DNA sequencer, with fluorescent dideoxynucleotide analogues according to the manufacturer's instructions. The DNA sequence was analysed using software supplied by Applied Biosystems.
   Two clones isolated from the mouse testis cDNA library shared large regions of nucleotide sequence identity 68-1 and 68-2 and appeared to encode a novel member of the haemopoietin receptor family and the inventors gave the putative receptor the working name "NR6".
(ii) In a parallel series of experiments, a commercial mouse brain cDNA library (STRATAGENE #967319, Balb/c day-20, whole brain cDNA/Uni-ZAP XR Vector) was used to infect *E.coli* strain XL1-Blue MRF'. Infected bacteria were grown on 90x135mm square agar plates to give about 25,000 plaques per plate. Plaques were then transferred to positively charged nylon membranes, Hybond-N(+) (Amersham RPN 203B), bacteria were lysed and the DNA was denatured with denaturing 0.5 M NaOH, 1.5 M NaCl at room temperature for 7 min. The membranes were neutralized with 0.5 M Tris-HCL pH7.2, 1.5 M NaCl, 1 mM EDTA at room temperature for 10 min before the DNA fixation by UV crosslinking.
   A mixture of WSDWS and WSEWS oligonucleotide probes (SEQ ID NOs: 7 and 8) were labelled with a [α-³²P]-ATP (TOYOBO #PNK-104 Kination kit). The membranes from the mouse brain cDNA library were then hybridized with the mixture of WSDWS and WSEWS oligonucleotide probes in the Rapid Hybridization Buffer (Amersham, RPN1636) at 42°C for 16 hours. Filters were washed with 1xSSC/0.1% (w/v) SDS at 42°C before autoradiography. Plaques that appeared positive on orientated duplicate filters were picked and replated on E. coli, XL1-Blue MRF' with the process of immobilisation on nylon membranes, hybridization of membranes with oligonucleotide probes, washing and autoradiography repeated until pure plaques had been obtained.
   The cDNA fragment from pure positively hybridizing plaques was isolated by excision with the helper phage strain ExAssist according to the manufacturer=s instructions (Stratagene, #967319). Sequencing was performed after the amplification with Ampli-Taq DNA polymerase and Taq dideoxy terminator cycle sequencing kit (Perkin Elmer, #401150) by 25 cycles of 96°C for 10 sec, 50°C for 5 sec, 60°C for 4 min followed by 60°C for 5 min with the sequencing primers on an ABI model 377 DNA sequencer.
   One clone, MBC-8, from the mouse brain library shared large regions of nucleotide sequence identity with both the 68-1 and 68-2 clones isolated from the mouse testis cDNA library.
(iii) In a third series of experiments, total RNA was prepared from the mouse osteoblastic cell line, KUSA, according to the method of Chirgwin *et al*. (15), and poly(A)+RNA was further purified by oligo(dT)-cellulose chromatography (Pharmacia Biotech). Complementary DNA was synthesized by oligo(dT) priming, inserted into the UniZAP XR directional cloning vector (Stratagene), and packaged into X phage using Gigapack Gold (Stratagene), yielding 1.25 x 10⁷ independent clones.

Approximately 10⁶ clones were screened essentially as described in (ii) above. Briefly, probes were labeled with ³²P using T4 polynucleotide kinase and prehybridization was performed for 4 hr in the Rapid hybridization buffer (Amersham LIFE SCIENCE) at 42°C. Filters (Hybond N+, Amersham) were then hybridized for 19 hr under the same condition with the addition of ³²P-labeled WSXWS mix oligonucleotides and washed 3 times. The final wash was for 30 min in 1 x SSPE, 0.1% (w/v) SDS at 42°C. Filters were then exposed with an intensifying screen to Kodak X-OMAT AR film for 5 days.

Isolated clones were subjected to the *in vivo* excision of pBluescript SK(-) phagemid (Stratagene), and plasmid DNA was prepared by the standard method. DNA sequences were determined using an ABI PRISM 377 DNA Sequencer (Perkin Elmer) with appropriate synthetic oligonucleotide primers. A clone pKUSA166 shared large regions of nucleotide sequence identity with the MBC-8, 68-1 and 68-2 clones isolated from the mouse brain and testis cDNA libraries.

### EXAMPLE 3

### Isolation of further NR6 cDNA clones using probes specific for NR6

(i) In order to identify other cDNA libraries containing cDNA clones for NR6, the inventors performed PCR upon 1 *µ*l aliquots of λ-bacteriophage cDNA libraries made from mRNA from various human tissues and using oligonucleotides 2070 and 2057, designed from the sequence of 68-1 and 68-2, as primers. Reactions contained 5 µl of 10 x concentrated PCR buffer (Boehringer Mannheim GmbH, Mannheim, Germany), 1 µl of 10 mM dATP, dCTP, dGTP and dTTP, 2.5 µl of the oligonucleotides HYB2 and either T3 or T7 at a concentration of 100 mg/ml, 0.5 µl of Taq polymerase (Boehringer Mannheim GmbH) and water to a final volume of 50 µl. PCR was carried out in a Perkin-Elmer 9600 by heating the reactions to 96°C for 2 min and then for 25 cycles at 96°C for 30 sec, 55°C for 30 sec and 72°C for 2 min. PCR products were resolved on an agarose gel, immobilized on a nylon membrane and hybridized with ³²P-labelled oligonucleotide 1943 (SEQ ID NO:42).

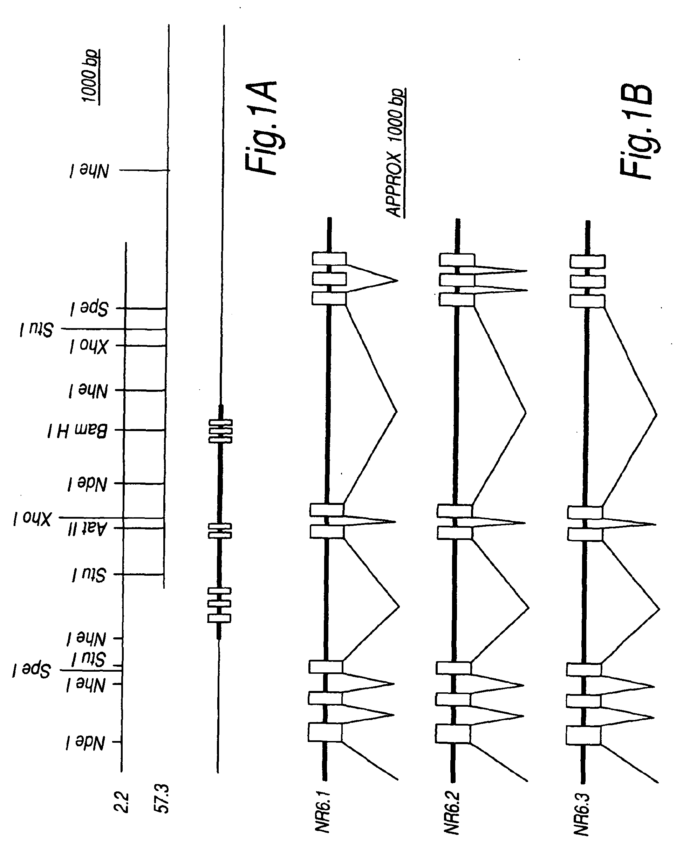

In addition to the original library, a mouse brain cDNA library appeared to contain NR6 cDNAs. These were screened using a ³²P-labelled oligonucleotides 1944, 2106, 2120 (Example 1) or with a fragment of the original NR6 cDNA clone from 68-1 (nucleotide 934 to the end of NR6.1 in Figure 1) labelled with ³²P using a random decanucleotide labelling kit (Bresatec). Conditions used were similar to those described in (i) above except that for the labelled oligonucleotides, filters were washed at 55°C rather than 45°C, while for the NR6 cDNA fragment prehybridization and hybridization was carried out in 2xSSC and filters were washed at 0.2 x SSC at 65°C. Again, as described in (i) above, positively hybridising plaques were purified, the cDNAs were recovered and cloned into plasmids pBluescript II or pUC19. Independent cDNA clones were sequenced on both strands.

Using this procedure, 6 further clones, 68-5, 68-35, 68-41, 68-51, 68-77 and 73-23, contained large regions of sequence identity with 68-1, 68-2, MBC-8 and pKUSA166.

In a parallel series of experiments, further screening was performed with hybridization probes prepared from the 1.7 kbp EcoRI-XhoI fragment excised from pKUSA166. This fragment was excised and labeled with ³²P by using T7QuickPrime Kit (Pharmacia Biotech). Approximately 6x10⁵ clones were screened. Hybond N+ filters (Amersham) were first prehybridized for 4hr at 42°C in 50% (v/v) formamide, 5xSSPE, 5xDenhardt's solution, 0.1% (w/v) SDS, and 0.1mg/ml denatured salmon sperm DNA. Hybridization was for 16 hours under the same conditions with the addition of ³²P- labelled NR6- cDNA fragment probes. Finally the filters were washed once for 1hr in 0.2xSSC, 0.1% (w/v) SDS at 68°C. Eight clones were isolated, and phage clones were subjected to the *in vivo* excision of the pBluescript SK(-) phagemid (Stratagene). The plasmid DNAs were prepared by the standard method. DNA sequences were determined by an ABI PRISM 377 DNA Sequencer using appropriate synthetic oligonucleotide primers.

Using this procedure 8 further clones from the KUSA library contained large regions of sequence identity with 68-1, 68-2, MBC-8, pKUSA166, 68-5, 68-35, 68-41, 68-51, 68-77 and 73-23 were isolated.

### EXAMPLE 4

### Isolation of genomic DNA encoding NR6

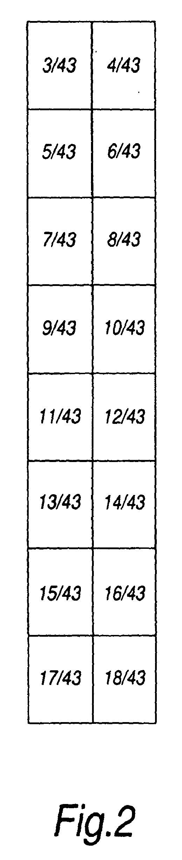

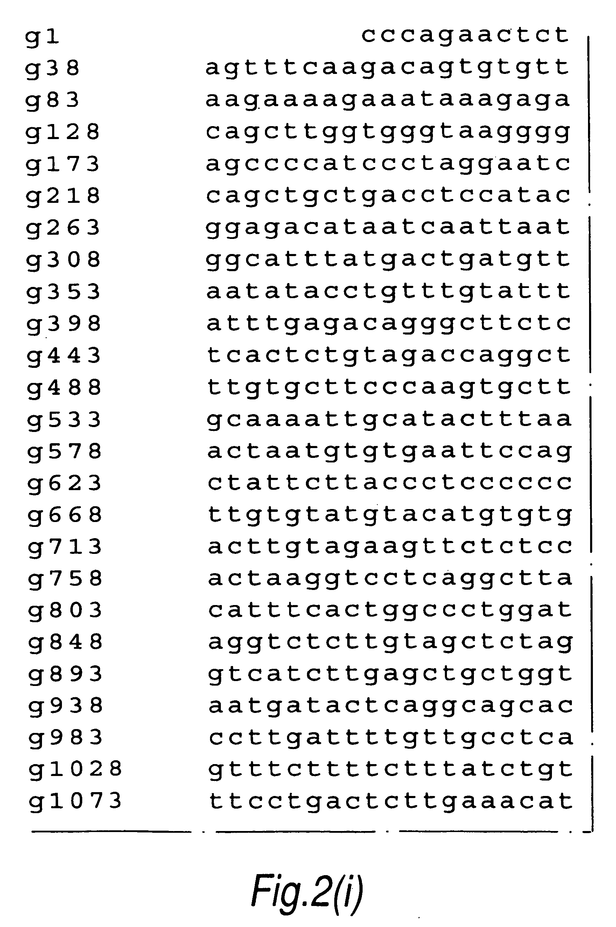

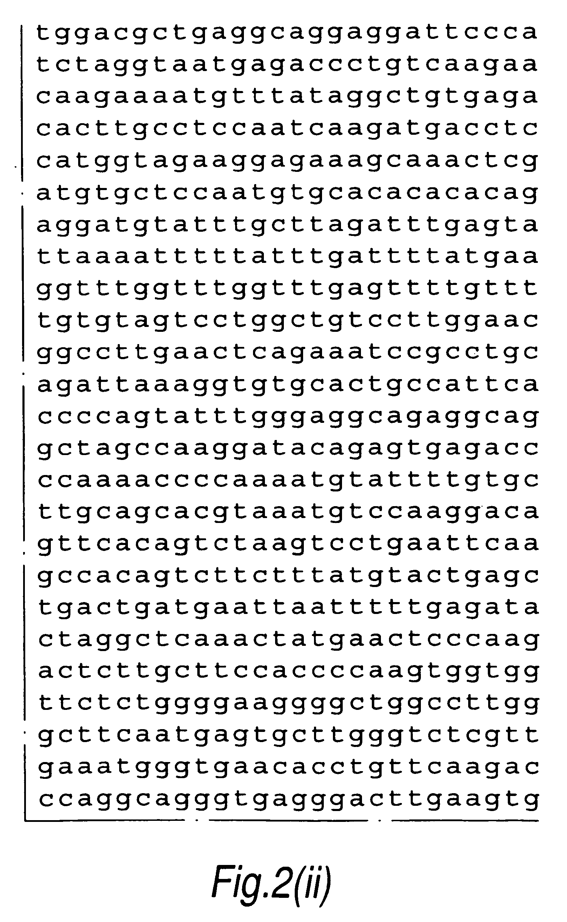

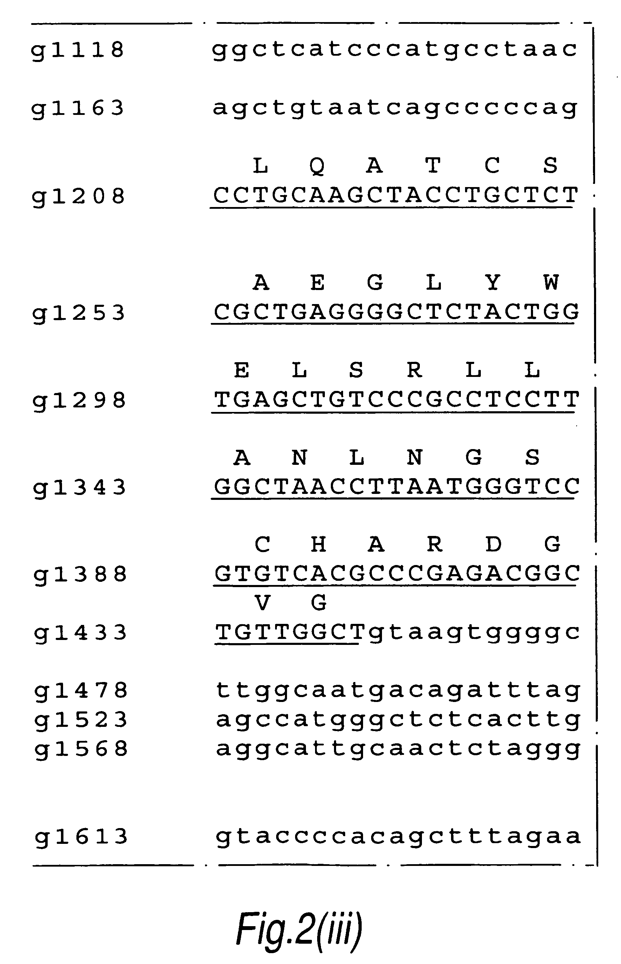

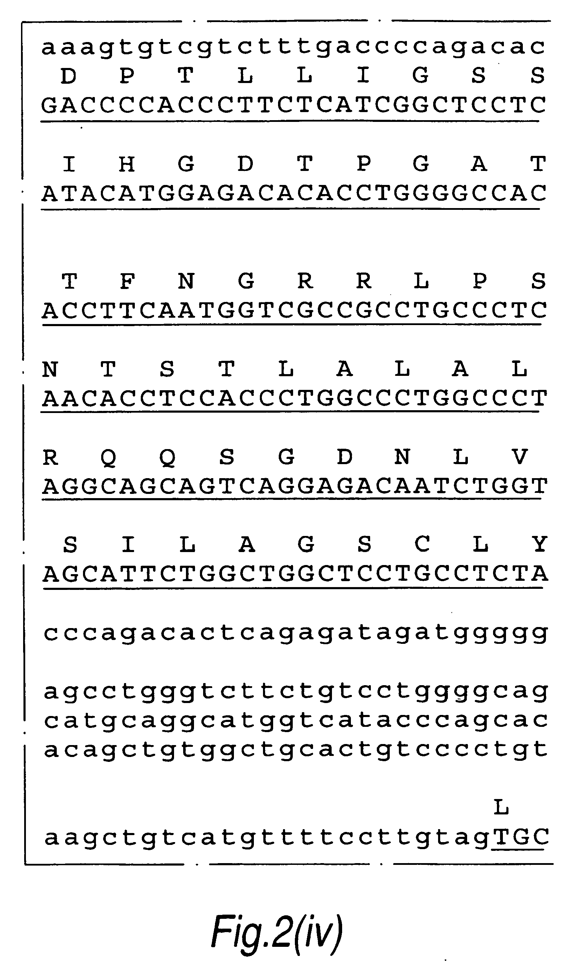

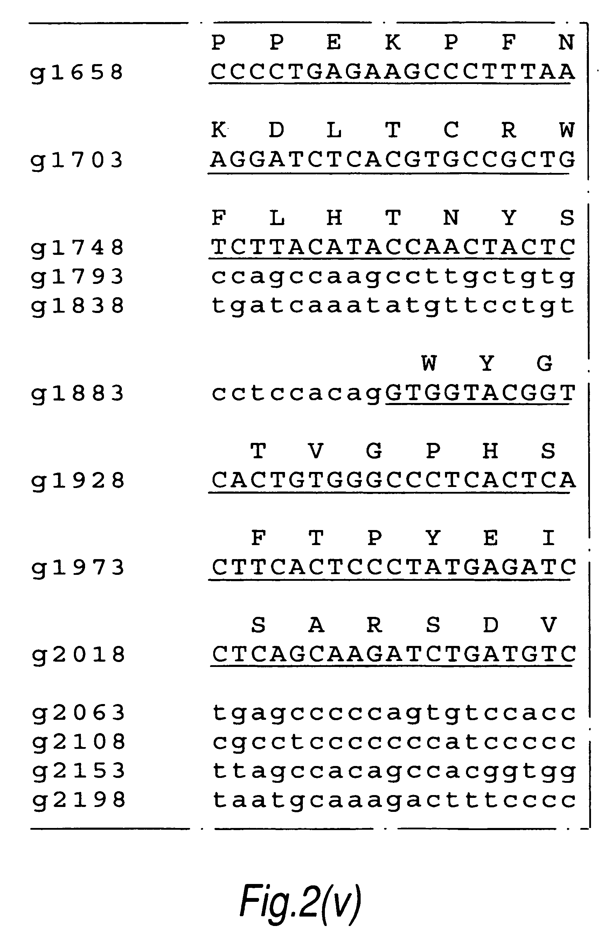

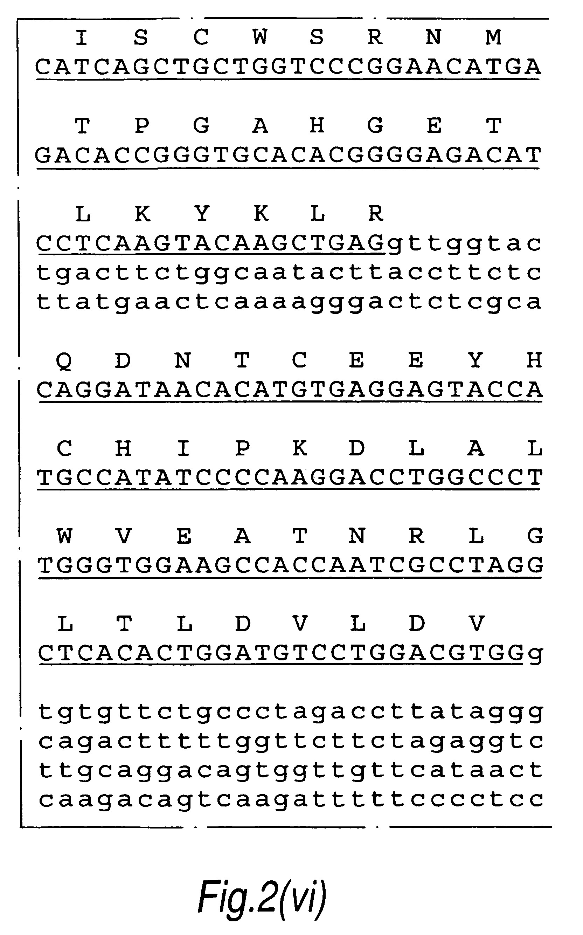

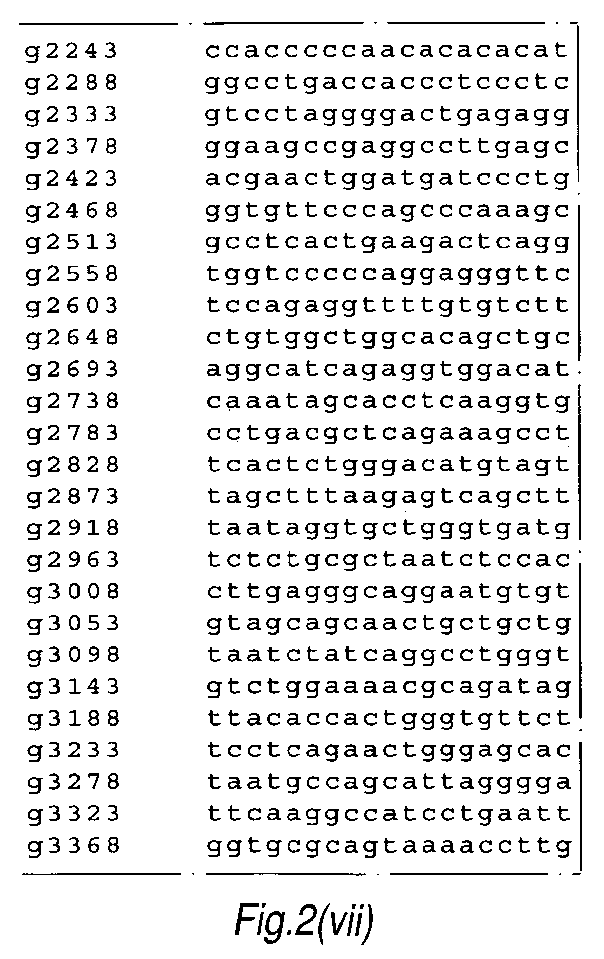

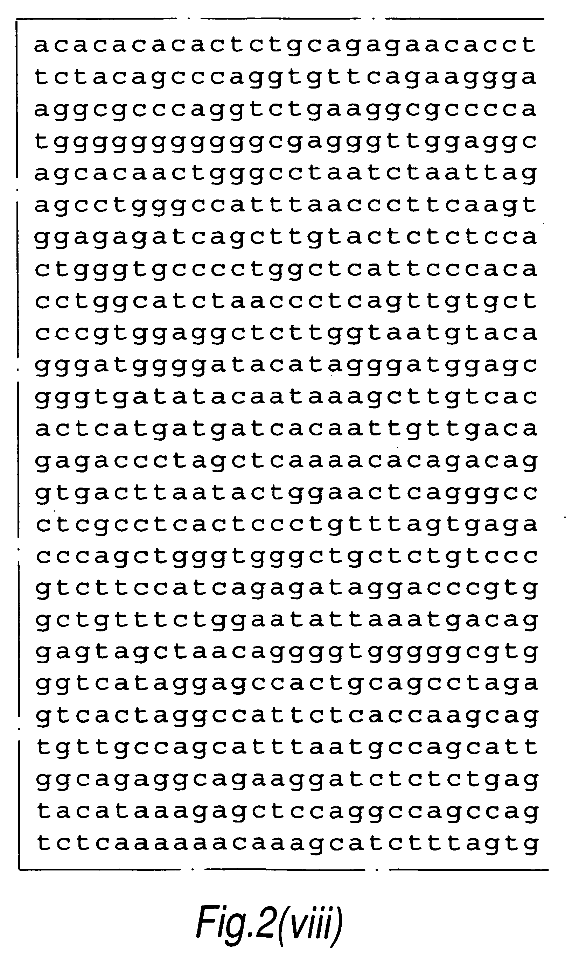

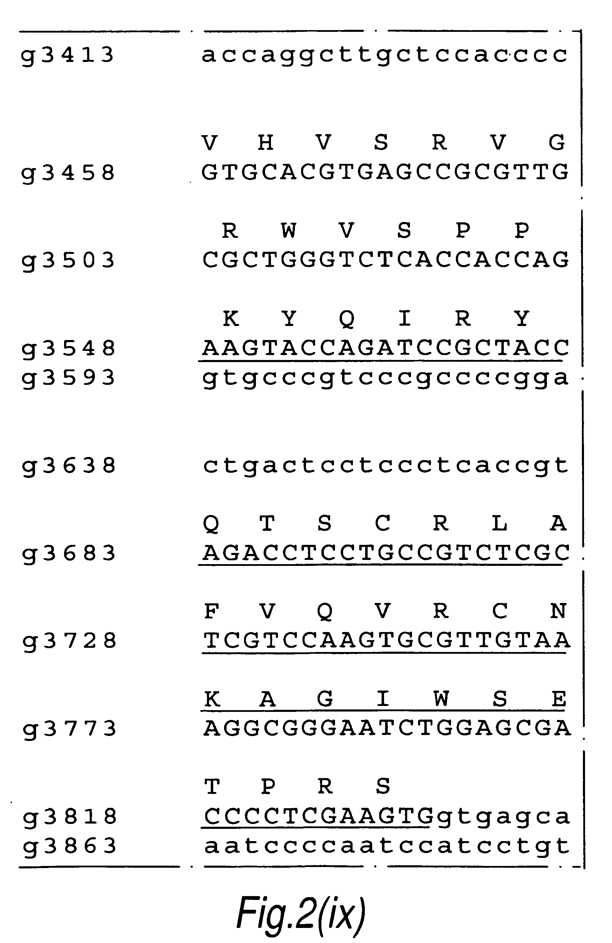

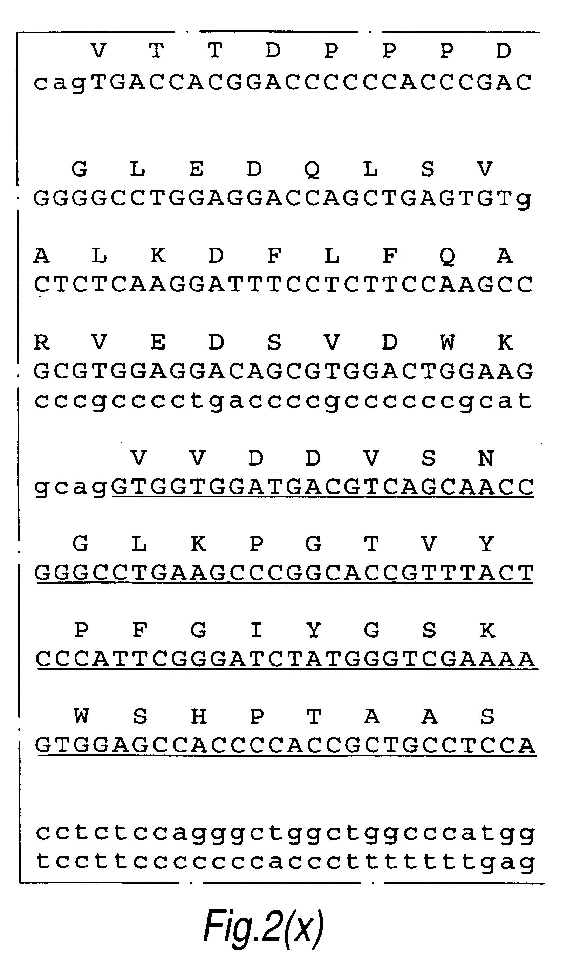

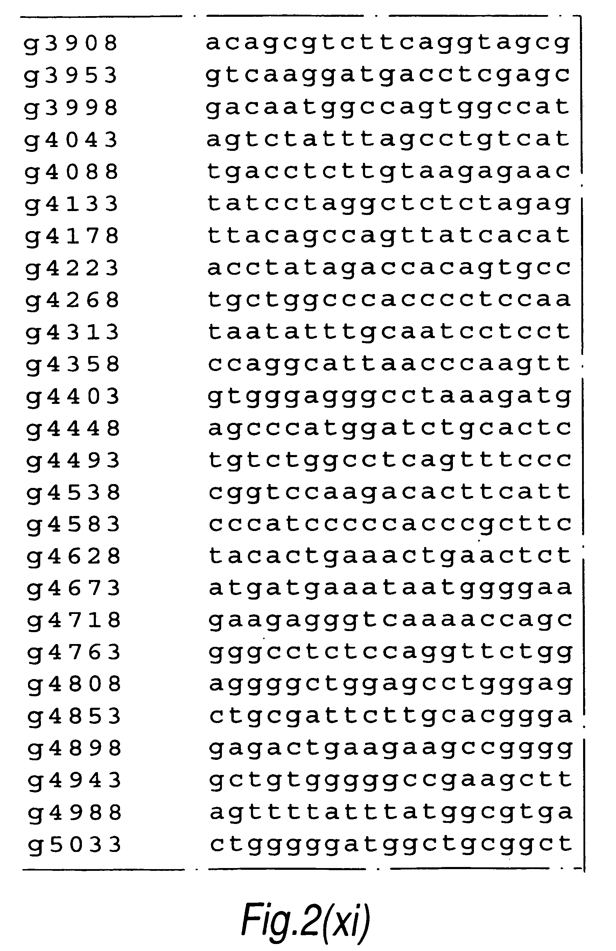

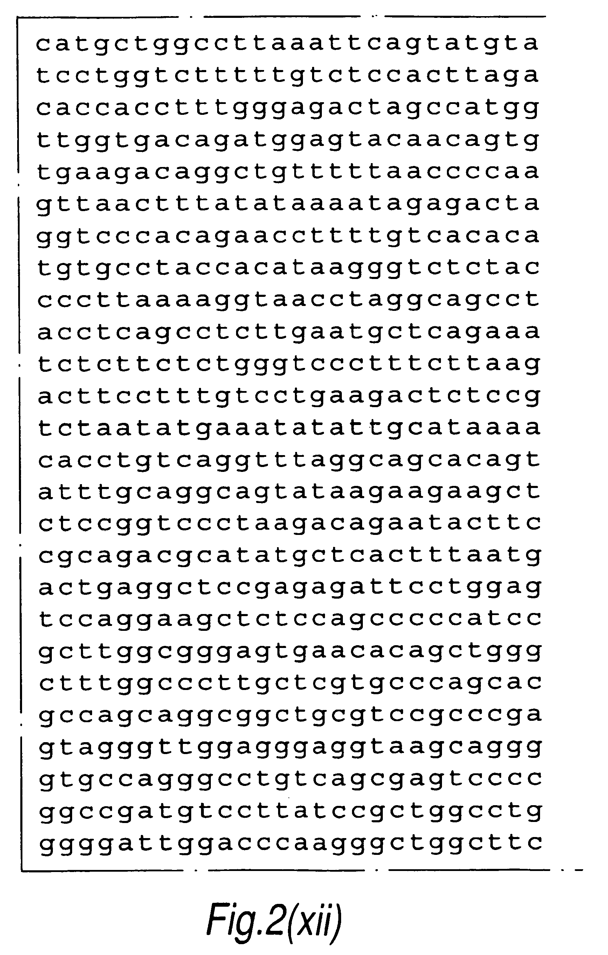

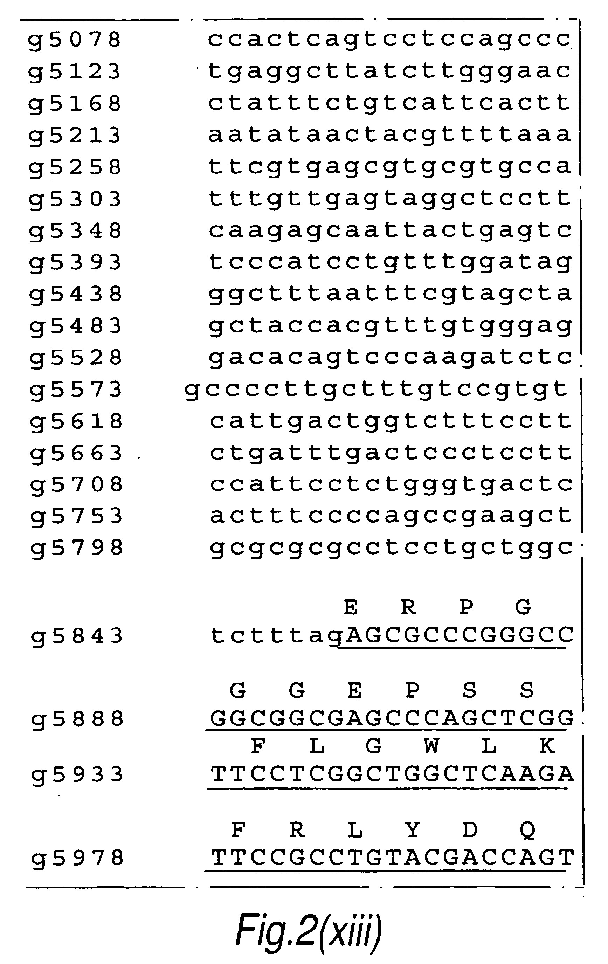

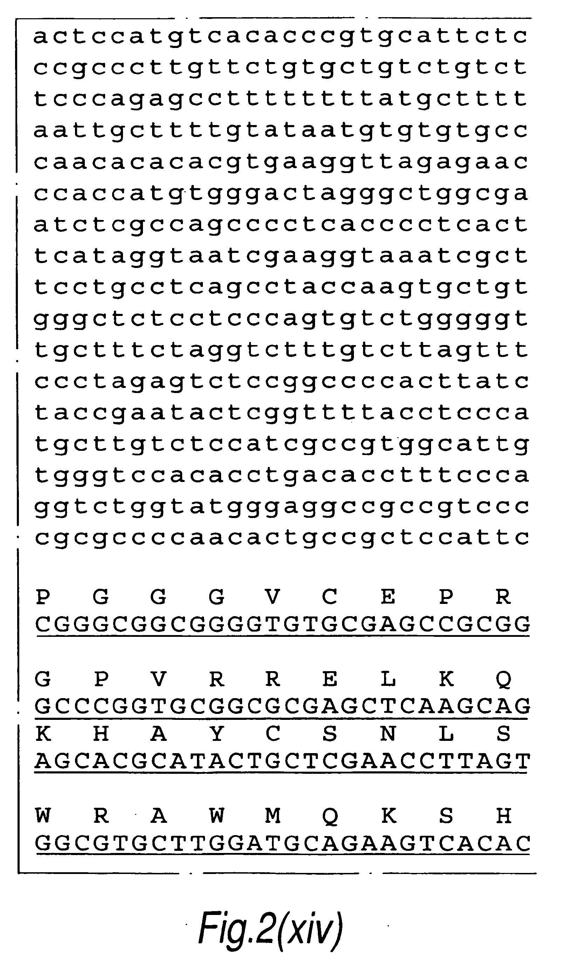

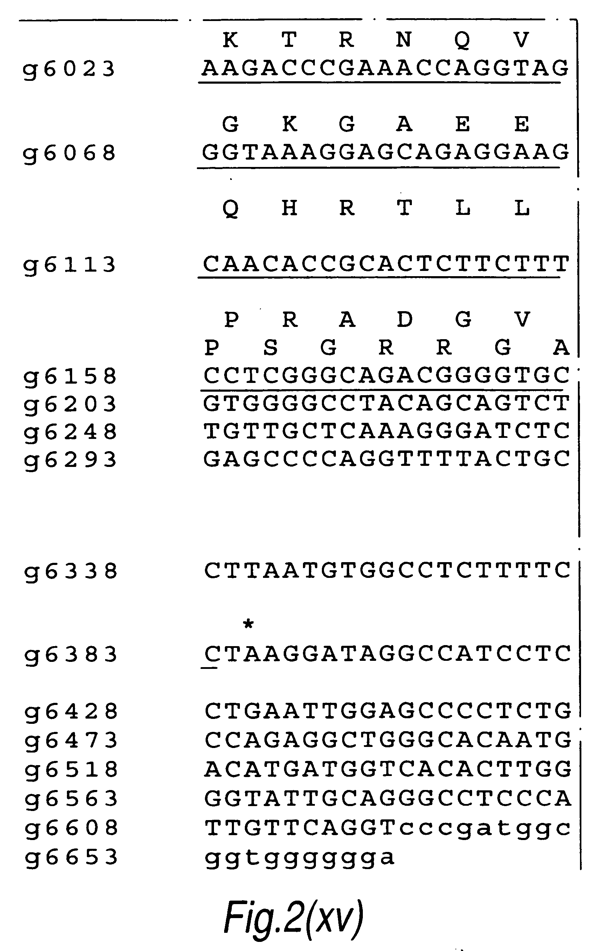

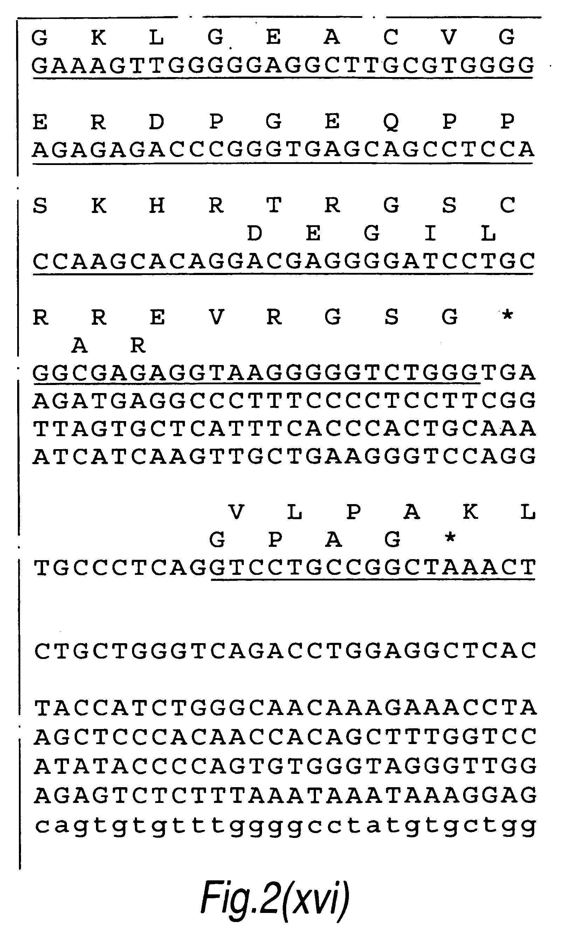

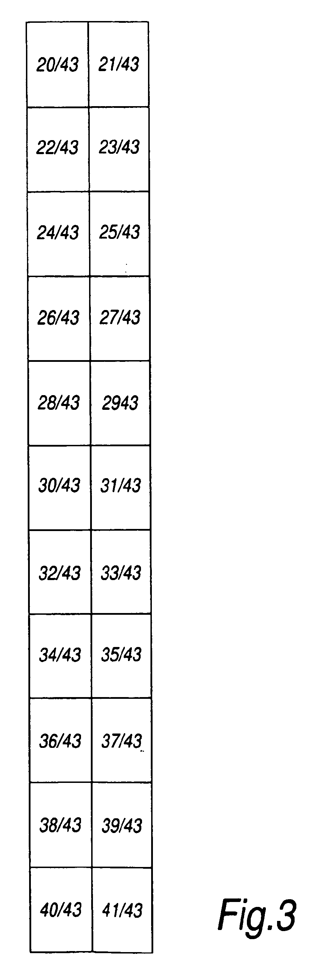

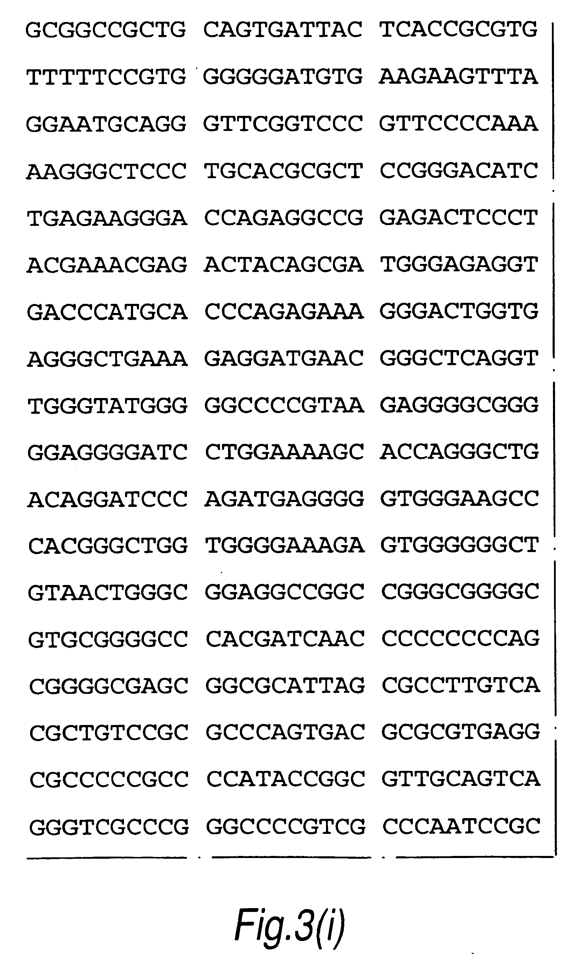

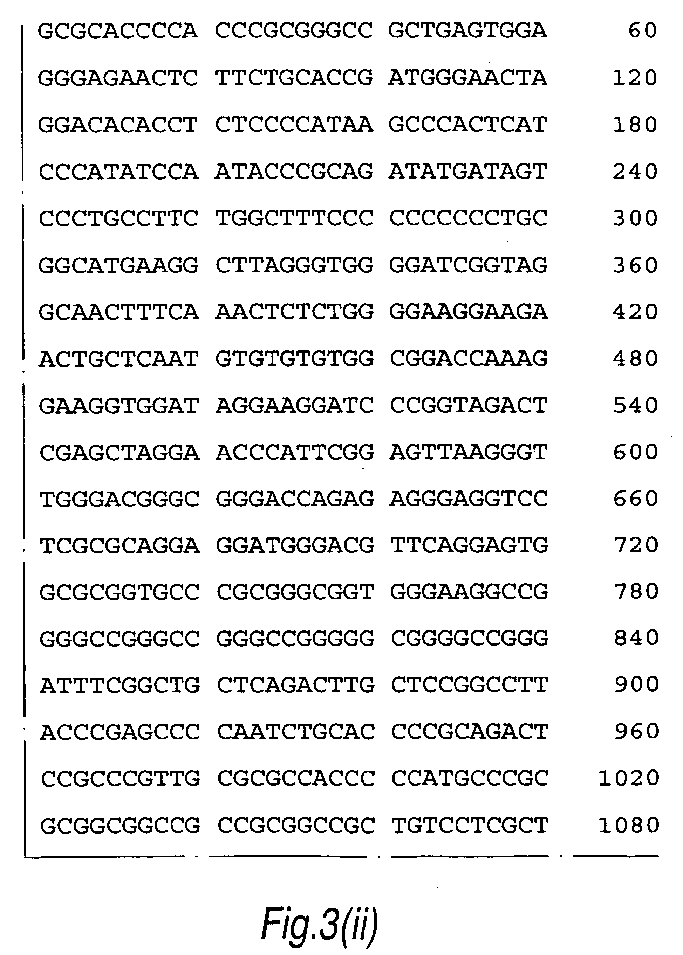

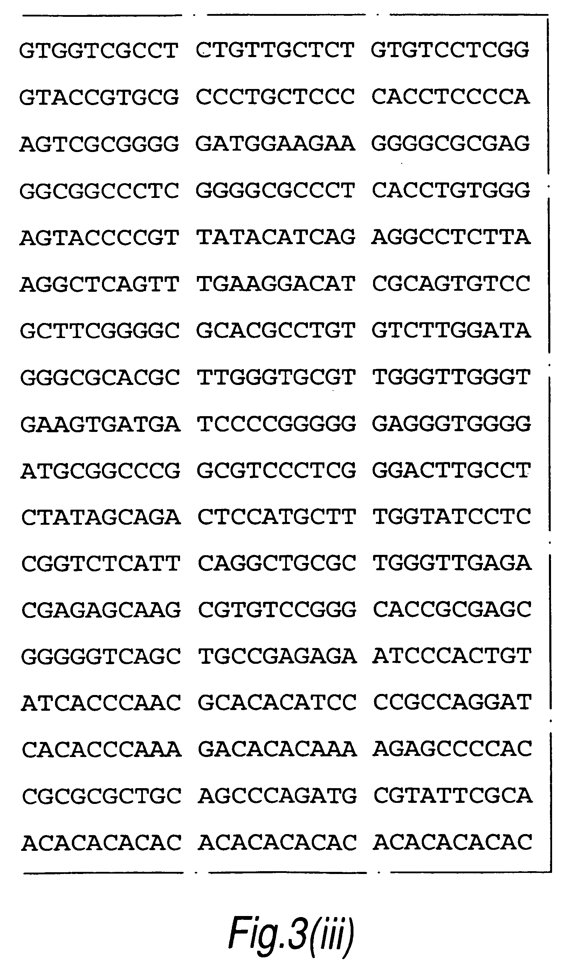

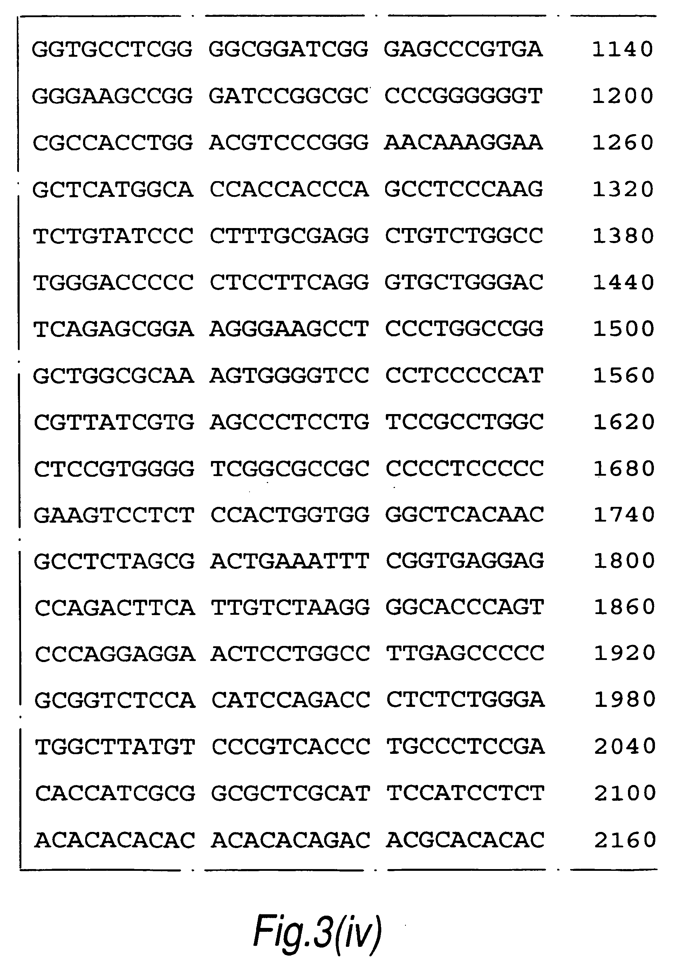

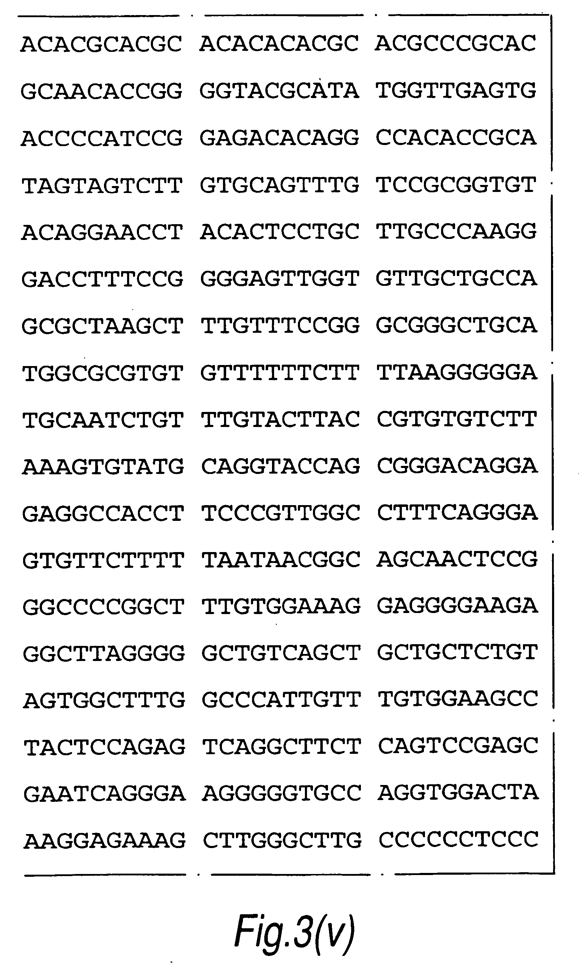

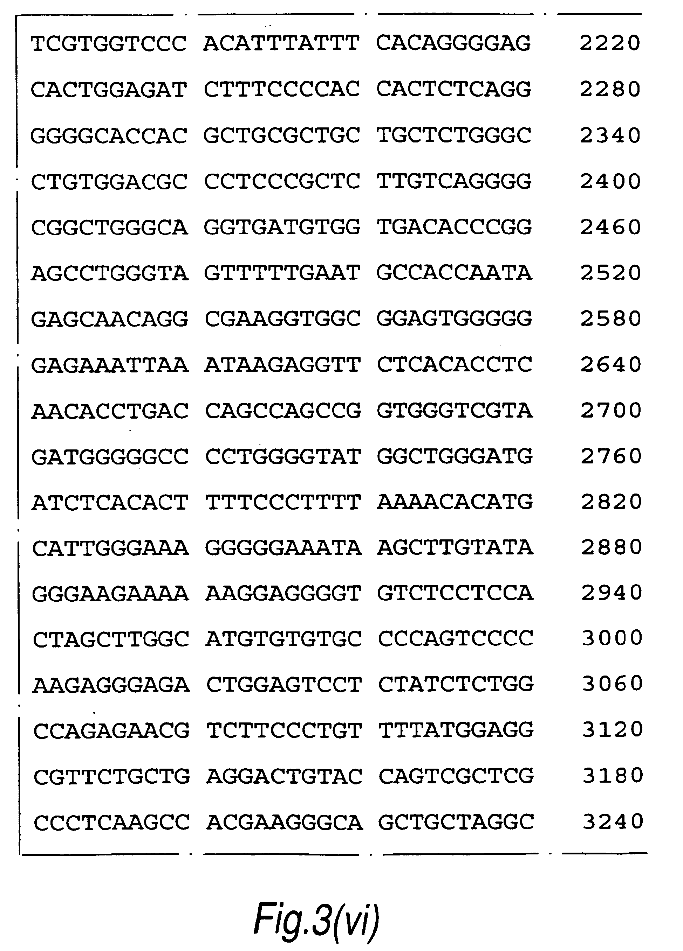

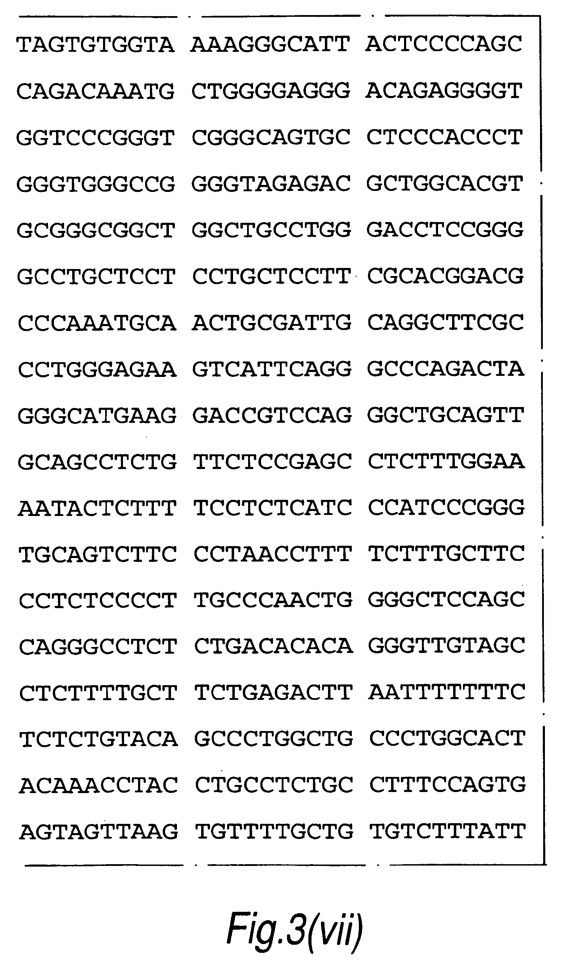

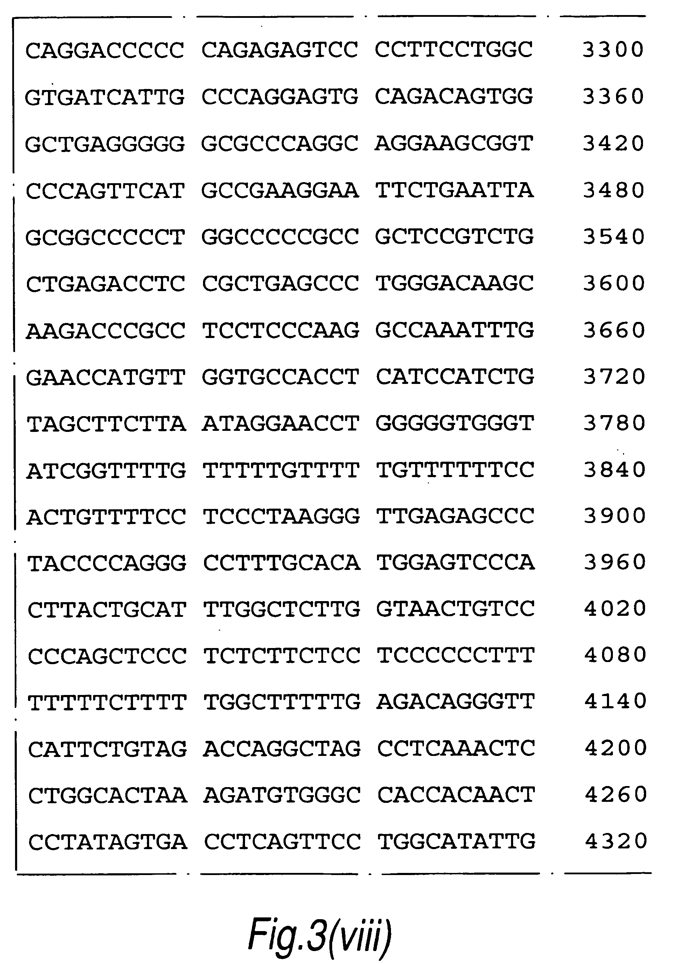

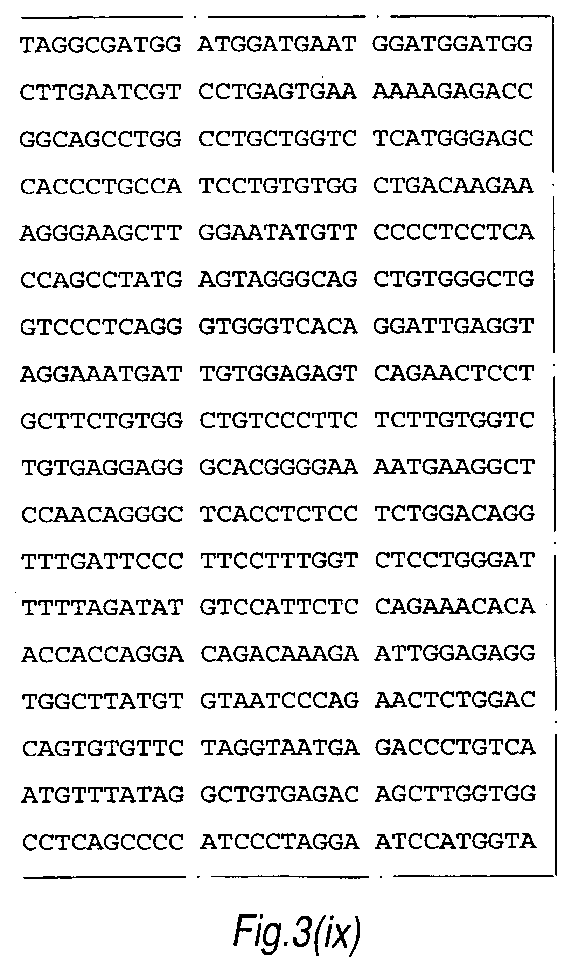

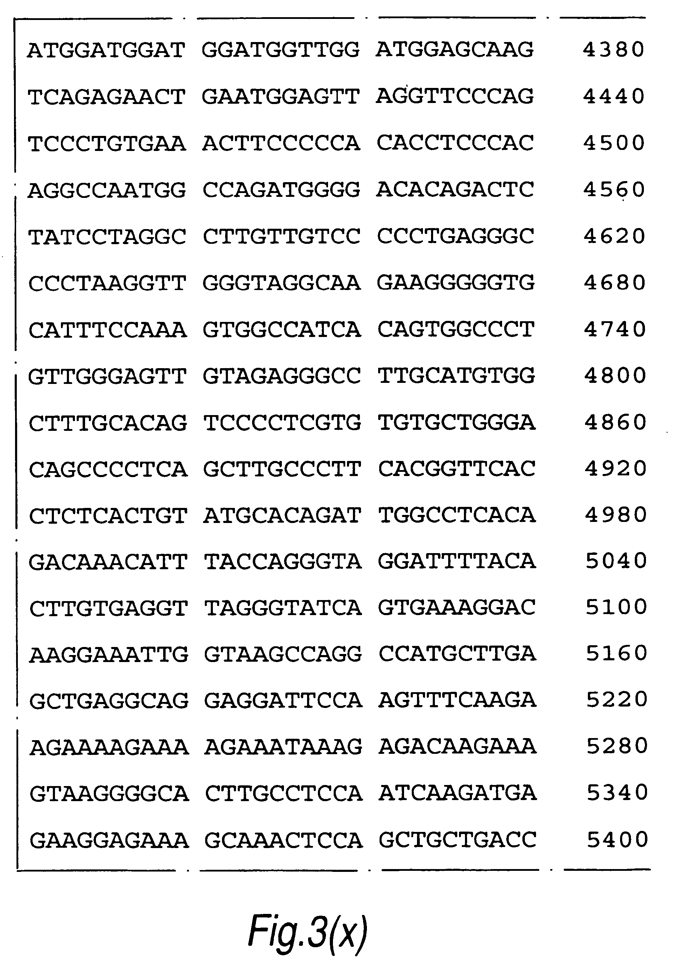

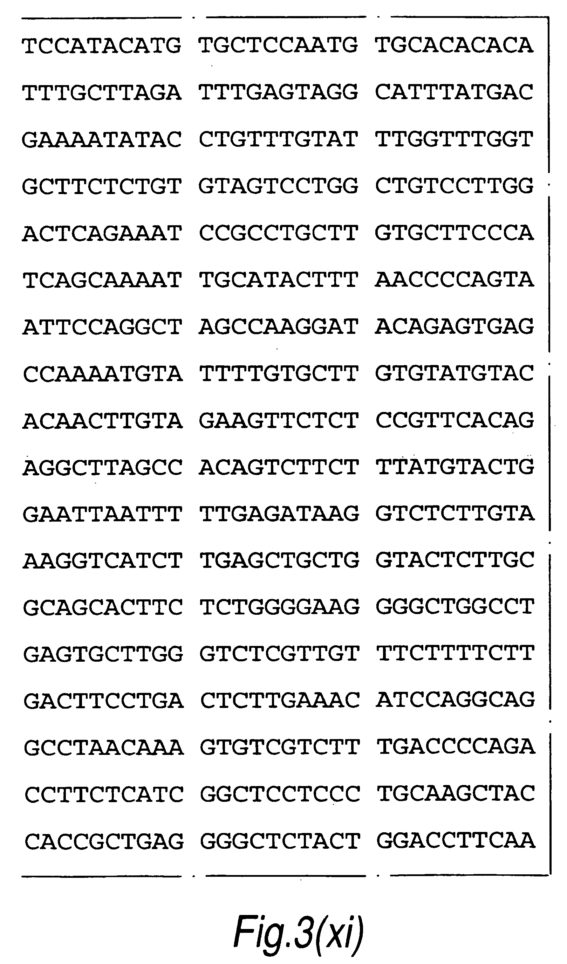

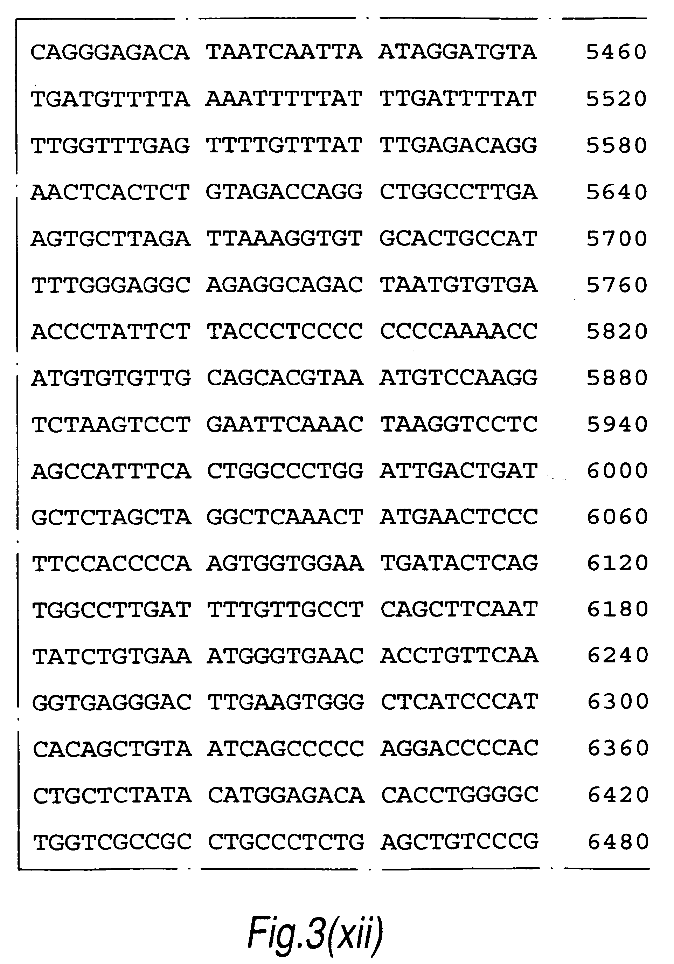

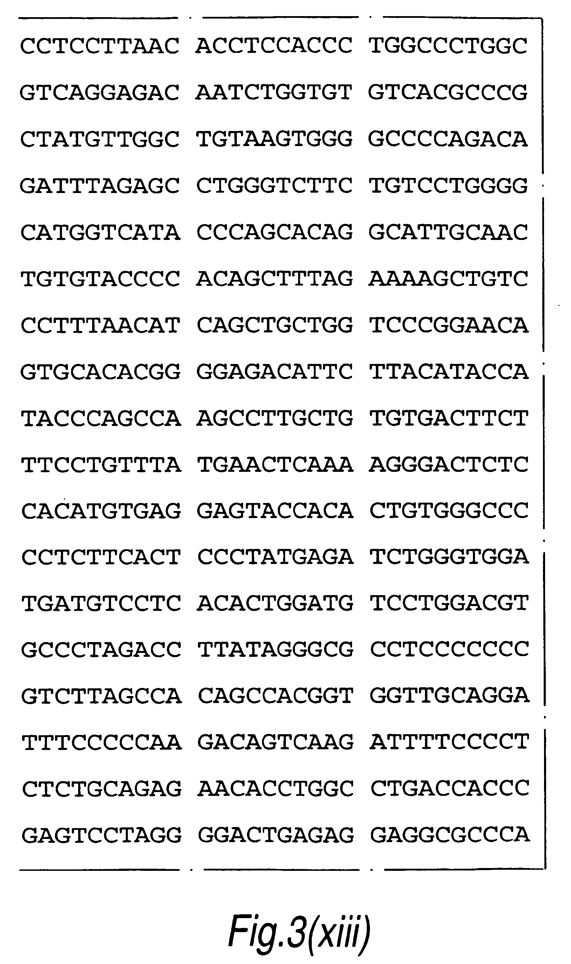

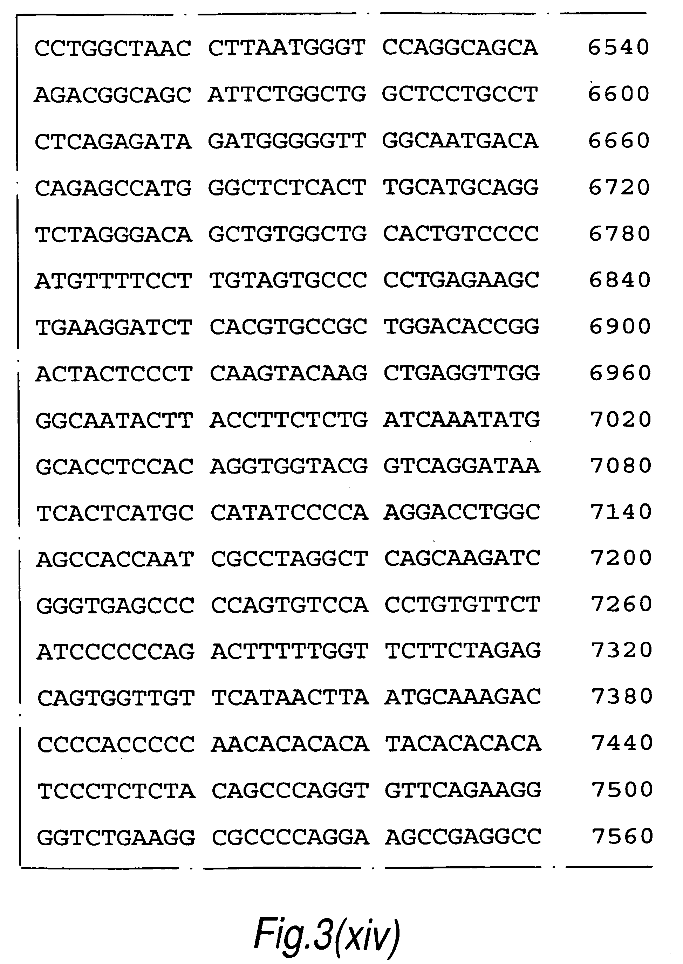

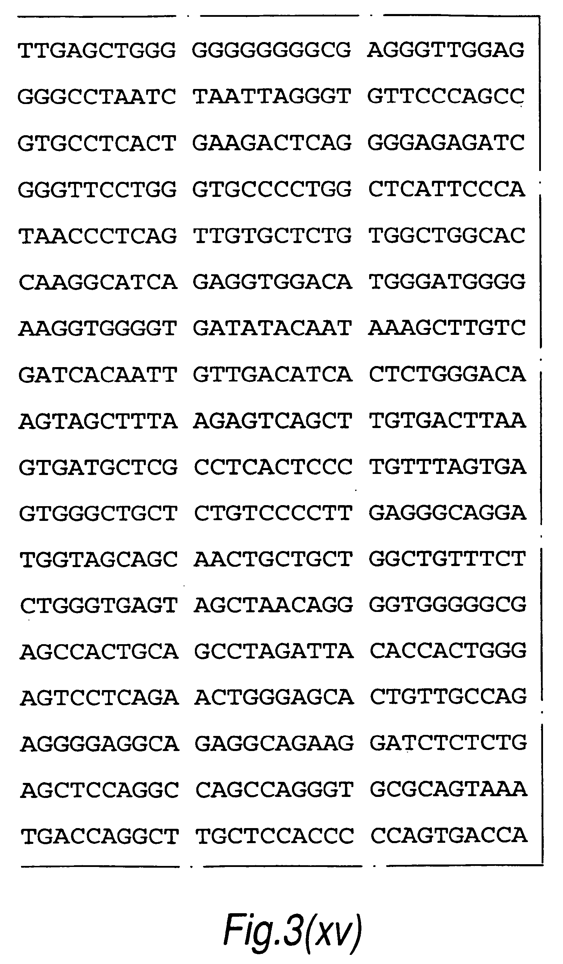

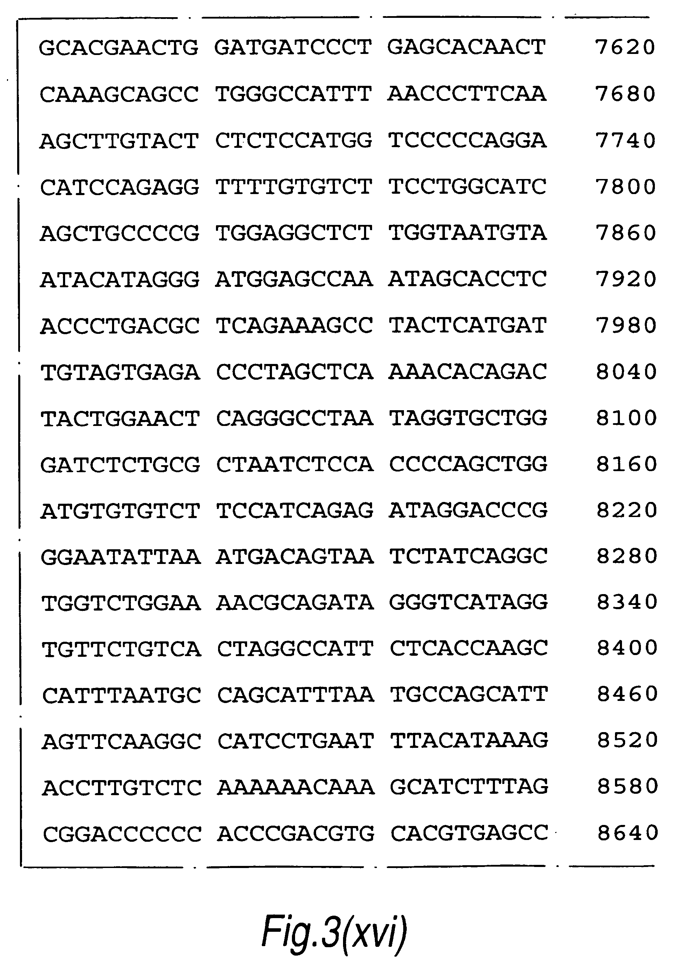

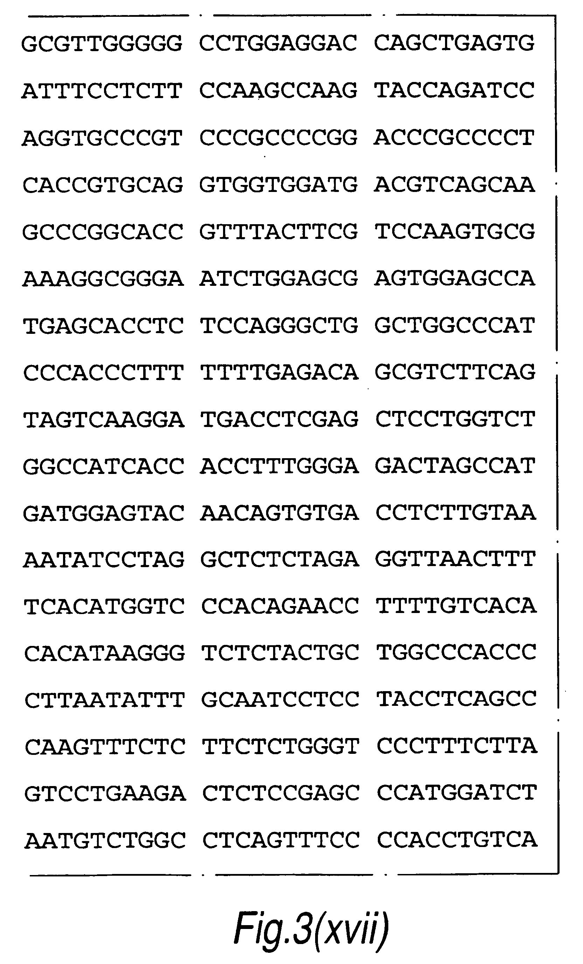

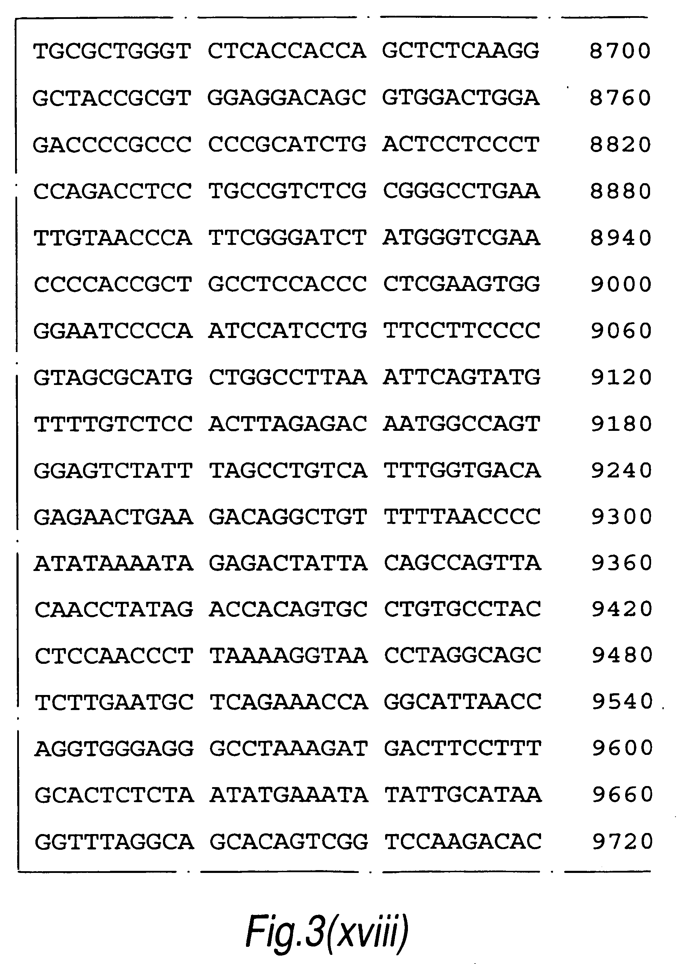

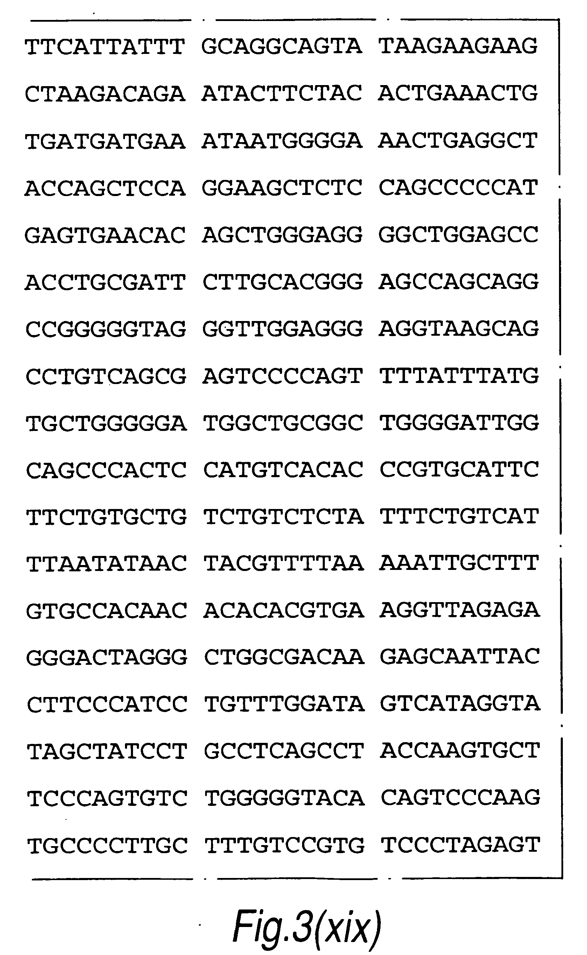

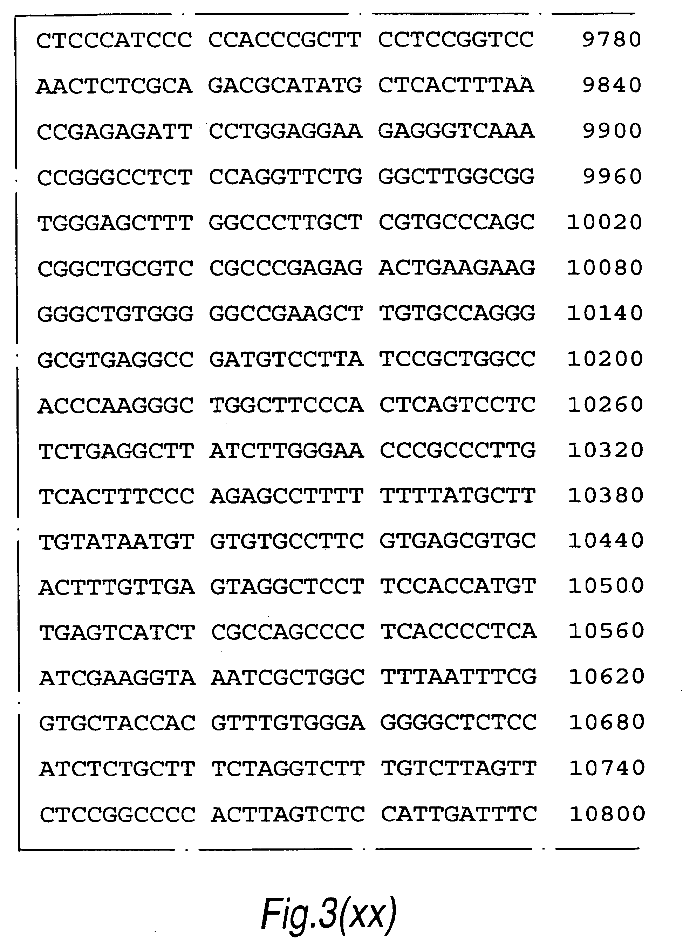

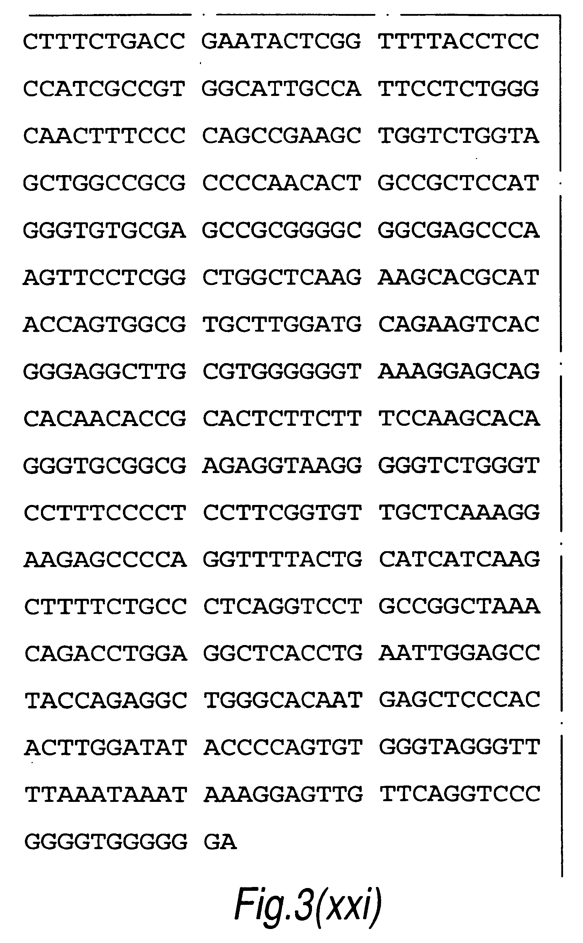

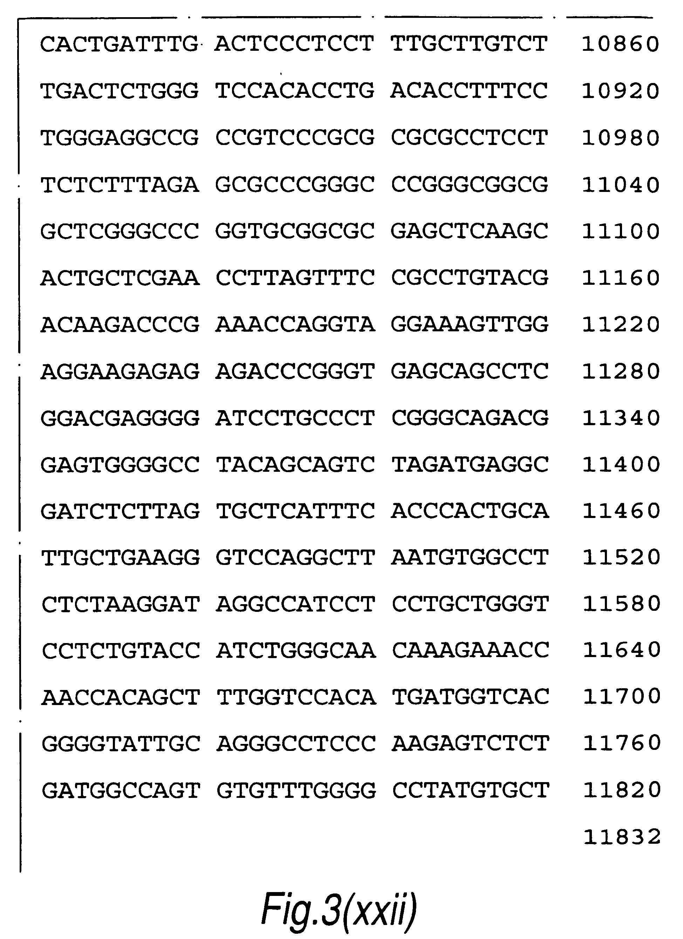

DNA encoding the murine NR6 genomic locus was also isolated using the 68-1 cDNA as a probe. Two positive clones, 2-2 and 57-3, were isolated from a mouse 129/Sv strain genomic DNA library cloned into λ FIX. These clones were overlapping and the position of the restriction sites, introns and exons were determined in the conventional manner. The region of the genomic clones containing exons and the intervening introns were sequenced on both strands using an Applied Biosystems automated DNA sequencer, with fluorescent dideoxynucleotide analogues according to the manufacturer's instructions. Figure 2 shows the nucleotide sequence and corresponding amino acid sequence of the translation regions. This is also shown in SEQ ID NOs:30 and 31. Figure 3 provides the genomic NR6 gene sequence but with additional 5' sequence. This is also represented in SEQ ID NO:38 in relation to this sequence. The coding exons of NR6 span approximately 11kb of the mouse genome. There are 9 coding exons separated by 8 introns:

| | | |
|---|---|---|
| exon1 | at least 239nt | intron1 5195nt |
| exon2 | 282nt | intron2 214nt |
| exon3 | 130nt | intron3 107nt |
| exon4 | 170nt | intron4 1372nt |
| exon5 | 158nt | intron5 68nt |
| exon6 | 169nt | intron6 2020nt |
| exon7 | 188nt | intron7 104nt |
| exon8 | 43nt | intron8 181nt |
| exon9 | 252nt | |

Exon 1 encodes the signal sequence, exon 2 the Ig-like domain, exons 3 to 6 the hemopoietin domain. Exons 7, 8 and 9 are alternatively spliced.

### EXAMPLE 5

### 5' RACE analysis of NR6

5'-RACE was used to investigate the nature of the sequence 5' of nucleotide 960, encoding Ile321 of NR6.1, 2 and 3. The nucleotide and corresponding amino acid sequences are shown in SEQ ID NOs:12, 14 and 16, respectively. 5'-RACE was performed using Advantage KlenTaq polymerase (CLONTECH, CAT NO. K1905-1) on mouse brain Marathon-ready cDNA (CLONTECH, CAT NO. 7450-1) according to the manufacturer's instructions. Briefly, the first rounds of amplification were performed using 5µl of cDNA in a total volume of 50µl, with 1mM each of the primers AP1&M116 [SEQ ID NO:2] or AP1&M159 [SEQ ID NO:4] by 35 cycles of 94°C x 0.5min, 68°C x 2.0min on GeneAmp 2400 (Perkin-Elmer). An amount of 5µl of 50-fold diluted product from the first amplification was then re-amplified ; for the products generated with primers AP1 and M116 [SEQ ID NO:2] in the first amplification, 1 mM of the primers AP2&M108 [SEQ ID NO:3] were used in the second amplification. For the products generated with primers AP1 and M116 [SEQ ID NO:2] in the first amplification, two separate secondary reactions were performed, one reaction with 1 mM primers AP2&M242 [SEQ ID NO:5] and the other with 1 mM primers AP2&M112 [SEQ ID NO:6]. Amplification was achieved using 25 cycles of 94°C x 0.5min, 68°C x 2.0min. These samples were analyzed by agarose gel electrophoresis. When a single ethidium bromide staining amplification product was observed, it was purified by QIAquick PCR purification kit according to the manufacturer's instructions (QIAGEN, CAT NO. DG-0281) and its sequence was directly determined using both primers used in the secondary amplification step, that is AP2 and either M108 [SEQ ID NO:3], M242 [SEQ ID NO:5] or M112 [SEQ ID NO:6].

### EXAMPLE 6

### Cloning of NR6

From the initial screens of mouse brain and testis cDNA libraries with the degenerate WSXWS oligonucleotides and subsequent screening of cDNA libraries from mouse testis, mouse brain and the KUSA osteoblastic cells line a total of 18 NR6 cDNAs have been isolated. Nucleotide sequence of NR6 was also determined from 5'RACE analysis of brain cDNA. Additionally, two murine genomic DNA clones encoding NR6 have also been isolated.

Comparison of the NR6 cDNA clones revealed a common region of nucleotide sequence which included a 123 base pairs 5'-untranslated region and 1221 base pairs open reading frame, stretching from the putative initiation methionine, Met1 to Gln407 (SEQ ID NOs:12, 14 and 16, respectively). Within this common open reading frame, a haemopoietin receptor domain was observed which contained the four conserved cysteine residues and the five amino acid motif WSXWS typical of members of the haemopoietin receptor family, was observed.

Further analyses revealed that after nucleotide 1221, three different classes of NR6 cDNAs could be found, these were termed NR6.1, NR6.2 and NR6.3 (SEQ ID NOs:12, 14 and 16, respectively). Each encoded a receptor that appeared to lack a classical transmembrane domain and, would, therefore be likely to be secreted into the extracellular environment. Although the putative C-terminal region of the three classes of NR6 proteins appear to be different, the cDNAs encoding them also had a common region of 3'-untranslated region.

With regard to SEQ ID NOs:12, 14 and 16, the number of both nucleotides and amino acids begins at the putative initiation methione. NR6.1 and NR6.2 are identical to nucleotide 1223 encoding Q407, this represents the end of an exon. NR6.1 splices out an exon present only in NR6.2 and uses a different reading frame for the final exon which is shared with NR6.2. The 3N-untranslated region is shared by NR6.1, NR6.2 and NR6.3, NR6.2 splices in an exon starting with nucleotide 1224 encoding D408 and ending with nucleotide 1264 encoding the first nucleotide in the codon for G422 and uses a different reading frame for the final exon which is shared with NR6.2 (see Figure 1). NR6.3 fails to splice from position nucleotide 1224, therefore, translation continues through the intron, giving rise to the C-terminal protein region.

The sequence of NR6 cDNA products generated by 5'-RACE amplification from mouse brain cDNA preparation is shown in SEQ ID NO:18. The nucleotide sequence identified using 5'-RACE appeared to be identical to the sequence of cDNAs encoding NR6.1, NR6.2, and NR6.3 from nucleotide C151, the first nucleotide for the codon for Pro51. 5' of this nucleotide, the sequences diverged and the sequence is unique not being found in NR6.1, NR6.2 or NR6.3. Additionally, there is a single nucleotide difference, with the sequence from the RACE containing an G rather than an A at nucleotide 475, resulting in Thr159 becoming Ala.

Analysis of the genomic clones, revealed that they were overlapping and contained exons encoding the majority of the coding region of the three forms of NR6 (Figures 1, 2 and 3). These genomic clones, contained exons encoding from Asp50 (nucleotide 148) of the NR6 cDNAs. Sequence 5' of this in the cDNAs, including the 5'-untranslated region and the region encoding Met1 to Gln49 (SEQ ID NOs:12, 14 and 16), and the 5' end predicted from analysis of 5' RACE products (SEQ ID NO:18) were not present in the two genomic clones isolated.

Analysis of the NR6 genomic DNA clones also provided an explanation of the three classes of NR6 cDNAs found. It is likely that NR6.1, NR6.2 and NR6.3 arise through alternative splicing of NR6 mRNA (Figure 1). The last amino acid residue that these different NR6 proteins are predicted to share is Gln407. SEQ ID NO:18 shows that Gln407 is the last amino acid encoded by the exon that covers nucleotides g5850 to g6037 (see Figure 2). Alternative splicing from the end of this exon (Figure 1) accounts for the generation of cDNAs encoding NR6.1 (SEQ ID NO:12), NR6.2 (SEQ ID NO:14) and NR6.3 (SEQ ID NO:16). In the case of NR6.1, the region from g6038 to g6425 is spliced out, leading to juxtaposition of g6037 and g6426. In the case of NR6.2, the region from g6038 to 6141 is spliced out, an exon from 6142 to g6183 is retained and then this is followed by splicing out of the region from g6183 to g6425. NR6.3 appears to arise when there is no splicing from nucleotide g6038. For all three forms, a secreted rather then transmembrane form is generated, these differ however in their predicted C-terminal region. The genomic NR6 sequence with additional 5' sequence is shown in Figure 3.

### EXAMPLE 7

### ESTs

Databases were searched with the murine NR6 corresponding to the unspliced version shown in SEQ ID NO:16. The murine NR6 sequence used is shown in SEQ ID NO:22.
The databases searched were:
(i) dbEST - Database of Expressed Sequence Tags National Center for Biotechnology Information National Library of Medicine, 38A, 8N8058600 Rockville Pike, Bethesda, MD 20894 Phone: 0011-1-301-496-2475 Fax: 0015-1-301-480-9241 USA.
(ii) DNA Data Bank of Japan DNA Database Release 3689. Prepared by: Sanzo Miyazawa Manager/Database Administrator HidenoriHayashida Scientific Reviewer Yukiko Yamazaki/Eriko Hatada/Hiroaki Serizawa Annotators/reviewers Motono Horie/Shigeko Suzuki/Yumiko SataoSecretaries/typists DNA Data Bank of JapanNational Institute of Genetics Center for Genetic Information research Laboratory of Genetic Information Analyses 1111 YataMishima, Shizuoka 411 Japan.
(iii) EMBL Nucleic Acid Sequence Data Bank Release 47.0.
(iv) EMBL Nucleic Acid Sequence Data Bank Weekly Updates Since Release 44.
(v) Genetic Sequence Data Bank NCBI-GenBank Release 94 National Center for Biotechnology Information National Library of Medicine, 38A, 8N805 8600 Rockville Pike, Bethesda, MD 20894 Phone: 0011-1-301-495-2475 Fax: 0015-1-301-480-9241 USA.
(vi) Cumulative Updates since NCBI-GenBank Release 88 National Center for Biotechnology Information National Library of Medicine, 38A, 8N805 8600 Rockville Pike, Bethesda, MD 20894 USA.

The search of the databases with the murine probe identified several EST's having sequence similarity to the probe. The EST's were:
W66776 (murine sequence)
MM5839 (murine sequence)
AA014965 (murine sequence)
W46604 (human sequence)
W46603 (human sequence)
H14009 (human sequence)
N78873 (human sequence)
R87407 (human sequence).

### EXAMPLE 8

### Isolation of 3' cDNA clones encoding human NR6

PCR products encoding human NR6 were generated using oligonucleotides UP1 and LP1 (see below) based on human ESTs (Genbank Acc:H14009, Genbank Acc:AA042914) that were identified from databases searched with murine NR6 sequence (SEQ ID NO:22). PCR was performed on a human fetal liver cDNA library (Marathon ready cDNA CLONTECH #7403-1) using Advantage Klen Taq Polymerase mix (CLONTECH #8417-1) in the buffer supplied at 94°C for 30s and 68°C for 3 min for 35 cycles followed by 68°C for 4 min and then stopping at 15°C. A standard PCR programme for the Perkin-Elmer GeneAmp PCT system 2400 thermal cycle was used. The PCR yielded a prominent product of approximately 560 base pairs (bp; SEQ ID NO:18), which was radiolabelled with [α-³²P] dCTP using a random priming method (Amersham, RPN, 1607, Mega prime kit) and used to screen a human fetal kidney 5'-STRETCH PLUS cDNA library (CLONTECH #HL1150x). Library screens were performed using Rapid Hybridisation Buffer (Amersham, RPn 1636) according to manufacturer's instructions and membranes washed at 65°C for 30 min in 0.1xSSC/0.1% (w/v) SDS. Two independent cDNA clones were obtained as lambda phage and subsequently subcloned and sequenced. Both clones (HFK-63 and HFK-66) contained 1.4 kilobase (kb) inserts that showed sequence similarity with murine NR6. The sequence and corresponding amino acid translation of HFK-66 is shown in SEQ ID NO:24.

The translation protein sequences of clone HFK-66 shows a high degree of sequence similarity with the mouse NR6.

### OLIGONUCLEOTIDES

UP1: 5'TCC AGG CAG CGG TCG GGG GAC AAC 3' [SEQ ID NO:26]
LP1: 5' TTG CTC ACA TCG TCC ACC ACC TTC 3' [SEQ ID NO:27]

### EXAMPLE 9

### Genomic Structure of Human NR6

Human genomic DNA clones encoding human NR6 was isoloated by screening a human genomic library (Lambda FIXJII Stratagene 946203) with radiolabelled oligonucleotides, 2199 and 2200 (see below). These oligonucleotides were designed based on human ESTs (Genbank Acc:R87407, Genbank Acc:H14009) that were identified from databases searched with murine NR6. Filters were hybridised overnight at 37°C in 6xSSC containing 2 mg/ml bovine serum albumin, 2 mg/ml Ficoll, 2mg/ml polyvinylpyrrolidone, 100 mM ATP, 10 mg/ml tRNA, 2 mM sodium pyrophosphate, 2 mg/ml salmon sperm DNA, 0.1% (w/v) SDS and 200 mg/ml sodium azide and washed at 65°C in 6 x SSC/0.1% SDS. Five independent genomic clones were obtained and sequenced. The extend of sequence obtained has determined that the clones overlap and exhibit a similar genomic structure to murine NR6. Exon coding regions are almost identical over the region covered by the genomic clones while intron coding regions differ, although the size of the introns are comparable. The extent of known overlap is shown in Fig. 5.

### OLIGONUCLEOTIDES:

2199: 5' CCC ACG CTT CTC ATC GGA TTC TCC CTG 3' [SEQ ID NO:36]
2200: 5' CAG TCC ACA CTG TCC TCC ACT CGG TAG 3' [SEQ ID NO:37]

### EXAMPLE 10

### Northern Blot Analysis of Human NR6 mRNA Expression

Clontech Multiple Tissue Northern Blots (Human MTN Blot, CLONTECH #7760-1, Human MTN Blot IV, CLONTECH #7766-I, Human Brain MTN Blot II, CLONTECH #7755-1, Human Brain MTN Blot III, CLONTECH #7750) were probed with a radiolabelled 3' human NR6 cDNA clone, HFK-66 (SEQ ID NO:24). The clone was labelled with [α-³²P] dCTP using a random priming method (Amersham, RPN 1607, Mega prime kit). Hybridisation was performed in Express Hybridisation Solution (CLONTECH H50910) for 3 hours at 67°C and membranes were washed in 0.1xSSC/0.1% w/v SDS at 50°C.

A 1.8 kb transcript was detected in a variety of human tissues encompassing reproductive, digestive and neural tissues. High levels were observed in the heart, placenta, skeletal muscle, prostate and various areas of the brain, lower levels were observed in the testis, uterus, small intestine and colon. Photographs showing these Northern blots are available upon request. This expression pattern differs from the expression pattern observed with murine NR6.

### EXAMPLE 11

### Mouse NR6 Expression Vectors

### pEF-FLAG/mNR6.1

The mature coding region of mouse NR6.1 was amplified using the PCR to introduce an in-frame *Asc* I restriction enzyme site at the 5' end of the mature coding region and an Mlu I site at the 3' end, using the following oligonucleotides:-
5' oligo 5'-AGCTGGCGCGCCTCCCGGGCGGATCGGGAGCCCAC-3' [SEQ ID NO:30]
3' oligo 5'-AGCTACGCGTTTAGAGTTTAGCCGGCAG-3' [SEQ ID NO:31]

The resulting PCR derived DNA fragment was then digested with *Asc* I and *Mlu* I and cloned into the *Mlu* I site of pEF-FLAG. Expression of NR6 is under the control of the polypeptide chain elongation factor 1α promoter as described (16) and results in the secretion, using the IL3 signal sequence from pEF-FLAG, of N-terminal FLAG-tagged NR6 protein.

pEF-FLAG was generated by modifying the expression vector pEF-BOS as follows:-
pEF-BOS (16) was digested with Xba I and a linker was synthesized that encoded the mouse IL3 signal sequence (MVLASSTTSIHTMLLLLLMLFHLGLQASIS) and the FLAG epitope (DYKDDDDK). *Asc* I and *Mlu* I restriction enzyme sites were also introduced as cloning sites. The sequence of the linker is as follows:-

The two oligonucleotides were annealed together and ligated into the Xba I site of pEF-BOS to give pEF-FLAG.

### pCOS1/FLAG/mNR6 & pCHO1/FLAG/mNR6

A DNA fragment containing the sequences encoding IL3 signal sequence/Flag/mNR6 and the poly(A) adenylation signal from human G-CSF cDNA, was excised from pEF-FLAG/mNR6 using the restriction enzyme EcoR I. This DNA fragment was then inserted into the EcoR I cloning site of pCOS1 and pCHO1.

The pCOSI and pCHO1 vectors were constructed as follows. pCH01 is also described in reference (17) but with a different selectable marker.

pCOS1 was prepared by digesting HEF-12h-gα1 (see Figure 24 of International Patent Publication No. WO 92/19759) with *Eco*RI and *Sma*I and ligating the digesting product iwht an *Eco*RI-*Not*I-*Bam*HI adaptor (Takara 4510). The resulting plasmid comprises an EFIα promoter/enhancer, Nco^{r} marker gene, SV40E, ori and an Amp^{r} marker gene.

pCH01 was constructed by digesting DHFR-PMh-grl (see Figure 25 of International Patent Publication No. WO 92/19759) with PvuI and Eco47III and ligating same with pCOSI digested with PvuI and Eco47III. The resulting vector, pCH01, comprises an EFIα promoter/enhancer, an DHFR marker gene, SV40E, Ori and a Amp^{r} gene.

### EXAMPLE 12

mRN6 has been expressed as an N' Flag tagged protein following transfection of CHO cells and as a C' Flag tagged protein following transfection of KUSA cells in both cases varying levels of dimeric and aggregated NR6 were secreted.

### EXAMPLE 13

### Murine NR6 expression

NR6 expression studies were conducted in murine Northern Blots. At the level of sensitivity used in the adult mouse, NR6 expression was detected in salivary gland, lung and testis. During embryonic development, NR6 is expressed in fetal tissues from day 10 of gestation through to birth. In cell lines, NR6 expression has been observed in the T-lymphoid line CTLL-2 as well as in FD-PyMT (FDC-P1 myeloid cells expressing polyoma midle T gene), and fibroblastoid cells including bone marrow and fetal liver stromal lines.

### EXAMPLE 14

### Expression, purification and characterisation of CHO and KUSA mNR6

The methods provide for the production of a dimeric form of CHO derived N' FLAG-mNR6 without refolding. All other methods are capable of producing NR6 and are encompassed by the present invention.

### A. Production of CHO derived N' FLAG-mNR6 (dimeric form)

### (i) Protein Production

To analyse structure and functional activity, a cDNA fragment containing the entire coding sequence of murine NR6 with an N-terminal FLAG (N' FLAG) sequence was cloned into the EcoR1 site of the expression vector pCHO1. For stable production of N-terminal FLAG-tagged NR6 the vector contains the DHFR (dihydrofolate reductase) gene as a selective marker with the NR6 gene under the control of an EF1a promoter. CHO cells were transfected with the construct using a polycationic liposome transfection reagent (Lipofectamine, GibcoBRL).

### (ii) Lipofectamine transfection method

Using six well tissue culture plates either 2 x 10⁵ KUSA cells in 2ml IMDM + 10% (v/v) FCS or 2 x 10⁵ CHO cells were cultured in 2ml α-MEM + 10% (v/v) FCS until 70% confluent. 2µg DNA diluted in 100µl OPTI-MEM I (Gibco BRL, USA) was mixed gently with 12µl lipofectamine diluted in 100µl OPTI-MEM I and incubated at room temperature for 30min to allow DNA complex formation. DNA complexes were gently diluted in a total volume of 1ml of OPTI-MEM I and overlaid onto washed KUSA or CHO cell monolayers. A further 1ml IMDM + 20% (v/v) FCS (KUSA cells) or 1ml α-MEM + 20% (v/v) FCS (CHO cells) was added to transfected cells after 5 hours. At 24 hours, the culture medium was replaced with fresh complete growth medium. At 48 hours after transfection, selection was applied. A methotrexate resistant clone secreting comparatively high levels of NR6 was selected and expanded for further analysis.

### (iii) Protein expression

CHO cells were grown to confluence in roller bottles in nucleoside free α-MEM + 10% (v/v) FCS. Selection was maintained by using 100 ng/ml Methotrexate in the conditioned media according to manufacturer instructions. Expression was monitored by Biosensor and harvesting found to be optimal at 3 to 4 days.

### B. Protein Analysis

### (i) Biosensor analysis

Expression and purification was monitored by Biosensor analysis (BiaCoreTM, Sweden) where anti FLAG peptide M2 antibody (Kodak Eastman, USA), specific for the FLAG peptide sequence was bound to the sensorchip. Fractions were analysed for binding to the sensor surface (resonance units) and the sample then removed from the surface using 50 mM Diethylamine pH 12.0 prior to analysis of the next fraction. Immobilisation and running conditions of the Biosensor follow the manufacturer's instructions.

### (ii) Protein Production

In order to generate and characterise NR6, conditioned media (2 L) produced by CHO cells was harvested after day 3, post confluence. Conditioned media was concentrated using diafiltration with a 10,000 molecular weight cut-off. (Easy flow, Sartorius, Aus). At a volume of 200 ml (i.e. 10 x concentrated) the sample was buffer exchanged into 20 mM Tris, 0.15M NaCl, 0.02% (v/v) Tween 20 pH 7.5 (Buffer A).

### (iii) Immunoprecipitation and Western Blot analysis of mNR6

Concentrated conditioned media (1ml) was immunoprecipitated with M2 affinity resin (20µl, Kodak Eastman). To examine the structural characterisation of mNR6 SDS PAGE was performed under reducing and non-reducing conditions. Separation was performed on NOVEX 4-20% (v/v) Tris/glycine gradient gels and protein transfered on PVDF membrane. Western blots were probed with biotinylated M2 antibody (primary, 1:500) and then streptavidin peroxidase (secondary, 1:3000). Samples were visualised by autoradiography using electrochemiluminescence (ECL, Dupont, USA).

By regressional analysis of prestained standards (BIORAD, Aus.) the molecular weight of the monomeric unit was calculated to be 65,000 daltons. Under non-reducing conditions the molecular weight was calculated to be 127,000 indicating that NR6 is a disulphide linked dimer. A tetrameric complex running at approximately 250,000 daltons was also observed. Although a band running at approximately 50,000 daltons was observed, no monomeric NR6 was detected under non-reducing conditions indicating that the majority of NR6 expressed in this system is disulphide linked.

### (iv) Affinity Chromatography of mNR6

Concentrated conditioned media (200 ml) was applied to M2 affinity resin (5ml) under gravity. To enhance recovery the unbound fraction was reapplied to the column four times prior to extensive washing of the column with 200 volumes of Buffer A. Biosensor analysis indicates that approximately 20% of the M2 binding originally present in the concentrate remains in the unbound fraction. The bound fraction was eluted from the column using an immunodesorbant (50 ml); actisep (Sterogene Labs, USA).

### (v) Ion exchange and Desalting of mNR6

In order to buffer exchange mNR6 prior to anion chromatography, 10 ml batches of the eluted fraction (50 ml) were applied to an XK column (400 x 26 mm I.D.). containing G25 sepharose (Pharmacia, Sweden). Chromatography was developed at 4 ml/min using an FPLC (Pharmacia, Sweden) equipped with an online UV280 and conductivity monitor. The mobile phase was 10 mM Tris, 0.1M NaCl, 0.02% v/v Tween, pH 8.0. 10 ml fractions were collected between 12.5 min and 25 min to optimise recovery and removal of salt. Fractions were analysed by Biosensor analysis and pooled according to binding.

All pooled active fractions were diluted with an equal volume of 20 mM Tris, 0.02% (v/v) Tween, pH 8.5 (Buffer B) and then loaded onto a Mono Q 5/5 (Pharmacia, Sweden) at a flow rate of 2 ml/min. The column was washed with buffer B. Elution was performed using a linear gradient between buffer B and buffer B containing 0.6M NaCl over 30 min at a flow rate of 1 ml/min. Fractions (1 minute) were collected and analysed on the Biosensor and also by SDS PAGE and Western blot analysis. Fractions 15 to 26 (approximately 0.4M NaCl) appear to contain the majority of mNR6 as indicated by the Biosensor.

### C. Production of CHO derived N' FLAG-mNR6 (monomeric form)

### (i) Protein Production

A cDNA fragment containing the entire coding sequence of murine NR6 with an N-terminal FLAG^{™} sequence was cloned into the expression vector pCHO1 for production of N-terminal FLAG-tagged protein. This vector contains a neomycin resistance gene with expression of the NR6 gene under the control of an EF1α promoter. This expression construct was transfected into CHO cells using Lipofectamine (Gibco BRL, USA) according to the manufacturer instructions. Transfected cells were cultured in IMDM + 10% (v/v) FCS with resistant cells selected in geneticin (600*µ*g/ml, Gibco BRL, USA). A neomycin resistant clone, secreting comparatively high levels of NR6 was selected and expanded for further analysis.

### (ii) Protein expression

N' FLAG-NR6 expressed in serum free conditioned media (10 litre) was harvested from transfected CHO and cells. Collected media was concentrated using a CH2 ultrafiltration system equipped with a S1Y10 cartridge (Amicion molecular weight cut-off 10,000). Preliminary examination of the expressed product under reducing and non-reducing SDS PAGE followed by western blot analysis was performed. Visualisation of the protein on Westerns was specific to the primary antibody anti FLAG M2. Under reducing conditions a band approximately at 65,000 daltons was observed. Under non-reducing conditions, dimer and larger molecular weight aggregates were observed. These are disulphide linked monomers as they are not present in the reducing gel. Small amounts of monomer appear to be present in non-reducing gels.

### (iii) Affinity Chromatography of NR6

Concentrated conditioned media was applied to an anti FLAG M2 affinity resin (100 x 16 mm I.D.). After washing the unbound proteins off the column, the bound proteins were eluted using FLAG peptide (60µg/ml) in PBS.

### (iv) Ion Exchange Chromatography of NR6

Eluted fractions from affinity column were dialysed overnight against 20 mM Tris-HC1 pH 8.5 (buffer C) containing 50 mM Dithiothretol (DTT) using 25,000 cut-off dialysis tubing (Spectra/Por7, Spectrum). The dialysed fractions were loaded onto Mono Q 5/5 (Pharmacia. Sweden) previously equilibrated with buffer C containing 5 mM DTT. Chromatography was developed using a linear gradient between buffer C and buffer C containing 1.0 M NaCl at a flow rate of 0.5 ml / min.

### (v) Refolding of NR6

Fractions containing NR6 from the Mono Q were adjusted to 50 mM DTT and left overnight at 4°C. To initiated refolding the sample was then dialysed against 50 mM Tris-HCl (pH 8.5), 2 M Urea, 0.1% (v/v) Tween 20, 10 mM Glutathione (reduced) and 2 mM Glutathione (oxidised) at a final protein concentration of 100 µg/ml. Folding was carried out at ambient temperature with one change of the buffer over 24 hours.

### (v) Reversed Phase High Performance Liquid Chromatography (RP-HPLC)

The folded product was further purified by RP-HPLC using a Vydac C4 resin (250 x 4.6 mm I.D.) previously equilibrated with 0.1% (v/v) Trifluoroacetic acid (TFA). Elution was carried out using a linear gradient from 0 to 80% (v/v) acetonitrile / 0.1% (v/v) TFA at a flow rate of 1 ml per minute.

### D. pCHO1/NR6/FLAG

In order to determine the native N termini of NR6, a C terminal FLAG NR6 CHO cell line was established.

The plasmid pKUSA166 (murine NR6 cDNA cloned into the EcoR I site of pBLUESCRIPT) was digested with BamH I to remove the sequences encoding the last 15 amino acids of murine NR6. Synthetic oligonucleotides which encode the 3' end of mouse NR6 followed by the FLAG peptide tag were annealed and ligated into the BamH I site of pKUSA166. The sequence of the oligonucleotides was as follows:-

The 5' end of the linker introduces a silent mutation (CTG > TTG), to destroy the 5' BamH I site upon insertion of the linker. The NR6 cDNA (with native signal sequence) with the C-terminal FLAG was cut out of pKUSA166 with EcoR I and BamH I and cloned into the EcoR I - BamH I cloning sites of pCHO-1. This vector results in the secretion of NR6 protein with a C-terminal flag tag (C' FLAG-mRN6).

This vector results in the secretion of NR6 protein from KUSA cells. The vector pCHO1 has been previously described in (17) although with a different secretable marker.

### (i) Production of polyclonal NR6 antiserum

The following peptide from the N terminal area of NR6 was chosen for production of polyclonal antiserum to NR6
VISPQDPTLLIGSSLQATCSIHGDTP [SEQ ID NO:39]

The peptide was conjugated to KLH and injected into rabbits. Production and purification of the polyclonal antibody specific to the NR6 peptide sequence follows standard methods.

### (ii) Protein expression

KUSA cells transfected with cDNA of C terminal tagged mNR6 were grown to confluence in flasks (800ml) using IMDM media containing 10% (v/v) FBS. Conditioned media (100 ml) was harvested 3 -4 days post confluence.

### (iii) Characterisation of NR6 by Immunoprecipitation and Western blotting

In order to establish that NR6 with the predicted sequence is produced in KUSA cells transfected with the cDNA, western blot analysis using both M2 antibody and purified NR6 specific rabbit antibody were performed. Conditioned media (1 to 5 ml) was immunoprecipitated with M2 affinity resin (10-20 µl). Then after sufficient time for binding, the beads were washed with MT-PBS and subsequently NR6 eluted with 100 µg/ml FLAG peptide (40 µl, (1, 5 minute incubation). The sample was then subjected to reducing and non reducing SDS PAGE followed by western blot analysis. Both purified NR6 polyclonal antibody (purified by protein G) and M2 antibody recognise a band under reducing conditions of a molecular weight size approximately 65,000 daltons. Since the two antibodies reconising resides at the N terminus and C terminus it is reasonable to assume that full length NR6 is produced. Biotinylation of the respective antibodies by standard methods reduces the background. Under non-reducing conditions polyclonal NR6 bind antibodies to a band of a molecular weight of approximately 127,000, consistent with a dimeric NR6 disulphide linked form. Minor components of tetrameric NR6 are present, no monomeric NR6 is evident using polyclonal NR6 antibodies.

### EXAMPLE 15

### Generation of NR6 knockout mice

To construct the NR6 targeting vector, 4.1kb of genomic NR6 DNA containing exons 2 through to 6 was deleted and replaced with G418-resistance cassette, leaving 5' and 3' NR6 arms of 2.9 and 4.5 kb respectively. A 4.5 kb Xhol fragment of the murine genomic NR6 clone 2.2 (Figure 3) containing exons 7, 8 and 3' flanking sequence was subcloned into the XhoI site of pBluescript generating pBSNR6Xho4.5. A 2.9kb NotI-Stul fragment within NR6 intron 1 from the same genomic clone was inserted into NotI and EcoRV digested pBSNR6Xho4.5 creating pNR6-Ex2-6. This plasmid was digested with ClaI, which was situated between the two NR6 fragments, and following blunt ending, ligated with a blunted 6kb HindIII fragment from placZneo, which contains the lacZgene and a PGKneo cassette, to generate the final targeting vector, pNR61acZneo. pNR61acZneo was linearised with NotI and electroporated into W9.5 embryonic stem cells. After 48 hours, transfected cells were selected in 175 µg/ml G418 and resistant clones picked and expanded after a further 8 days.

Clones in which the targetting vector had recombined with the endogenous NR6 gene were identified by hybridising SpeI-digested genomic DNA with a 0.6 kb XhoI-StuI fragment from genomic NR6 clone 2.2. This probe (probe A, Figure 4), which is located 3' to the NR6 sequences in the targeting vector, distinguished between the endogenous (9.9 kb) and targeted (7.1 kb) NR6 loci (Figure 5).

Genomic DNA was digested with SpeI for 16hrs at 37°C, electrophoresed through 0.8% (w/v) agarose, transferred to nylon membranes and hybridised to ³²P-labelled probe in a solution containing 0.5M sodium phosphate, 7% (w/v) SDS, 1mM EDTA and washed in a solution containing 40mM sodium posphate, 1% (w/v) SDS at 65°C. Hybridising bands were visualised by autoradiography for 16 hours at -70°C using Kodak XAR-5 film and intensifying screens. Two targeted ES cell clones, W9.5NR6-2-44 and W9.5NR6-4-2, were injected into C57B1/6 blastocysts to generate chimeric mice. Male chimeras were mated with C57B1/6 females to yield NR6 heterozygotes which were subsequently interbred to produce wild-type (NR6^{+/+}), heterozygous (NR6^{+/-}) and mutant (NR6^{-/-}) mice. The genotypes of offspring were determined by Southern Blot analysis of genomic DNA extracted from tail biopsies.

Genotyping of mice at weaning from matings between NR^{+/-} heterozygous mice derived from both targated ES cell clones revealed an absence of homozygous NR6^{-/-} mutants. As no unusual loss of mice was observed between birth and weaning, this suggest that lack of NR6 is lethal during embryonic development or immediately after birth. Genotyping of embryonic tissues at various stages of development suggests that death occurs late in gestation (beyond day 16) or at birth.

### EXAMPLE 16

### Oligonucleotides

| |
|---|
| 1943: |
| 5' GTC CAA GTG CGT TGT AAC CCA 3' |
| 2070: |
| 5' GCT GAG TGT GCG CTG GGT CTC ACC 3' |
| 2057: |
| 5' GGC TCC ACT CGC TCC AGA 3' |

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### BIBLIOGRAPHY:

1. Du, X.X. and Williams, D.A. (1994) Blood 83: 2023-2030.
2. Yang, Y.C. and Yin, T. (1992) Biofactors 4: 15-21.
3. Paul, S.R., Bennett, F., Calvetti, J.A., Kelleher, K., Wood. C.R.. O'Hara. R.J.J., Leary. A.C., Sibley, B., Clark, S.C., Williams, D.A. and Yang, Y.-C. (1990) Proc. Natl. Acad. Sci. USA 87: 7512.
4. Musashi, M., Clark, S.C., Sudo, T., Urdal, D.L., and Ogawa, M. (1991) Blood 78: 1448-1451.
5. Schibler, K.R., Yang, Y.C. and Christensen, R.D. (1992) Blood 80: 900-3.
6. Tsuji, K., Lyman, S.D., Sudo, T., Clark, S.C., and Ogawa, M. (1992) Blood 79: 2855-60.
7. Burstein, S.A., Mei, R.L., Henthorn, J., Friese, P. and turner, K. (1992) J. Cell. Physiol. 153: 305-12.
8. Hangoc, G., Yin, T., Cooper, S., Schendel, P., Yang, Y.C. and Broxmeyer, H.E. (1993) Blood 81: 965-72.
9. Teramura, M., Kobayashi, S., Hoshino, S., Oshimi, K. and Mizoguchi, H. (1992) Blood 79: 327-31.
10. Yonemura, Y., Kawakita, M., Masuda, T., Fujimoto, K., Kato, K. and Takatsuki, K. (1992) Exp. Hematol. 20: 1011-6.
11. Baumann, H. and Schendel, P. (1991) J. Biol. Chem. 266: 20424-7.
12. Kawashima, I., Ohsumi, J., Mita-Honjo, K., Shimoda-Takano, K., Ishikawa, H., Sakakibara, S., Miyadai, K. and Takiguchi, Y. (1991) Febs. Lett. 283: 199-202.
13. Keller, D.C., Du, X.X., Srour, E.f., Hoffman, R. and Williams, D.A. (1993) Blood 82: 1428-35.
14. Sambrook et al (1989) Cloning: A Laboratory Manual. Cold Spring Harbour Laboratory, Cold Spring Harbour, NY.
15. Chirgwin et al (1979) Biochemistry 18: 5294-5299.
16. Mizushima and Nagata (1990) Nucl. Acids Res., 18: 5322.
17. FEBS Lett (1994) 356: 244-248.
18. Bazan, J.F. (1990) Proc Natl Acad Sci USA, 87, 6934-8
19. de Vos, A.M., Ultsch. M. and Kossiakoff. A.A. (1992) Science, 255, 306-12
20. Layton, M.J., Cross, B.A., Metcalf, D., Ward, L.D., Simpson, R.J. and Nicola, N.A. (1992) Proceedings of the National Academy of Sciences of the United States of America 89: 8616-8620
21. Taga, T., Hibi, M., Hirata, T., Tamasaki, K., Tasukawa, K., Matsuda, T., Hirano, T. and Kishimoto, T. (1989) Cell 58: 573-581
22. Merberg, D.M., Wolf, S.F. and Clark, S.C. (1992) Sequence similarity between NKSF and the IL-6/G-CSF family (1992) Immunology Today 13: 77-78
23. Cearing, D.P. and Cosman, D. (1991) Cell 66:9-10
24. Wrighton, N.C., Farrell, F.X., Chang, R., Kashyap, A.K., Barbone, F.P., Mulcahy, L.S., Johnson, D.L., Barrett, R.W., Jolliffe, L.K. and Dower, W.J. (1996) Science 273: 458-464.
25. Cwirla, S.E., Balasubramanian, P., Duffin, D.J., Wagstrom, C.R., Gates, C.M., Singer, S.C., Davis, A.M., Tansik, R.L., Mattheakis, L.C., Boytos, C.M., Schatz, P.J., Baccanari, D.P., Wrighton, N.C., Barret, R.W. and Dower, W.J. (1997) Science 276: 1696--9, 1997
26. Samuel Davis et al (1996) Cell 87:1161-1169.
27. Chitra Suri et al (1996) Cell 87: 1171-1180.
28. Nicholas C. Wrighton et al (1996) Science 273: 458-463.
29. Oded Livnah et al (1996) Science 273: 464-471.
30. Cwirla, Steven E. et al (1997) Science 276: 1696-1699.

## Claims

1. A nucleic acid molecule comprising a sequence of nucleotides encoding a haemopoietin receptor, or the complementary sequence thereto, wherein said encoding sequence is as set forth in SEQ ID NO: 12, 14, 16, 18, 24, 28 or 38 or a nucleotide sequence having at least 60% similarity to the nucleotide sequence set forth in SEQ ID NO: 12, 14, 16, 18, 24, 28 or 38, respectively, or a nucleotide sequence capable of hybridising thereto under high stringency conditions of at least 31% v/v to at least 50% v/v formamide and from at least 0.01M to at least 0,15M salt for hybridisation, and at least 0.01M to at least 0.15M salt for washing at 42°C.

2. A nucleic acid molecule according to claim 1 wherein said haemopoietin receptor is of murine origin.

3. A nucleic acid molecule according to claim 1 wherein said haemopoietin receptor is of human origin.

4. An expression vector comprising a nucleic acid molecule as claimed in any one of claims 1 to 3.

5. An isolated nucleic acid molecule comprising (a) a sequence of nucleotides encoding a haemopoietin receptor having an amino acid sequence as set forth in SEQ ID NO: 13, 15, 17, 19, 25 or 29; (b) a sequence of nucleotides encoding a haemopoietin receptor having an amino acid sequence which has at least 50% similarity to the amino acid sequence set forth in (a); or (c) a fragment of the nucleotide sequence of (a) encoding a haemopoietin receptor.

6. An isolated haemopoietin receptor comprising the amino acid sequence as set forth in SEQ ID NO: 13, 15, 17, 19, 25 or 29.

7. A pharmaceutical composition comprising a haemopoietin receptor in soluble form and one or more pharmaceutically acceptable carriers and/or diluents wherein said haemopoietin receptor comprises the amino acid sequence:
(i) encoded by a nucleotide sequence selected from the nucleotide sequence set forth in SEQ ID NO: 12 or 14 or 16 or 18 or 24 or 28 or 38 or a nucleotide sequence having at least 60% similarity to the nucleotide sequence set forth in SEQ ID NO: 12 or 14 or 16 or 18 or 24 or 28 or 38 and which is capable of hybridising thereto under high stringency conditions of at least 31% v/v to at least 50% v/v formamide and from at least 0.01M to at least 0.15M salt for hybridisation, and at least 0.01M to at least 0.15M salt for washing at 42°C; and
(ii) as encoded by a sequence of nucleotides defined in claim 5.

8. An isolated antibody or a preparation of antibodies to a haemopoietin receptor, said haemopoietin receptor comprising the amino acid sequence:
(i) encoded by a nucleotide sequence selected from the nucleotide sequence set forth in SEQ ID NO: 12 or 14 or 16 or 18 or 24 or 28 or 38 or a nucleotide sequence having at least 60% similarity to the nucleotide sequence set forth in SEQ ID NO: 12 or 14 or 16 or 18 or 24 or 28 or 38 and which is capable of hybridising thereto under high stringency conditions of at least 31% v/v to at least 50% v/v formamide and from at least 0.01M to at least 0.15M salt for hybridisation, and at least 0.01M to at least 0.15M salt for washing at 42°C; and
(ii) as encoded by a sequence of nucleotides defined in claim 5.

9. The antibody of claim 8 wherein said antibody is a polyclonal or a monoclonal antibody or a fragment thereof.

10. The antibody of claim 8 or 9 wherein said antibody is a synthetic antibody or a hybrid antibody.

11. The antibody of any one of claims 8 to 10 for use in therapy.

12. A method for detecting a haemopoietin receptor encoded by a sequence of nucleotides as defined in claim 1 or 5 or having an amino acid sequence as defined in claim 6 in a biological sample from a subject, said method comprising contacting said biological sample with an antibody specific for said haemopoietin receptor for a time and under conditions sufficient for an antibody-haemopoietin receptor complex to form and then detecting said complex.

13. A genetic construct comprising a vector portion and a gene portion, wherein said gene is as defined in any one of claims 1 to 3, and wherein said gene portion is capable of encoding a novel haemopoietin receptor or a functional or immunologically interactive-derivative thereof.

14. A prokaryotic or eukaryotic cell containing an expression vector as defined in claim 4 or a genetic construct as claimed in claim 13.

15. The use of an isolated haemopoietin receptor as defined in claim 6 in the manufacture of a medicament for the treatment of conditions resulting from defective or deficient haemopoietin receptors as defined in claim 6.

## Patentansprüche

1. Nukleinsäuremolekül, umfassend eine Sequenz von Nukleotiden, die einen Hämatopoetinrezeptor kodiert, oder die dazu komplementäre Sequenz, wobei die kodierende Sequenz wie in SEQ ID NO: 12, 14, 16, 18, 24, 28 oder 38 dargestellt, oder eine Nukleotidsequenz mit mindestens 60 % Ähnlichkeit zu der in SEQ ID NO: 12, 14, 16, 18, 24, 28 bzw. 38 dargestellten Nukleotidsequenz ist, oder eine Nukleotidsequenz ist, die imstande ist, unter hochstringenten Bedingungen von mindestens 31 Vol.-% bis mindestens 50 Vol.-% Formamid und von mindestens 0,01 M bis mindestens 0,15 M Salz zur Hybridisierung und mindestens 0,01 M bis mindestens 0,15 M Salz zum Waschen bei 42 °C damit zu hybridisieren.

2. Nukleinsäuremolekül nach Anspruch 1, wobei der Hämatopoetinrezeptor murinen Ursprungs ist.

3. Nukleinsäuremolekül nach Anspruch 1, wobei der Hämatopoetinrezeptor menschlichen Ursprungs ist.

4. Expressionsvektor, umfassend ein Nukleinsäuremolekül nach einem der Ansprüche 1 bis 3.

5. Isoliertes Nukleinsäuremolekül, umfassend (a) eine Sequenz von Nukleotiden, die einen Hämatopoetinrezeptor mit einer Aminosäurensequenz, wie in SEQ ID NO: 13, 15, 17, 19, 25 oder 29 dargestellt, kodiert; (b) eine Sequenz von Nukleotiden, die einen Hämatopoetinrezeptor mit einer Aminosäuresequenz kodiert, die mindestens 50 % Ähnlichkeit mit der in (a) dargestellten Aminosäuresequenz besitzt; oder (c) ein Fragment der Nukleotidsequenz von (a), das einen Hämatopoetinrezeptor kodiert.

6. Isolierter Hämatopoetinrezeptor, umfassend die in SEQ ID NO: 13, 15, 17, 19, 25 oder 29 dargestellte Aminosäuresequenz.

7. Pharmazeutische Zusammensetzung, umfassend einen Hämatopoetinrezeptor in löslicher Form und einen oder mehrere pharmazeutisch akzeptable Träger und/oder Verdünnungsmittel, wobei der Hämatopoetinrezeptor die Aminosäuresequenz umfasst, die:
(I) von einer Nukleotidsequenz, die ausgewählt ist unter der in SEQ ID NO: 12 oder 14 oder 16 oder 18 oder 24 oder 28 oder 38 dargestellten Nukleotidsequenz oder von einer Nukleotidsequenz, die mindestens 60 % Ähnlichkeit zu der in SEQ ID NO: 12 oder 14 oder 16 oder 18 oder 24 oder 28 oder 38 dargestellten Nukleotidsequenz besitzt und unter hochstringenten Bedingungen von mindestens 31 Vol.-% bis mindestens 50 Vol.-% Formamid und von mindestens 0,01 M bis mindestens 0,15 M Salz zur Hybridisierung und mindestens 0,01 M bis mindestens 0,15 M Salz zum Waschen bei 42 °C damit zu hybridisieren, kodiert wird; und die
(II) von einer in Anspruch 5 definierten Nukleotidsequenz kodiert wird.

8. Isolierter Antikörper oder Antikörperpräparation gegen einen Hämatopoetinrezeptor, wobei der Hämatopoetinrezeptor die Aminosäurensequenz umfasst, die:
(I) von einer Nukleotidsequenz, die ausgewählt ist unter der in SEQ ID NO: 12 oder 14 oder 16 oder 18 oder 24 oder 28 oder 38 dargestellten Nukleotidsequenz, oder von einer Nukleotidsequenz, die mindestens 60 % Ähnlichkeit zu der in SEQ ID NO: 12 oder 14 oder 16 oder 18 oder 24 oder 28 oder 38 dargestellten Nukleotidsequenz besitzt und unter hochstringenten Bedingungen von mindestens 31 Vol.-% bis mindestens 50 Vol.-% Formamid und von mindestens 0,01 M bis mindestens 0,15 M Salz zur Hybridisierung und mindestens 0,01 M bis mindestens 0,15 M Salz zum Waschen bei 42 °C damit zu hybridisieren, kodiert wird; und die
(II) von einer in Anspruch 5 definierten Nukleotidsequenz kodiert wird.

9. Antikörper nach Anspruch 8, wobei der Antikörper ein polyklonaler oder monoklonaler Antikörper oder ein Fragment davon ist.

10. Antikörper nach Anspruch 8 oder 9, wobei der Antikörper ein synthetischer Antikörper oder ein Hybridantikörper ist.

11. Antikörper nach einem der Ansprüche 8 bis 10 zur therapeutischen Verwendung.

12. Verfahren zur Detektion eines Hämatopoetinrezeptors, der von einer Nukleotidsequenz wie in Anspruch 1 oder 5 definiert kodiert wird oder eine Aminosäuresequenz wie in Anspruch 6 definiert besitzt, in einer biologischen Probe aus einem Individuum, wobei das Verfahren umfasst, dass man die biologische Probe über einen Zeitraum und unter Bedingungen, die ausreichend sind, die Bildung eines Antikörper-Hämatopoetinrezeptor-Komplexes zu erlauben, mit einem für den Hämopoietinrezeptor spezifischen Antikörper in Kontakt bringt, und dann den Komplex detektiert.

13. Genetisches Konstrukt, umfassend einen Vektoranteil und einen Genanteil, wobei das Gen wie in einem der Ansprüche 1 bis 3 definiert ist, und wobei der Genanteil imstande ist, einen neuartigen Hämatopoetinrezeptor oder ein funktional oder immunologisch interaktives Derivat davon zu kodieren.

14. Prokaryontische oder eukaryontische Zelle, enthaltend einen Expressionsvektor nach Anspruch 4 oder ein genetisches Konstrukt nach Anspruch 13.

15. Verwendung eines isolierten Hämatopoetinrezeptors nach Anspruch 6 zur Herstellung eines Medikaments zur Behandlung von Krankheitszuständen, die sich aus defekten oder defizienten Hämatopoetinrezeptoren, wie in Anspruch 6 definiert, ergeben.

## Revendications

1. Molécule d'acide nucléique comprenant une séquence de nucléotides codant pour un récepteur d'hématopoïétine, ou la séquence complémentaire de celle-ci, dans laquelle ladite séquence codante est telle qu'exposée dans SEQ ID N° : 12, 14, 16, 18, 24, 28 ou 38 ou une séquence de nucléotides ayant une similarité d'au moins 60 % avec la séquence de nucléotides exposée dans SEQ ID N° : 12, 14, 16, 18, 24, 28 ou 38, respectivement, ou une séquence de nucléotides capable de s'hybrider à celle-ci dans des conditions de stringence élevée d'au moins 31 % v/v à au moins 50 % v/v de formamide et d'au moins 0,01 M à au moins 0,15 M de sel pour l'hybridation, et d'au moins 0,01 M à au moins 0,15 M de sel pour un lavage à 42°C.

2. Molécule d'acide nucléique selon la revendication 1, dans laquelle ledit récepteur d'hématopoïétine est d'origine murine.

3. Molécule d'acide nucléique selon la revendication 1, dans laquelle ledit récepteur d'hématopoïétine est d'origine humaine.

4. Vecteur d'expression comprenant une molécule d'acide nucléique selon l'une quelconque des revendications 1 à 3.

5. Molécule d'acide nucléique isolée comprenant (a) une séquence de nucléotides codant pour un récepteur d'hématopoïétine ayant une séquence d'acides aminés telle qu'exposée dans SEQ ID N° : 13, 15, 17, 19, 25 ou 29 ; (b) une séquence de nucléotides codant pour un récepteur d'hématopoïétine ayant une séquence d'acides aminés qui présente une similarité d'au moins 50 % avec la séquence d'acides aminés exposée dans (a) ; ou (c) un fragment de la séquence de nucléotides de (a) codant pour un récepteur d'hématopoïétine.

6. Récepteur d'hématopoïétine isolé comprenant la séquence d'acides aminés exposée dans SEQ ID N° : 13, 15, 17, 19, 25 ou 29.

7. Composition pharmaceutique comprenant un récepteur d'hématopoïétine sous une forme soluble et un ou plusieurs vecteurs et/ou diluants pharmaceutiquement acceptables, dans laquelle ledit récepteur d'hématopoïétine comprend la séquence d'acides aminés :
(i) codée par une séquence de nucléotides choisie parmi les séquences de nucléotides exposées dans SEQ ID N° : 12 ou 14 ou 16 ou 18 ou 24 ou 28 ou 38 ou une séquence de nucléotides ayant une similarité d'au moins 60 % avec la séquence de nucléotides exposée dans SEQ ID N° : 12 ou 14 ou 16 ou 18 ou 24 ou 28 ou 38, et qui est capable de s'hybrider à celle-ci dans des conditions de stringence élevée d'au moins 31 % v/v à au moins 50 % v/v de formamide et d'au moins 0,01 M à au moins 0,15 M de sel pour l'hybridation, et d'au moins 0,01 M à au moins 0,15 M de sel pour un lavage à 42°C ; et
(ii) telle que codée par une séquence de nucléotides définie dans la revendication 5.

8. Anticorps isolé ou une préparation d'anticorps dirigé(s) contre un récepteur d'hématopoïétine, ledit récepteur d'hématopoïétine comprenant la séquence d'acides aminés :
(i) codée par une séquence de nucléotides choisie parmi les séquences de nucléotides exposées dans SEQ ID N° : 12 ou 14 ou 16 ou 18 ou 24 ou 28 ou 38 ou une séquence de nucléotides ayant une similarité d'au moins 60 % avec la séquence de nucléotides exposée dans SEQ ID N° : 12 ou 14 ou 16 ou 18 ou 24 ou 28 ou 38, et qui est capable de s'hybrider à celle-ci dans des conditions de stringence élevée d'au moins 31 % v/v à au moins 50 % v/v de formamide et d'au moins 0,01 M à au moins 0,15 M de sel pour l'hybridation, et d'au moins 0,01 M à au moins 0,15 M de sel pour un lavage à 42 °C; et
(ii) telle que codée par une séquence de nucléotides définie dans la revendication 5.

9. L'anticorps selon la revendication 8, dans lequel ledit anticorps est un anticorps polyclonal ou monoclonal ou un fragment de ceux-ci.

10. Anticorps selon la revendication 8 ou 9, dans lequel ledit anticorps est un anticorps synthétique ou un anticorps hybride.

11. Anticorps selon l'une quelconque des revendications 8 à 10, pour utilisation en thérapie.

12. Procédé de détection d'un récepteur d'hématopoïétine codé par une séquence de nucléotides telle que définie dans la revendication 1 ou 5, ou ayant une séquence d'acides aminés telle que définie dans la revendication 6, dans un échantillon biologique d'un sujet, ledit procédé comprenant la mise en contact dudit échantillon biologique avec un anticorps spécifique dudit récepteur d'hématopoïétine pour un temps et dans des conditions suffisantes pour la formation d'un complexe anticorps-récepteur d'hématopoïétine, puis la détection dudit complexe.

13. Construit génétique comprenant une partie de vecteur et une partie de gène, dans lequel ledit gène est tel que défini dans l'une quelconque des revendications 1 à 3, et dans lequel ladite partie de gène est capable de coder pour un nouveau récepteur d'hématopoïétine ou un dérivé fonctionnel ou immunologiquement interactif de celui-ci.

14. Cellule procaryote ou eucaryote contenant un vecteur d'expression tel que défini dans la revendication 4, ou un construit génétique selon la revendication 13.

15. L'utilisation d'un récepteur d'hématopoïétine isolé tel que défini dans la revendication 6, dans la fabrication d'un médicament pour le traitement de conditions résultant de récepteurs d'hématopoïétine défectueux ou déficients, tel que défini dans la revendication 6.


### SEQUENCE LISTING

(1) GENERAL INFORMATION:
   (i) APPLICANT:(Other than US) AMRAD OPERATIONS PTY LTD (US only) Douglas James HILTON, Nicos Antony NICOLA, Alison FARLEY, Tracey WILLSON, Jian-Guo ZHANG, Warren ALEXANDER, Steven RAKAR, Louis FABRI, Tetsuo KOJIMA, Masatsugu MAEDA, Yasumfumi KIKUCHI, Andrew NASH
   (ii) TITLE OF INVENTION: A NOVEL HAEMOPOIETIN RECEPTOR AND GENETIC SEQUENCES ENCODING SAME
   (iii) NUMBER OF SEQUENCES: 39
   (iv) CORRESPONDENCE ADDRESS:
      (A) ADDRESSEE: DAVIES COLLISON CAVE
      (B) STREET: 1 LITTLE COLLINS STREET
      (C) CITY: MELBOURNE
      (D) STATE: VICTORIA
      (E) COUNTRY: AUSTRALIA
      (F) ZIP: 3000
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25
   (vi) CURRENT APPLICATION DATA:
      (A) APPLICATION NUMBER:
         PCT INTERNATIONAL APPLICATION
      (B) FILING DATE: 11-SEP-1997
   (vi) PRIOR APPLICATION DATA:
      (A) APPLICATION NUMBER: PO2246/96
      (B) FILING DATE: 11-SEP-1996
   (viii) ATTORNEY/AGENT INFORMATION:
      (A) NAME: HUGHES DR, E JOHN L.
      (C) REFERENCE/DOCKET NUMBER: EJH/AF
   (ix) TELECOMMUNICATION INFORMATION:
      (A) TELEPHONE: +61 3 9254 2777
      (B) TELEFAX: +61 3 9254 2770
(2) INFORMATION FOR SEQ ID NO:1:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:1:
      Trp Ser Xaa Trp Ser
(2) INFORMATION FOR SEQ ID NO:2:
   (i) SEQUENCE CHARACTERISTICS:
      (A) LENGTH: base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:2:
      ACTCGCTCCA GATTCCCGCC TTTT 24
(2) INFORMATION FOR SEQ ID NO:3:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:3:
      TCCCGCCTTT TTCGACCCAT AGAT 24
(2) INFORMATION FOR SEQ ID NO:4:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:4:
      GGTACTTGGC TTGGAAGAGG AAAT 24
(2) INFORMATION FOR SEQ ID NO:5:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:5:
      CGGCTCACGT GCACGTCGGG TGGG 24
(2) INFORMATION FOR SEQ ID NO:6:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO : 6:
      AGCTGCTGTT AAAGGGCTTC TC 22
(2) INFORMATION FOR SEQ ID NO:7:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:7:
      (A/G)CTCCA(A/G)TC(A/G) CTCCA 15
(2) INFORMATION FOR SEQ ID NO:8:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:8:
      (A/G) CTCCA (C/T) TC (A/G) CTCCA 15
(2) INFORMATION FOR SEQ ID NO:9:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:9:
      AAGTGTGACC ATCATGTGGA C 21
(2) INFORMATION FOR SEQ ID NO:10:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:10:
      GGAGGTGTTA AGGAGGCG 18
(2) INFORMATION FOR SEQ ID NO:11:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:11:
      ATGCCCGCGG GTCGCCCG 18
(2) INFORMATION FOR SEQ ID NO:12:
   (i) SEQUENCE CHARACTERISTICS:
      (A) LENGTH: 1506 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY : CDS
      (B) LOCATION: 1..1242
   (xi) SEQUENCE DESCRIPTION: SEQ ID NO:12:
(2) INFORMATION FOR SEQ ID NO:13:
   (i) SEQUENCE CHARACTERISTICS:
      (A) LENGTH: 413 amino acids
      (B) TYPE: amino acid
      (D) TOPOLOGY: linear
   (ii) MOLECULE TYPE: protein
   (xi) SEQUENCE DESCRIPTION: SEQ ID NO : 13 :
   Met Pro Ala Gly Arg Pro Gly Pro Val Ala Gln Ser Ala Arg Arg Pro
   1 5 10 15
   Pro Arg Pro Leu Ser Ser Leu Trp Ser Pro Leu Leu Leu Cys Val Leu
   20 25 30
   Gly Val Pro Arg Gly Gly Ser Gly Ala His Thr Ala Val Ile Ser Pro
   35 40 45
   Gln Asp Pro Thr Leu Leu Ile Gly Ser Ser Leu Gln Ala Thr Cys Ser
   50 55 60
   Ile His Gly Asp Thr Pro Gly Ala Thr Ala Glu Gly Leu Tyr Trp Thr
   65 70 75 80
   Leu Asn Gly Arg Arg Leu Pro Ser Glu Leu Ser Arg Leu Leu Asn Thr
   85 90 95
   Ser Thr Leu Ala Leu Ala Leu Ala Asn Leu Asn Gly Ser Arg Gln Gln
   100 105 110
   Ser Gly Asp Asn Leu Val Cys His Ala Arg Asp Gly Ser Ile Leu Ala
   115 120 125
   Gly Ser Cys Leu Tyr Val Gly Leu Pro Pro Glu Lys Pro Phe Asn Ile
   130 135 140
   Ser Cys Trp Ser Arg Asn Met Lys Asp Leu Thr Cys Arg Trp Thr Pro
   145 150 155 160
   Gly Ala His Gly Glu Thr Phe Leu His Thr Asn Tyr Ser Leu Lys Tyr
   165 170 175
   Lys Leu Arg Trp Tyr Gly Gln Asp Asn Thr Cys Glu Glu Tyr His Thr
   180 185 190
   Val Gly Pro His Ser Cys His Ile Pro Lys Asp Leu Ala Leu Phe Thr
   195 200 205
   Pro Tyr Glu Ile Trp Val Glu Ala Thr Asn Arg Leu Gly Ser Ala Arg
   210 215 220
   Ser Asp Val Leu Thr Leu Asp Val Leu Asp Val Val Thr Thr Asp Pro
   225 230 235 240
   Pro Pro Asp Val His Val Ser Arg Val Gly Gly Leu Glu Asp Gln Leu
   245 250 255
   Ser Val Arg Trp Val Ser Pro Pro Ala Leu Lys Asp Phe Leu Phe Gln
   260 265 270
   Ala Lys Tyr Gln Ile Arg Tyr Arg Val Glu Asp Ser Val Asp Trp Lys
   275 280 285
   Val Val Asp Asp Val Ser Asn Gln Thr Ser Cys Arg Leu Ala Gly Leu
   290 295 300
   Lys Pro Gly Thr Val Tyr Phe Val Gln Val Arg Cys Asn Pro Phe Gly
   305 310 315 320
   Ile Tyr Gly Ser Lys Lys Ala Gly Ile Trp Ser Glu Trp Ser His Pro
   325 330 335
   Thr Ala Ala Ser Thr Pro Arg Ser Glu Arg Pro Gly Pro Gly Gly Gly
   340 345 350
   Val Cys Glu Pro Arg Gly Gly Glu Pro Ser Ser Gly Pro Val Arg Arg
   355 360 365
   Glu Leu Lys Gln Phe Leu Gly Trp Leu Lys Lys His Ala Tyr Cys Ser
   370 375 380
   Asn Leu Ser Phe Arg Leu Tyr Asp Gln Trp Arg Ala Trp Met Gln Lys
   385 390 395 400
   Ser His Lys Thr Arg Asn Gln Val Leu Pro Ala Lys Leu
   405 410
(2) INFORMATION FOR SEQ ID NO:14:
   (i) SEQUENCE CHARACTERISTICS:
      (A) LENGTH: 1549 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 1..1278
   (xi) SEQUENCE DESCRIPTION: SEQ ID NO:14:
(2) INFORMATION FOR SEQ ID NO:15:
   (i) SEQUENCE CHARACTERISTICS:
      (A) LENGTH: 425 amino acids
      (B) TYPE: amino acid
      (D) TOPOLOGY: linear
   (ii) MOLECULE TYPE: protein
   (xi) SEQUENCE DESCRIPTION: SEQ ID NO:15:
   Met Pro Ala Gly Arg Pro Gly Pro Val Ala Gln Ser Ala Arg Arg Pro
    1               5                  10                  15
   Pro Arg Pro Leu Ser Ser Leu Trp Ser Pro Leu Leu Leu Cys Val Leu
               20                  25                  30
   Gly Val Pro Arg Gly Gly Ser Gly Ala His Thr Ala Val Ile Ser Pro
   35 40 45
   Gln Asp Pro Thr Leu Leu Ile Gly Ser Ser Leu Gln Ala Thr Cys Ser
   50 55 60
   Ile His Gly Asp Thr Pro Gly Ala Thr Ala Glu Gly Leu Tyr Trp Thr
   65 70 75 80
   Leu Asn Gly Arg Arg Leu Pro Ser Glu Leu Ser Arg Leu Leu Asn Thr
   85 90 95
   Ser Thr Leu Ala Leu Ala Leu Ala Asn Leu Asn Gly Ser Arg Gln Gln
   100 105 110
   Ser Gly Asp Asn Leu Val Cys His Ala Arg Asp Gly Ser Ile Leu Ala
   115 120 125
   Gly Ser Cys Leu Tyr Val Gly Leu Pro Pro Glu Lys Pro Phe Asn Ile
   130 135 140
   Ser Cys Trp Ser Arg Asn Met Lys Asp Leu Thr Cys Arg Trp Thr Pro
   145 150 155 160
   Gly Ala His Gly Glu Thr Phe Leu His Thr Asn Tyr Ser Leu Lys Tyr
   165 170 175
   Lys Leu Arg Trp Tyr Gly Gln Asp Asn Thr Cys Glu Glu Tyr His Thr
   180 185 190
   Val Gly Pro His Ser Cys His Ile Pro Lys Asp Leu Ala Leu Phe Thr
   195 200 205
   Pro Tyr Glu Ile Trp Val Glu Ala Thr Asn Arg Leu Gly Ser Ala Arg
   210 215 220
   Ser Asp Val Leu Thr Leu Asp Val Leu Asp Val Val Thr Thr Asp Pro
   225 230 235 240
   Pro Pro Asp Val His Val Ser Arg Val Gly Gly Leu Glu Asp Gln Leu
   245 250 255
   Ser Val Arg Trp Val Ser Pro Pro Ala Leu Lys Asp Phe Leu Phe Gln
   260 265 270
   Ala Lys Tyr Gln Ile Arg Tyr Arg Val Glu Asp Ser Val Asp Trp Lys
   275 280 285
   Val Val Asp Asp Val Ser Asn Gln Thr Ser Cys Arg Leu Ala Gly Leu
   290 295 300
   Lys Pro Gly Thr Val Tyr Phe Val Gln Val Arg Cys Asn Pro Phe Gly
   305 310 315 320
   Ile Tyr Gly Ser Lys Lys Ala Gly Ile Trp Ser Glu Trp Ser His Pro
   325 330 335
   Thr Ala Ala Ser Thr Pro Arg Ser Glu Arg Pro Gly Pro Gly Gly Gly
   340 345 350
   Val Cys Glu Pro Arg Gly Gly Glu Pro Ser Ser Gly Pro Val Arg Arg
   355 360 365
   Glu Leu Lys Gln Phe Leu Gly Trp Leu Lys Lys His Ala Tyr Cys Ser
   370 375 380
   Asn Leu Ser Phe Arg Leu Tyr Asp Gln Trp Arg Ala Trp Met Gln Lys
   385 390 395 400
   Ser His Lys Thr Arg Asn Gln Asp Glu Gly Ile Leu Pro Ser Gly Arg
   405 410 415
   Arg Gly Ala Ala Arg Gly Pro Ala Gly
   420 425
(2) INFORMATION FOR SEQ ID NO:16:
   (i) SEQUENCE CHARACTERISTICS:
      (A) LENGTH: 938 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 1..468
   (xi) SEQUENCE DESCRIPTION: SEQ ID NO:16:
   GGC ACC GTT TAC TTC GTC CAA GTG CGT TGT AAC CCA TTC GGG ATC TAT 48
   Gly Thr Val Tyr Phe Val Gln Val Arg Cys Asn Pro Phe Gly Ile Tyr
   1 5 10 15
   GGG TCG AAA AAG GCG GGA ATC TGG AGC GAG TGG AGC CAC CCC ACC GCT 96
   Gly Ser Lys Lys Ala Gly Ile Trp Ser Glu Trp Ser His Pro Thr Ala
   20 25 30
   GCC TCC ACC CCT CGA AGT GAG CGC CCG GGC CCG GGC GGC GGG GTG TGC 144
   Ala Ser Thr Pro Arg Ser Glu Arg Pro Gly Pro Gly Gly Gly Val Cys
   35 40 45
   GAG CCG CGG GGC GGC GAG CCC AGC TCG GGC CCG GTG CGG CGC GAG CTC      192
   Glu Pro Arg Gly Gly Glu Pro Ser Ser Gly Pro Val Arg Arg Glu Leu
        50                  55                  60
   AAG CAG TTC CTC GGC TGG CTC AAG AAG CAC GCA TAC TGC TCG AAC CTT      240
   Lys Gln Phe Leu Gly Trp Leu Lys Lys His Ala Tyr Cys Ser Asn Leu
    65                  70                  75                  80
   AGT TTC CGC CTG TAC GAC CAG TGG CGT GCT TGG ATG CAG AAG TCA CAC      288
   Ser Phe Arg Leu Tyr Asp Gln Trp Arg Ala Trp Met Gln Lys Ser His
                    85                  90                  95
   AAG ACC CGA AAC CAG GTA GGA AAG TTG GGG GAG GCT TGC GTG GGG GGT      336
   Lys Thr Arg Asn Gln Val Gly Lys Leu Gly Glu Ala Cys Val Gly Gly
               100                 105                 110
   AAA GGA GCA GAG GAA GAG AGA GAC CCG GGT GAG CAG CCT CCA CAA CAC      384
   Lys Gly Ala Glu Glu Glu Arg Asp Pro Gly Glu Gln Pro Pro Gln His
           115                 120                 125
   CGC ACT CTT CTT TCC AAG CAC AGG ACG AGG GGA TCC TGC CCT CGG GCA      432
   Arg Thr Leu Leu Ser Lys His Arg Thr Arg Gly Ser Cys Pro Arg Ala
       130                 135                 140
   GAC GGG GTG CGG CGA GAG GTA AGG GGG TCT GGG TGAGTGGGGC CTACAGCAGT    485
   Asp Gly Val Arg Arg Glu Val Arg Gly Ser Gly
   145                 150                 155
   CTAGATGAGG CCCTTTCCCC TCCTTCGGTG TTGCTCAAAG GGATCTCTTA GTGCTCATTT    545
   CACCCACTGC AAAGAGCCCC AGGTTTTACT GCATCATCAA GTTGCTGAAG GGTCCAGGCT    605
   TAATGTGGCC TCTTTTCTGC CCTCAGGTCC TGCCGGCTAA ACTCTAAGGA TAGGCCATCC    665
   TCCTGCTGGG TCAGACCTGG AGGCTCACCT GAATTGGAGC CCCTCTGTAC CTATCTGGGC    725
   AACAAAGAAA CCTACCATGA GGCTGGGGCA CAATGAGCTC CCACAACCAC AGCTTTGGTC    785
   CACATGATGG TCACACTTGG ATATACCCCA GTGTGGGTAA GGTTGGGGTA TTGCAGGGCC 845
   TCCCAACAAT CTCTTTAAAT AAATAAAGGA GTTGTTCAGG TAAAAAAAAA AAAAAAAAAA 905
   AAAAAAAAAA AAAAAAAAAA AAAAAAAAAA AAA 938
(2) INFORMATION FOR SEQ ID NO:17:
   (i) SEQUENCE CHARACTERISTICS:
      (A) LENGTH: 155 amino acids
      (B) TYPE: amino acid
      (D) TOPOLOGY: linear
   (ii) MOLECULE TYPE: protein
   (xi) SEQUENCE DESCRIPTION: SEQ ID NO:17:
   Gly Thr Val Tyr Phe Val Gln Val Arg Cys Asn Pro Phe Gly Ile Tyr
   1 5 10 15
   Gly Ser Lys Lys Ala Gly Ile Trp Ser Glu Trp Ser His Pro Thr Ala
   20 25 30
   Ala Ser Thr Pro Arg Ser Glu Arg Pro Gly Pro Gly Gly Gly Val Cys
   35 40 45
   Glu Pro Arg Gly Gly Glu Pro Ser Ser Gly Pro Val Arg Arg Glu Leu
   50 55 60
   Lys Gln Phe Leu Gly Trp Leu Lys Lys His Ala Tyr Cys Ser Asn Leu
   65 70 75 80
   Ser Phe Arg Leu Tyr Asp Gln Trp Arg Ala Trp Met Gln Lys Ser His
   85 90 95
   Lys Thr Arg Asn Gln Val Gly Lys Leu Gly Glu Ala Cys Val Gly Gly
   100 105 110
   Lys Gly Ala Glu Glu Glu Arg Asp Pro Gly Glu Gln Pro Pro Gln His
   115 120 125
   Arg Thr Leu Leu Ser Lys His Arg Thr Arg Gly Ser Cys Pro Arg Ala
   130 135 140
   Asp Gly Val Arg Arg Glu Val Arg Gly Ser Gly
   145 150 155
(2) INFORMATION FOR SEQ ID NO:18:
   (i) SEQUENCE CHARACTERISTICS:
      (A) LENGTH: 834 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 1..834
   (xi) SEQUENCE DESCRIPTION: SEQ ID NO:18:
(2) INFORMATION FOR SEQ ID NO:19:
   (i) SEQUENCE CHARACTERISTICS:
      (A) LENGTH: 278 amino acids
      (B) TYPE: amino acid
      (D) TOPOLOGY: linear
   (ii) MOLECULE TYPE: protein
   (xi) SEQUENCE DESCRIPTION: SEQ ID NO:19:
(2) INFORMATION FOR SEQ ID NO:20:
   (i) SEQUENCE CHARACTERISTICS:
      (A) LENGTH: 143 base pairs
      (B) TYPE: nucleic acids
      (D) TOPOLOGY: linear
   (ii) MOLECULE TYPE: protein
   (xi) SEQUENCE DESCRIPTION: SEQ ID NO:20:
   GGCATGAAGG CTTAGGGTGG GGATCGGTAG GACCCATGCA CCCAGAGAAA GGGACTGGTG 60
   GCAACTTTCA AACTCTCTGG GGAAGGAAGA AGGGCTGAAA GAGG 104
   ATG AAC GGG CTC AGA CAC AGC TGT AAT CAG CCC CCA GGA 143
   Met Asn Gly Leu Arg His Ser Cys Asn Gln Pro Pro Gly
   5 10
(2) INFORMATION FOR SEQ ID NO:21:
   (i) SEQUENCE CHARACTERISTICS:
      (A) LENGTH: 13 amino acids
      (B) TYPE: amino acids
      (D) TOPOLOGY: linear
   (ii) MOLECULE TYPE: protein
   (xi) SEQUENCE DESCRIPTION: SEQ ID NO:21:
   Met Asn Gly Leu Arg His Ser Cys Asn Gln Pro Pro Gly
   5 10
(2) INFORMATION FOR SEQ ID NO:22:
   (i) SEQUENCE CHARACTERISTICS:
      (A) LENGTH: 1930 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:22:
   GGCACGAGCT TCGCTGTCCG CGCCCAGTGA CGCGCGTGCG GACCCGAGCC CCAATCTGCA 60
   CCCCGCAGAC TCGCCCCCGC CCCATACCGG CGTTGCAGTC ACCGCCCGTT GCGCGCCACC 120
   CCCAATGCCC GCGGGTCGCC CGGGCCCCGT CGCCCAATCC GCGCGGCGGC CGCCGCGGCC 180
   GCTGTCCTCG CTGTGGTCGC CTCTGTTGCT CTGTGTCCTC GGGGTGCCTC GGGGCGGATC 240
   GGGAGCCCAC ACAGCTGTAA TCAGCCCCCA GGACCCCACC CTTCTCATCG GCTCCTCCCT 300
   GCAAGCTACC TGCTCTATAC ATGGAGACAC ACCTGGGGCC ACCGCTGAGG GGCTCTACTG 360
   GACCCTCAAT GGTCGCCGCC TGCCCTCTGA GCTGTCCCGC CTCCTTAACA CCTCCACCCT 420
   GGCCCTGGCC CTGGCTAACC TTAATGGGTC CAGGCAGCAG TCAGGAGACA ATCTGGTGTG 480
   TCACGCCCGA GACGGCAGCA TTCTGGCTGG CTCCTGCCTC TATGTTGGCT TGCCCCCTGA 540
   GAAGCCCTTT AACATCAGCT GCTGGTCCCG GAACATGAAG GATCTCACGT GCCGCTGGAC 600
   ACCGGGTGCA CACGGGGAGA CATTCTTACA TACCAACTAC TCCCTCAAGT ACAAGCTGAG 660
   GTGGTACGGT CAGGATAACA CATGTGAGGA GTACCACACT GTGGGCCCTC ACTCATGCCA 720
   TATCCCCAAG GACCTGGCCC TCTTCACTCC CTATGAGATC TGGGTGGAAG CCACCAATCG 780
   CCTAGGCTCA GCAAGATCTG ATGTCCTCAC ACTGGATGTC CTGGACGTGG TGACCACGGA 840
   CCCCCCACCC GACGTGCACG TGAGCCGCGT TGGGGGCCTG GAGGACCAGC TGAGTGTGCG 900
   CTGGGTCTCA CCACCAGCTC TCAAGGATTT CCTCTTCCAA GCCAAGTACC AGATCCGCTA 960
   CCGCGTGGAG GACAGCGTGG ACTGGAAGGT GGTGGATGAC GTCAGCAACC AGACCTCCTG 1020
   CCGTCTCGCG GGCCTGAAGC CCGGCACCGT TTACTTCGTC CAAGTGCGTT GTAACCCATT 1080
   CGGGATCTAT GGGTCGAAAA AGGCGGGAAT CTGGAGCGAG TGGAGCCACC CCACCGCTGC 1140
   CTCCACCCCT CGAAGTGAGC GCCCGGGCCC GGGCGGCGGG GTGTGCGAGC CGCGGGGCGG 1200
   CGAGCCCAGC TCGGGCCCGG TGCGGCGCGA GCTCAAGCAG TTCCTCGGCT GGCTCAAGAA 1260
   GCACGCATAC TGCTCGAACC TTAGTTTCCG CCTGTACGAC CAGTGGCGTG CTTGGATGCA 1320
   GAAGTCACAC AAGACCCGAA ACCAGGTAGG AAAGTTGGGG GAGGCTTGCG TGGGGGGTAA 1380
   AGGAGCAGAG GAAGAGAGAG ACCCGGGTGA GCAGCCTCCA CAACACCGCA CTCTTCTTTC 1440
   CAAGCACAGG ACGAGGGGAT CCTGCCCTCG GGCAGACGGG GTGCGGCGAG AGGTAAGGGG 1500
   GTCTGGGTGA GTGGGGCCTA CAGCAGTCTA GATGAGGCCC TTTCCCCTCC TTCGGTGTTG 1560
   CTCAAAGGGA TCTCTTAGTG CTCATTTCAC CCACTGCAAA GAGCCCCAGG TTTTACTGCA 1620
   TCATCAAGTT GCTGAAGGGT CCAGGCTTAA TGTGGCCTCT TTTCTGCCCT CAGGTCCTGC 1680
   CGGCTAAACT CTAAGGATAG GCCATCCTCC TGCTGGGTCA GACCTGGAGG CTCACCTGAA 1740
   TTGGAGCCCC TCTGTACCTA TCTGGGCAAC AAAGAAACCT ACCATGAGGC TGGGGCACAA 1800
   TGAGCTCCCA CAACCACAGC TTTGGTCCAC ATGATGGTCA CACTTGGATA TACCCCAGTG 1860
   TGGGTAAGGT TGGGGTATTG CAGGGCCTCC CAACAATCTC TTTAAATAAA TAAAGGAGTT 1920
   GTTCAGGTAA 1930
(2) INFORMATION FOR SEQ ID NO : 23:
   (i) SEQUENCE CHARACTERISTICS:
      (A) LENGTH: 560 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:23:
   TCCAGGCAGC GGTCGGGGGA CAACCTCGTG TGCCACGCCC GTGACGGCAG CATCCTGGCT 60
   GGCTCCTGCC TCTATGTTGG CCTGCCCCCA GAGAAACCCG TCAACATCAG CTGCTGGTCC 120
   AAGAACATGA AGGACTTGAC CTGCCGCTGG ACGCCAGGGG CCCACGGGGA GACCTTCCTC 180
   CACACCAACT ACTCCCTCAA GTACAAGCTT AGGTGGTATG GCCAGGACAA CACATGTGAG 240
   GAGTACCACA CAGTGGGGCC CCACTCCTGC CACATCCCCA AGGACCTGGC TCTCTTTACG 300
   CCCTATGAGA TCTGGGTGGA GGCCACCAAC CGCCTGGGCT CTGCCCGCTC CGATGTACTC 360
   ACGCTGGATA TCCTGGATGT GGTGACCACG GACCCCCCGC CCGACGTGCA CGTGAGCCGC 420
   GTCGGGGGCC TGGAGGACCA GCTGAGCGTG CGCTGGGTGT CGCCACCCGC CCTCAAGGAT 480
   TTCCTTTTTC AAGCCAAATA CCAGATCCGC TACCGAGTGG AGGACAGTGT GGAATGGAAG 540
   GTGGTGGACG ATGTGAGCAA 560
(2) INFORMATION FOR SEQ ID NO:24:
   (i) SEQUENCE CHARACTERISTICS:
      (A) LENGTH: 1391 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: CDS
      (B) LOCATION: 1..1053
   (xi) SEQUENCE DESCRIPTION: SEQ ID NO:24:
   ACC CTC AAC GGG CGC CGC CTG CCC CCT GAG CTC TCC CGT GTA CTC AAC      48
   Thr Leu Asn Gly Arg Arg Leu Pro Pro Glu Leu Ser Arg Val Leu Asn
    1               5                  10                  15
   GCC TCC ACC TTG GCT CTG GCC CTG GCC AAC CTC AAT GGG TCC AGG CAG      96
   Ala Ser Thr Leu Ala Leu Ala Leu Ala Asn Leu Asn Gly Ser Arg Gln
               20                  25                  30
   CGG TCG GGG GAC AAC CTC GTG TGC CAC GCC CGT GAC GGC AGC ATC CTG     144
   Arg Ser Gly Asp Asn Leu Val Cys His Ala Arg Asp Gly Ser Ile Leu
           35                  40                  45
   GCT GGC TCC TGC CTC TAT GTT GGC CTG CCC CCA GAG AAA CCC GTC AAC     192
   Ala Gly Ser Cys Leu Tyr Val Gly Leu Pro Pro Glu Lys Pro Val Asn
       50                  55                  60
   ATC AGC TGC TGG TCC AAG AAC ATG AAG GAC TTG ACC TGC CGC TGG ACG     240
   Ile Ser Cys Trp Ser Lys Asn Met Lys Asp Leu Thr Cys Arg Trp Thr
   65                  70                  75                  80
   CCA GGG GCC CAC GGG GAG ACC TTC CTC CAC ACC AAC TAC TCC CTC AAG     288
   Pro Gly Ala His Gly Glu Thr Phe Leu His Thr Asn Tyr Ser Leu Lys
                   85                  90                  95
   TAC AAG CTT AGG TGG TAT GGC CAG GAC AAC ACA TGT GAG GAG TAC CAC     336
   Tyr Lys Leu Arg Trp Tyr Gly Gln Asp Asn Thr Cys Glu Glu Tyr His
               100                 105                 110
   ACA GTG GGG CCC CAC TCC TGC CAC ATC CCC AAG GAC CTG GCT CTC TTT 384
   Thr Val Gly Pro His Ser Cys His Ile Pro Lys Asp Leu Ala Leu Phe
   115 120 125
   ACG CCC TAT GAG ATC TGG GTG GAG GCC ACC AAC CGC CTG GGC TCT GCC 432
   Thr Pro Tyr Glu Ile Trp Val Glu Ala Thr Asn Arg Leu Gly Ser Ala
   130 135 140
   CGC TCC GAT GTA CTC ACG CTG GAT ATC CTG GAT GTG GTG ACC ACG GAC 480
   Arg Ser Asp Val Leu Thr Leu Asp Ile Leu Asp Val Val Thr Thr Asp
   145 150 155 160
   CCC CCG CCC GAC GTG CAC GTG AGC CGC GTC GGG GGC CTG GAG GAC CAG 528
   Pro Pro Pro Asp Val His Val Ser Arg Val Gly Gly Leu Glu Asp Gln
   165 170 175
   CTG AGC GTG CGC TGG GTG TCG CCA CCC GCC CTC AAG GAT TTC CTC TTT 576
   Leu Ser Val Arg Trp Val Ser Pro Pro Ala Leu Lys Asp Phe Leu Phe
   180 185 190
   CAA GCC AAA TAC CAG ATC CGC TAC CGA GTG GAG GAC AGT GTG GAC TGG 624
   Gln Ala Lys Tyr Gln Ile Arg Tyr Arg Val Glu Asp Ser Val Asp Trp
   195 200 205
   AAG GTG GTG GAC GAT GTG AGC AAC CAG ACC TCC TGC CGC CTG GCC GGC 672
   Lys Val Val Asp Asp Val Ser Asn Gln Thr Ser Cys Arg Leu Ala Gly
   210 215 220
   CTG AAA CCC GGC ACC GTG TAC TTC GTG CAA GTG CGC TGC AAC CCC TTT 720
   Leu Lys Pro Gly Thr Val Tyr Phe Val Gln Val Arg Cys Asn Pro Phe
   225 230 235 240
   GGC ATC TAT GGC TCC AAG AAA GCC GGG ATC TGG AGT GAG TGG AGC CAC 768
   Gly Ile Tyr Gly Ser Lys Lys Ala Gly Ile Trp Ser Glu Trp Ser His
   245 250 255
   CCC ACA GCC GCC TCC ACT CCC CGC AGT GAG CGC CCG GGC CCG GGC GGC      816
   Pro Thr Ala Ala Ser Thr Pro Arg Ser Glu Arg Pro Gly Pro Gly Gly
               260                 265                 270
   GGG GCG TGC GAA CCG CGG GGC GGA GAG CCG AGC TCG GGG CCG GTG CGG      864
   Gly Ala Cys Glu Pro Arg Gly Gly Glu Pro Ser Ser Gly Pro Val Arg
           275                 280                 285
   CGC GAG CTC AAG CAG TTC CTG GGC TGG CTC AAG AAG CAC GCG TAC TGC      912
   Arg Glu Leu Lys Gln Phe Leu Gly Trp Leu Lys Lys His Ala Tyr Cys
       290                 295                 300
   TCC AAC CTC AGC TTC CGC CTC TAC GAC CAG TGG CGA GCC TGG ATG CAG      960
   Ser Asn Leu Ser Phe Arg Leu Tyr Asp Gln Trp Arg Ala Trp Met Gln
   305                 310                 315                 320
   AAG TCG CAC AAG ACC CGC AAC CAG CAC AGG ACG AGG GGA TCC TGC CCT     1008
   Lys Ser His Lys Thr Arg Asn Gln His Arg Thr Arg Gly Ser Cys Pro
                   325                 330                 335
   CGG GCA GAC GGG GCA CGG CGA GAG GTC CTG CCA GAT AAG CTG TAGGGGCTCA  1060
   Arg Ala Asp Gly Ala Arg Arg Glu Val Leu Pro Asp Lys Leu
               340                 345                 350
   GGCCACCCTC CCTGCCACGT GGAGACGCAG AGGCCGAACC CAAACTGGGG CCACCTCTGT    1120
   ACCCTCACTT CAGGGCACCT GAGCCCCTCA GCAGGAGCTG GGGTGGCCCC TGAGCTCCAA    1180
   CGGCCATAAC AGCTCTGACT CCCACGTGAG GCCACCTTTG GGTGCACCCC AGTGGGTGTG    1240
   TGTGTGTGTG TGAGGGTTGG TTGAGTTGCC TAGAACCCCT GCCAGGGCTG GGGGTGAGAA    1300
   GGGGAGTCAT TACTCCCCAT TACCTAGGGC CCCTCCAAAA GAGTCCTTTT AAATAAATGA    1360
   GCTATTTAGG TGCAAAAAAA AAAAAAAAAA A                                   1391
(2) INFORMATION FOR SEQ ID NO:25:
   (i) SEQUENCE CHARACTERISTICS:
      (A) LENGTH: 350 amino acids
      (B) TYPE: amino acid
      (D) TOPOLOGY: linear
   (ii) MOLECULE TYPE: protein
   (xi) SEQUENCE DESCRIPTION: SEQ ID NO:25:
   Thr Leu Asn Gly Arg Arg Leu Pro Pro Glu Leu Ser Arg Val Leu Asn
    1               5                  10                  15
   Ala Ser Thr Leu Ala Leu Ala Leu Ala Asn Leu Asn Gly Ser Arg Gln
               20                  25                  30
   Arg Ser Gly Asp Asn Leu Val Cys His Ala Arg Asp Gly Ser Ile Leu
           35                  40                  45
   Ala Gly Ser Cys Leu Tyr Val Gly Leu Pro Pro Glu Lys Pro Val Asn
       50                  55                  60
   Ile Ser Cys Trp Ser Lys Asn Met Lys Asp Leu Thr Cys Arg Trp Thr
   65                  70                  75                  80
   Pro Gly Ala His Gly Glu Thr Phe Leu His Thr Asn Tyr Ser Leu Lys
                   85                  90                  95
   Tyr Lys Leu Arg Trp Tyr Gly Gln Asp Asn Thr Cys Glu Glu Tyr His
               100                 105                 110
   Thr Val Gly Pro His Ser Cys His Ile Pro Lys Asp Leu Ala Leu Phe
           115                 120                 125
   Thr Pro Tyr Glu Ile Trp Val Glu Ala Thr Asn Arg Leu Gly Ser Ala
       130                 135                 140
   Arg Ser Asp Val Leu Thr Leu Asp Ile Leu Asp Val Val Thr Thr Asp
   145 150 155 160
   Pro Pro Pro Asp Val His Val Ser Arg Val Gly Gly Leu Glu Asp Gln
   165 170 175
   Leu Ser Val Arg Trp Val Ser Pro Pro Ala Leu Lys Asp Phe Leu Phe
   180 185 190
   Gln Ala Lys Tyr Gln Ile Arg Tyr Arg Val Glu Asp Ser Val Asp Trp
   195 200 205
   Lys Val Val Asp Asp Val Ser Asn Gln Thr Ser Cys Arg Leu Ala Gly
   210 215 220
   Leu Lys Pro Gly Thr Val Tyr Phe Val Gln Val Arg Cys Asn Pro Phe
   225 230 235 240
   Gly Ile Tyr Gly Ser Lys Lys Ala Gly Ile Trp Ser Glu Trp Ser His
   245 250 255
   Pro Thr Ala Ala Ser Thr Pro Arg Ser Glu Arg Pro Gly Pro Gly Gly
   260 265 270
   Gly Ala Cys Glu Pro Arg Gly Gly Glu Pro Ser Ser Gly Pro Val Arg
   275 280 285
   Arg Glu Leu Lys Gln Phe Leu Gly Trp Leu Lys Lys His Ala Tyr Cys
   290 295 300
   Ser Asn Leu Ser Phe Arg Leu Tyr Asp Gln Trp Arg Ala Trp Met Gln
   305 310 315 320
   Lys Ser His Lys Thr Arg Asn Gln His Arg Thr Arg Gly Ser Cys Pro
   325 330 335
   Arg Ala Asp Gly Ala Arg Arg Glu Val Leu Pro Asp Lys Leu
   340 345 350
(2) INFORMATION FOR SEQ ID NO:26:
   (i) SEQUENCE CHARACTERISTICS:
      (A) LENGTH: 24 base pairs
      (B) TYPE: nucleic acid
      (C) STRANDEDNESS: single
      (D) TOPOLOGY : linear
   (ii) MOLECULE TYPE: DNA
   (xi) SEQUENCE DESCRIPTION: SEQ ID NO:26:
      TCCAGGCAGC GGTCGGGGGA CAAC 24
(2) INFORMATION FOR SEQ ID NO:27:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:27:
      TTGCTCACAT CGTCCACCAC CTTC 24
(2) INFORMATION FOR SEQ ID NO:28:
   (i) SEQUENCE CHARACTERISTICS:
      (A) LENGTH: 6663 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:28:
   CCCAGAACTC TTGGACGCTG AGGCAGGAGG ATTCCCAAGT TTCAAGACAG TGTGTTTCTA 60
   GGTAATGAGA CCCTGTCAAG AAAAGAAAAG AAATAAAGAG ACAAGAAAAT GTTTATAGGC 120
   TGTGAGACAG CTTGGTGGGT AAGGGGCACT TGCCTCCAAT CAAGATGACC TCAGCCCCAT 180
   CCCTAGGAAT CCATGGTAGA AGGAGAAAGC AAACTCGCAG CTGCTGACCT CCATACATGT 240
   GCTCCAATGT GCACACACAC AGGGAGACAT AATCAATTAA TAGGATGTAT TTGCTTAGAT 300
   TTGAGTAGGC ATTTATGACT GATGTTTTAA AATTTTTATT TGATTTTATG AAAATATACC 360
   TGTTTGTATT TGGTTTGGTT TGGTTTGAGT TTTGTTTATT TGAGACAGGG CTTCTCTGTG 420
   TAGTCCTGGC TGTCCTTGGA ACTCACTCTG TAGACCAGGC TGGCCTTGAA CTCAGAAATC 480
   CGCCTGCTTG TGCTTCCCAA GTGCTTAGAT TAAAGGTGTG CACTGCCATT CAGCAAAATT 540
   GCATACTTTA ACCCCAGTAT TTGGGAGGCA GAGGCAGACT AATGTGTGAA TTCCAGGCTA 600
   GCCAAGGATA CAGAGTGAGA CCCTATTCTT ACCCTCCCCC CCCAAAACCC CAAAATGTAT 660
   TTTGTGCTTG TGTATGTACA TGTGTGTTGC AGCACGTAAA TGTCCAAGGA CAACTTGTAG 720
   AAGTTCTCTC CGTTCACAGT CTAAGTCCTG AATTCAAACT AAGGTCCTCA GGCTTAGCCA 780
   CAGTCTTCTT TATGTACTGA GCCATTTCAC TGGCCCTGGA TTGACTGATG AATTAATTTT 840
   TGAGATAAGG TCTCTTGTAG CTCTAGCTAG GCTCAAACTA TGAACTCCCA AGGTCATCTT 900
   GAGCTGCTGG TACTCTTGCT TCCACCCCAA GTGGTGGAAT GATACTCAGG CAGCACTTCT 960
   CTGGGGAAGG GGCTGGCCTT GGCCTTGATT TTGTTGCCTC AGCTTCAATG AGTGCTTGGG 1020
   TCTCGTTGTT TCTTTTCTTT ATCTGTGAAA TGGGTGAACA CCTGTTCAAG ACTTCCTGAC 1080
   TCTTGAAACA TCCAGGCAGG GTGAGGGACT TGAAGTGGGC TCATCCCATG CCTAACAAAG 1140
   TGTCGTCTTT GACCCCAGAC ACAGCTGTAA TCAGCCCCCA GGACCCCACC CTTCTCATCG 1200
   GCTCCTCCCT GCAAGCTACC TGCTCTATAC ATGGAGACAC ACCTGGGGCC ACCGCTGAGG 1260
   GGCTCTACTG GACCTTCAAT GGTCGCCGCC TGCCCTCTGA GCTGTCCCGC CTCCTTAACA 1320
   CCTCCACCCT GGCCCTGGCC CTGGCTAACC TTAATGGGTC CAGGCAGCAG TCAGGAGACA 1380
   ATCTGGTGTG TCACGCCCGA GACGGCAGCA TTCTGGCTGG CTCCTGCCTC TATGTTGGCT 1440
   GTAAGTGGGG CCCCAGACAC TCAGAGATAG ATGGGGGTTG GCAATGACAG ATTTAGAGCC 1500
   TGGGTCTTCT GTCCTGGGGC AGAGCCATGG GCTCTCACTT GCATGCAGGC ATGGTCATAC 1560
   CCAGCACAGG CATTGCAACT CTAGGGACAG CTGTGGCTGC ACTGTCCCCT GTGTACCCCA 1620
   CAGCTTTAGA AAAGCTGTCA TGTTTTCCTT GTAGTGCCCC CTGAGAAGCC CTTTAACATC 1680
   AGCTGCTGGT CCCGGAACAT GAAGGATCTC ACGTGCCGCT GGACACCGGG TGCACACGGG 1740
   GAGACATTCT TACATACCAA CTACTCCCTC AAGTACAAGC TGAGGTTGGT ACCCAGCCAA 1800
   GCCTTGCTGT GTGACTTCTG GCAATACTTA CCTTCTCTGA TCAAATATGT TCCTGTTTAT 1860
   GAACTCAAAA GGGACTCTCG CACCTCCACA GGTGGTACGG TCAGGATAAC ACATGTGAGG 1920
   AGTACCACAC TGTGGGCCCT CACTCATGCC ATATCCCCAA GGACCTGGCC CTCTTCACTC 1980
   CCTATGAGAT CTGGGTGGAA GCCACCAATC GCCTAGGCTC AGCAAGATCT GATGTCCTCA 2040
   CACTGGATGT CCTGGACGTG GGTGAGCCCC CAGTGTCCAC CTGTGTTCTG CCCTAGACCT 2100
   TATAGGGCGC CTCCCCCCCA TCCCCCCAGA CTTTTTGGTT CTTCTAGAGG TCTTAGCCAC 2160
   AGCCACGGTG GTTGCAGGAC AGTGGTTGTT CATAACTTAA TGCAAAGACT TTCCCCCAAG 2220
   ACAGTCAAGA TTTTTCCCCT CCCCACCCCC AACACACACA TACACACACA CTCTGCAGAG 2280
   AACACCTGGC CTGACCACCC TCCCTCTCTA CAGCCCAGGT GTTCAGAAGG GAGTCCTAGG 2340
   GGACTGAGAG GAGGCGCCCA GGTCTGAAGG CGCCCCAGGA AGCCGAGGCC TTGAGCTGGG 2400
   GGGGGGGGCG AGGGTTGGAG GCACGAACTG GATGATCCCT GAGCACAACT GGGCCTAATC 2460
   TAATTAGGGT GTTCCCAGCC CAAAGCAGCC TGGGCCATTT AACCCTTCAA GTGCCTCACT 2520
   GAAGACTCAG GGGAGAGATC AGCTTGTACT CTCTCCATGG TCCCCCAGGA GGGTTCCTGG 2580
   GTGCCCCTGG CTCATTCCCA CATCCAGAGG TTTTGTGTCT TCCTGGCATC TAACCCTCAG 2640
   TTGTGCTCTG TGGCTGGCAC AGCTGCCCCG TGGAGGCTCT TGGTAATGTA CAAGGCATCA 2700
   GAGGTGGACA TGGGATGGGG ATACATAGGG ATGGAGCCAA ATAGCACCTC AAGGTGGGGT 2760
   GATATACAAT AAAGCTTGTC ACCCTGACGC TCAGAAAGCC TACTCATGAT GATCACAATT 2820
   GTTGACATCA CTCTGGGACA TGTAGTGAGA CCCTAGCTCA AAACACAGAC AGTAGCTTTA 2880
   AGAGTCAGCT TGTGACTTAA TACTGGAACT CAGGGCCTAA TAGGTGCTGG GTGATGCTCG 2940
   CCTCACTCCC TGTTTAGTGA GATCTCTGCG CTAATCTCCA CCCCAGCTGG GTGGGCTGCT 3000
   CTGTCCCCTT GAGGGCAGGA ATGTGTGTCT TCCATCAGAG ATAGGACCCG TGGTAGCAGC 3060
   AACTGCTGCT GGCTGTTTCT GGAATATTAA ATGACAGTAA TCTATCAGGC CTGGGTGAGT 3120
   AGCTAACAGG GGTGGGGGCG TGGTCTGGAA AACGCAGATA GGGTCATAGG AGCCACTGCA 3180
   GCCTAGATTA CACCACTGGG TGTTCTGTCA CTAGGCCATT CTCACCAAGC AGTCCTCAGA 3240
   ACTGGGAGCA CTGTTGCCAG CATTTAATGC CAGCATTTAA TGCCAGCATT AGGGGAGGCA 3300
   GAGGCAGAAG GATCTCTCTG AGTTCAAGGC CATCCTGAAT TTACATAAAG AGCTCCAGGC 3360
   CAGCCAGGGT GCGCAGTAAA ACCTTGTCTC AAAAAACAAA GCATCTTTAG TGACCAGGCT 3420
   TGCTCCACCC CCAGTGACCA CGGACCCCCC ACCCGACGTG CACGTGAGCC GCGTTGGGGG 3480
   CCTGGAGGAC CAGCTGAGTG TGCGCTGGGT CTCACCACCA GCTCTCAAGG ATTTCCTCTT 3540
   CCAAGCCAAG TACCAGATCC GCTACCGCGT GGAGGACAGC GTGGACTGGA AGGTGCCCGT 3600
   CCCGCCCCGG ACCCGCCCCT GACCCCGCCC CCCGCATCTG ACTCCTCCCT CACCGTGCAG 3660
   GTGGTGGATG ACGTCAGCAA CCAGACCTCC TGCCGTCTCG CGGGCCTGAA GCCCGGCACC 3720
   GTTTACTTCG TCCAAGTGCG TTGTAACCCA TTCGGGATCT ATGGGTCGAA AAAGGCGGGA 3780
   ATCTGGAGCG AGTGGAGCCA CCCCACCGCT GCCTCCACCC CTCGAAGTGG TGAGCACCTC 3840
   TCCAGGGCTG GCTGGCCCAT GGAATCCCCA ATCCATCCTG TTCCTTCCCC CCCACCCTTT 3900
   TTTTGAGACA GCGTCTTCAG GTAGCGCATG CTGGCCTTAA ATTCAGTATG TAGTCAAGGA 3960
   TGACCTCGAG CTCCTGGTCT TTTTGTCTCC ACTTAGAGAC AATGGCCAGT GGCCATCACC 4020
   ACCTTTGGGA GACTAGCCAT GGAGTCTATT TAGCCTGTCA TTTGGTGACA GATGGAGTAC 4080
   AACAGTGTGA CCTCTTGTAA GAGAACTGAA GACAGGCTGT TTTTAACCCC AATATCCTAG 4140
   GCTCTCTAGA GGTTAACTTT ATATAAAATA GAGACTATTA CAGCCAGTTA TCACATGGTC 4200
   CCACAGAACC TTTTGTCACA CAACCTATAG ACCACAGTGC CTGTGCCTAC CACATAAGGG 4260
   TCTCTACTGC TGGCCCACCC CTCCAACCCT TAAAAGGTAA CCTAGGCAGC CTTAATATTT 4320
   GCAATCCTCC TACCTCAGCC TCTTGAATGC TCAGAAACCA GGCATTAACC CAAGTTTCTC 4380
   TTCTCTGGGT CCCTTTCTTA AGGTGGGAGG GCCTAAAGAT GACTTCCTTT GTCCTGAAGA 4440
   CTCTCCGAGC CCATGGATCT GCACTCTCTA ATATGAAATA TATTGCATAA AATGTCTGGC 4500
   CTCAGTTTCC CCACCTGTCA GGTTTAGGCA GCACAGTCGG TCCAAGACAC TTCATTATTT 4560
   GCAGGCAGTA TAAGAAGAAG CTCCCATCCC CCACCCGCTT CCTCCGGTCC CTAAGACAGA 4620
   ATACTTCTAC ACTGAAACTG AACTCTCGCA GACGCATATG CTCACTTTAA TGATGATGAA 4680
   ATAATGGGGA AACTGAGGCT CCGAGAGATT CCTGGAGGAA GAGGGTCAAA ACCAGCTCCA 4740
   GGAAGCTCTC CAGCCCCCAT CCGGGCCTCT CCAGGTTCTG GGCTTGGCGG GAGTGAACAC 4800
   AGCTGGGAGG GGCTGGAGCC TGGGAGCTTT GGCCCTTGCT CGTGCCCAGC ACCTGCGATT 4860
   CTTGCACGGG AGCCAGCAGG CGGCTGCGTC CGCCCGAGAG ACTGAAGAAG CCGGGGGTAG 4920
   GGTTGGAGGG AGGTAAGCAG GGGCTGTGGG GGCCGAAGCT TGTGCCAGGG CCTGTCAGCG 4980
   AGTCCCCAGT TTTATTTATG GCGTGAGGCC GATGTCCTTA TCCGCTGGCC TGCTGGGGGA 5040
   TGGCTGCGGC TGGGGATTGG ACCCAAGGGC TGGCTTCCCA CTCAGTCCTC CAGCCCACTC 5100
   CATGTCACAC CCGTGCATTC TCTGAGGCTT ATCTTGGGAA CCCGCCCTTG TTCTGTGCTG 5160
   TCTGTCTCTA TTTCTGTCAT TCACTTTCCC AGAGCCTTTT TTTTATGCTT TTAATATAAC 5220
   TACGTTTTAA AAATTGCTTT TGTATAATGT GTGTGCCTTC GTGAGCGTGC GTGCCACAAC 5280
   ACACACGTGA AGGTTAGAGA ACTTTGTTGA GTAGGCTCCT TCCACCATGT GGGACTAGGG 5340
   CTGGCGACAA GAGCAATTAC TGAGTCATCT CGCCAGCCCC TCACCCCTCA CTTCCCATCC 5400
   TGTTTGGATA GTCATAGGTA ATCGAAGGTA AATCGCTGGC TTTAATTTCG TAGCTATCCT 5460
   GCCTCAGCCT ACCAAGTGCT GTGCTACCAC GTTTGTGGGA GGGGCTCTCC TCCCAGTGTC 5520
   TGGGGGTGAC ACAGTCCCAA GATCTCTGCT TTCTAGGTCT TTGTCTTAGT TTGCCCCTTG 5580
   CTTTGTCCGT GTCCCTAGAG TCTCCGGCCC CACTTATCCA TTGACTGGTC TTTCCTTTAC 5640
   CGAATACTCG GTTTTACCTC CCACTGATTT GACTCCCTCC TTTGCTTGTC TCCATCGCCG 5700
   TGGCATTGCC ATTCCTCTGG GTGACTCTGG GTCCACACCT GACACCTTTC CCAACTTTCC 5760
   CCAGCCGAAG CTGGTCTGGT ATGGGAGGCC GCCGTCCCGC GCGCGCCTCC TGCTGGCCGC 5820
   GCCCCAACAC TGCCGCTCCA TTCTCTTTAG AGCGCCCGGG CCCGGGCGGC GGGGTGTGCG 5880
   AGCCGCGGGG CGGCGAGCCC AGCTCGGGCC CGGTGCGGCG CGAGCTCAAG CAGTTCCTCG 5940
   GCTGGCTCAA GAAGCACGCA TACTGCTCGA ACCTTAGTTT CCGCCTGTAC GACCAGTGGC 6000
   GTGCTTGGAT GCAGAAGTCA CACAAGACCC GAAACCAGGT AGGAAAGTTG GGGGAGGCTT 6060
   GCGTGGGGGG TAAAGGAGCA GAGGAAGAGA GAGACCCGGG TGAGCAGCCT CCACAACACC 6120
   GCACTCTTCT TTCCAAGCAC AGGACGAGGG GATCCTGCCC TCGGGCAGAC GGGGTGCGGC 6180
   GAGAGGTAAG GGGGTCTGGG TGAGTGGGGC CTACAGCAGT CTAGATGAGG CCCTTTCCCC 6240
   TCCTTCGGTG TTGCTCAAAG GGATCTCTTA GTGCTCATTT CACCCACTGC AAAGAGCCCC 6300
   AGGTTTTACT GCATCATCAA GTTGCTGAAG GGTCCAGGCT TAATGTGGCC TCTTTTCTGC 6360
   CCTCAGGTCC TGCCGGCTAA ACTCTAAGGA TAGGCCATCC TCCTGCTGGG TCAGACCTGG 6420
   AGGCTCACCT GAATTGGAGC CCCTCTGTAC CATCTGGGCA ACAAAGAAAC CTACCAGAGG 6480
   CTGGGCACAA TGAGCTCCCA CAACCACAGC TTTGGTCCAC ATGATGGTCA CACTTGGATA 6540
   TACCCCAGTG TGGGTAGGGT TGGGGTATTG CAGGGCCTCC CAAGAGTCTC TTTAAATAAA 6600
   TAAAGGAGTT GTTCAGGTCC CGATGGCCAG TGTGTTTGGG GCCTATGTGC TGGGGTGGGG 6660
   GGA 6663
(2) INFORMATION FOR SEQ ID NO:29:
   (i) SEQUENCE CHARACTERISTICS:
      (A) LENGTH: 186 amino acids
      (B) TYPE: amino acid
      (D) TOPOLOGY: linear
   (ii) MOLECULE TYPE: protein
   (xi) SEQUENCE DESCRIPTION: SEQ ID NO:29:
   Asp Pro Thr Leu Leu Ile Gly Ser Ser Leu Gln Ala Thr Cys Ser Ile
   1 5 10 15
   His Gly Asp Thr Pro Gly Ala Thr Ala Glu Gly Leu Tyr Trp Thr Phe
   20 25 30
   Asn Gly Arg Arg Leu Pro Ser Glu Leu Ser Arg Leu Leu Asn Thr Ser
   35 40 45
   Thr Leu Ala Leu Ala Leu Ala Asn Leu Asn Gly Ser Arg Gln Gln Ser
   50 55 60
   Gly Asp Asn Leu Val Cys His Ala Arg Asp Gly Ser Ile Leu Ala Gly
   65 70 75 80
   Ser Cys Leu Tyr Val Gly Leu Pro Pro Glu Lys Pro Phe Asn Ile Ser
   85 90 95
   Cys Trp Ser Arg Asn Met Lys Asp Leu Thr Cys Arg Trp Thr Pro Gly
   100 105 110
   Ala His Gly Glu Thr Phe Leu His Thr Asn Tyr Ser Leu Lys Tyr Lys
   115 120 125
   Leu Arg Leu Val Arg Ser Gly * His Met * Gly Val Pro His Cys
   130 135 140
   Gly Pro Ser Leu Met Pro Tyr Pro Gln Gly Pro Gly Pro Leu His Ser
   145 150 155 160
   Leu * Asp Leu Gly Gly Ser His Gln Ser Pro Arg Leu Ser Lys Ile
   165 170 175
   * Cys Pro His Thr Gly Cys Pro Gly Arg
   180 185
(2) INFORMATION FOR SEQ ID NO:30:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:30:
      AGCTGGCGCG CCTCCCGGGC GGATCGGGAG CCCAC 35
(2) INFORMATION FOR SEQ ID NO:31:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:31:
      AGCTACGCGT TTAGAGTTTA GCCGGCAG 28
(2) INFORMATION FOR SEQ ID NO:32:
   (i) SEQUENCE CHARACTERISTICS:
      (A) LENGTH: 30 amino acids
      (B) TYPE : amino acid
      (C) STRANDEDNESS: single
      (D) TOPOLOGY: linear
   (ii) MOLECULE TYPE: DNA
   (xi) SEQUENCE DESCRIPTION: SEQ ID NO:32:
   Met Val Leu Ala Ser Ser Thr Thr Ser Ile His Thr Met Leu Leu Leu
   1 5 10 15
   Leu Leu Met Leu Phe His Leu Gly Leu Gln Ala Ser Ile Ser
   20 25 30
(2) INFORMATION FOR SEQ ID NO:33:
   (i) SEQUENCE CHARACTERISTICS:
      (A) LENGTH: 30 amino acids
      (B) TYPE: amino acid
      (C) STRANDEDNESS : single
      (D) TOPOLOGY: linear
   (ii) MOLECULE TYPE: DNA
   (xi) SEQUENCE DESCRIPTION: SEQ ID NO:33:
(2) INFORMATION FOR SEQ ID NO:34:
   (i) SEQUENCE CHARACTERISTICS:
      (A) LENGTH: 73 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:34:
   GATCTTGCCC TCGGGCAGAC GGGGTGCGGC GAGAGGTCCT GCCGGCGACT ACAAGGACGA 60
   CGATGACAAG TAG 73
(2) INFORMATION FOR SEQ ID NO:35:
   (i) SEQUENCE CHARACTERISTICS:
      (A) LENGTH: 73 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:35:
   AACGGGAGCC CGTCTGCCCC ACGCCGCTCT CCAGGACGGC CGCTGATGTT CCTGCTGCTA 60
   CTGTTCATCC TAG 73
(2) INFORMATION FOR SEQ ID NO:36:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:36:
      CCCACGCTTC TCATCGGATT CTCCCTG 27
(2) INFORMATION FOR SEQ ID NO:37:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:37:
      CAGTCCACAC TGTCCTCCAC TCGGTAG 27
(2) INFORMATION FOR SEQ ID NO:38:
   (i) SEQUENCE CHARACTERISTICS:
      (A) LENGTH: 11832 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:38:
   GCGGCCGCTG CAGTGATTAC TCACCGCGTG GCGCACCCCA CCCGCGGGCC GCTGAGTGGA 60
   TTTTTCCGTG GGGGGATGTG AAGAAGTTTA GGGAGAACTC TTCTGCACCG ATGGGAACTA 120
   GGAATGCAGG GTTCGGTCCC GTTCCCCAAA GGACACACCT CTCCCCATAA GCCCACTCAT 180
   AAGGGCTCCC TGCACGCGCT CCGGGACATC CCCATATCCA ATACCCGCAG ATATGATAGT 240
   TGAGAAGGGA CCAGAGGCCG GAGACTCCCT CCCTGCCTTC TGGCTTTCCC CCCCCCCTGC 300
   ACGAAACGAG ACTACAGCGA TGGGAGAGGT GGCATGAAGG CTTAGGGTGG GGATCGGTAG 360
   GACCCATGCA CCCAGAGAAA GGGACTGGTG GCAACTTTCA AACTCTCTGG GGAAGGAAGA 420
   AGGGCTGAAA GAGGATGAAC GGGCTCAGGT ACTGCTCAAT GTGTGTGTGG CGGACCAAAG 480
   TGGGTATGGG GGCCCCGTAA GAGGGGCGGG GAAGGTGGAT AGGAAGGATC CCGGTAGACT 540
   GGAGGGGATC CTGGAAAAGC ACCAGGGCTG CGAGCTAGGA ACCCATTCGG AGTTAAGGGT 600
   ACAGGATCCC AGATGAGGGG GTGGGAAGCC TGGGACGGGC GGGACCAGAG AGGGAGGTCC 660
   CACGGGCTGG TGGGGAAAGA GTGGGGGGCT TCGCGCAGGA GGATGGGACG TTCAGGAGTG 720
   GTAACTGGGC GGAGGCCGGC CGGGCGGGGC GCGCGGTGCC CGCGGGCGGT GGGAAGGCCG 780
   GTGCGGGGCC CACGATCAAC CCCCCCCCAG GGGCCGGGCC GGGCCGGGGG CGGGGCCGGG 840
   CGGGGCGAGC GGCGCATTAG CGCCTTGTCA ATTTCGGCTG CTCAGACTTG CTCCGGCCTT 900
   CGCTGTCCGC GCCCAGTGAC GCGCGTGAGG ACCCGAGCCC CAATCTGCAC CCCGCAGACT 960
   CGCCCCCGCC CCATACCGGC GTTGCAGTCA CCGCCCGTTG CGCGCCACCC CCATGCCCGC 1020
   GGGTCGCCCG GGCCCCGTCG CCCAATCCGC GCGGCGGCCG CCGCGGCCGC TGTCCTCGCT 1080
   GTGGTCGCCT CTGTTGCTCT GTGTCCTCGG GGTGCCTCGG GGCGGATCGG GAGCCCGTGA 1140
   GTACCGTGCG CCCTGCTCCC CACCTCCCCA GGGAAGCCGG GATCCGGCGC CCCGGGGGGT 1200
   AGTCGCGGGG GATGGAAGAA GGGGCGCGAG CGCCACCTGG ACGTCCCGGG AACAAAGGAA 1260
   GGCGGCCCTC GGGGCGCCCT CACCTGTGGG GCTCATGGCA CCACCACCCA GCCTCCCAAG 1320
   AGTACCCCGT TATACATCAG AGGCCTCTTA TCTGTATCCC CTTTGCGAGG CTGTCTGGCC 1380
   AGGCTCAGTT TGAAGGACAT CGCAGTGTCC TGGGACCCCC CTCCTTCAGG GTGCTGGGAC 1440
   GCTTCGGGGC GCACGCCTGT GTCTTGGATA TCAGAGCGGA AGGGAAGCCT CCCTGGCCGG 1500
   GGGCGCACGC TTGGGTGCGT TGGGTTGGGT GCTGGCGCAA AGTGGGGTCC CCTCCCCCAT 1560
   GAAGTGATGA TCCCCGGGGG GAGGGTGGGG CGTTATCGTG AGCCCTCCTG TCCGCCTGGC 1620
   ATGCGGCCCG GCGTCCCTCG GGACTTGCCT CTCCGTGGGG TCGGCGCCGC CCCCTCCCCC 1680
   CTATAGCAGA CTCCATGCTT TGGTATCCTC GAAGTCCTCT CCACTGGTGG GGCTCACAAC 1740
   CGGTCTCATT CAGGCTGCGC TGGGTTGAGA GCCTCTAGCG ACTGAAATTT CGGTGAGGAG 1800
   CGAGAGCAAG CGTGTCCGGG CACCGCGAGC CCAGACTTCA TTGTCTAAGG GGCACCCAGT 1860
   GGGGGTCAGC TGCCGAGAGA ATCCCACTGT CCCAGGAGGA ACTCCTGGCC TTGAGCCCCC 1920
   ATCACCCAAC GCACACATCC CCGCCAGGAT GCGGTCTCCA CATCCAGACC CTCTCTGGGA 1980
   CACACCCAAA GACACACAAA AGAGCCCCAC TGGCTTATGT CCCGTCACCC TGCCCTCCGA 2040
   CGCGCGCTGC AGCCCAGATG CGTATTCGCA CACCATCGCG GCGCTCGCAT TCCATCCTCT 2100
   ACACACACAC ACACACACAC ACACACACAC ACACACACAC ACACACAGAC ACGCACACAC 2160
   ACACGCACGC ACACACACGC ACGCCCGCAC TCGTGGTCCC ACATTTATTT CACAGGGGAG 2220
   GCAACACCGG GGTACGCATA TGGTTGAGTG CACTGGAGAT CTTTCCCCAC CACTCTCAGG 2280
   ACCCCATCCG GAGACACAGG CCACACCGCA GGGGCACCAC GCTGCGCTGC TGCTCTGGGC 2340
   TAGTAGTCTT GTGCAGTTTG TCCGCGGTGT CTGTGGACGC CCTCCCGCTC TTGTCAGGGG 2400
   ACAGGAACCT ACACTCCTGC TTGCCCAAGG CGGCTGGGCA GGTGATGTGG TGACACCCGG 2460
   GACCTTTCCG GGGAGTTGGT GTTGCTGCCA AGCCTGGGTA GTTTTTGAAT GCCACCAATA 2520
   GCGCTAAGCT TTGTTTCCGG GCGGGCTGCA GAGCAACAGG CGAAGGTGGC GGAGTGGGGG 2580
   TGGCGCGTGT GTTTTTTCTT TTAAGGGGGA GAGAAATTAA ATAAGAGGTT CTCACACCTC 2640
   TGCAATCTGT TTGTACTTAC CGTGTGTCTT AACACCTGAC CAGCCAGCCG GTGGGTCGTA 2700
   AAAGTGTATG CAGGTACCAG CGGGACAGGA GATGGGGGCC CCTGGGGTAT GGCTGGGATG 2760
   GAGGCCACCT TCCCGTTGGC CTTTCAGGGA ATCTCACACT TTTCCCTTTT AAAACACATG 2820
   GTGTTCTTTT TAATAACGGC AGCAACTCCG CATTGGGAAA GGGGGAAATA AGCTTGTATA 2880
   GGCCCCGGCT TTGTGGAAAG GAGGGGAAGA GGGAAGAAAA AAGGAGGGGT GTCTCCTCCA 2940
   GGCTTAGGGG GCTGTCAGCT GCTGCTCTGT CTAGCTTGGC ATGTGTGTGC CCCAGTCCCC 3000
   AGTGGCTTTG GCCCATTGTT TGTGGAAGCC AAGAGGGAGA CTGGAGTCCT CTATCTCTGG 3060
   TACTCCAGAG TCAGGCTTCT CAGTCCGAGC CCAGAGAACG TCTTCCCTGT TTTATGGAGG 3120
   GAATCAGGGA AGGGGGTGCC AGGTGGACTA CGTTCTGCTG AGGACTGTAC CAGTCGCTCG 3180
   AAGGAGAAAG CTTGGGCTTG CCCCCCTCCC CCCTCAAGCC ACGAAGGGCA GCTGCTAGGC 3240
   TAGTGTGGTA AAAGGGCATT ACTCCCCAGC CAGGACCCCC CAGAGAGTCC CCTTCCTGGC 3300
   CAGACAAATG CTGGGGAGGG ACAGAGGGGT GTGATCATTG CCCAGGAGTG CAGACAGTGG 3360
   GGTCCCGGGT CGGGCAGTGC CTCCCACCCT GCTGAGGGGG GCGCCCAGGC AGGAAGCGGT 3420
   GGGTGGGCCG GGGTAGAGAC GCTGGCACGT CCCAGTTCAT GCCGAAGGAA TTCTGAATTA 3480
   GCGGGCGGCT GGCTGCCTGG GACCTCCGGG GCGGCCCCCT GGCCCCCGCC GCTCCGTCTG 3540
   GCCTGCTCCT CCTGCTCCTT CGCACGGACG CTGAGACCTC CGCTGAGCCC TGGGACAAGC 3600
   CCCAAATGCA ACTGCGATTG CAGGCTTCGC AAGACCCGCC TCCTCCCAAG GCCAAATTTG 3660
   CCTGGGAGAA GTCATTCAGG GCCCAGACTA GAACCATGTT GGTGCCACCT CATCCATCTG 3720
   GGGCATGAAG GACCGTCCAG GGCTGCAGTT TAGCTTCTTA ATAGGAACCT GGGGGTGGGT 3780
   GCAGCCTCTG TTCTCCGAGC CTCTTTGGAA ATCGGTTTTG TTTTTGTTTT TGTTTTTTCC 3840
   AATACTCTTT TCCTCTCATC CCATCCCGGG ACTGTTTTCC TCCCTAAGGG TTGAGAGCCC 3900
   TGCAGTCTTC CCTAACCTTT TCTTTGCTTC TACCCCAGGG CCTTTGCACA TGGAGTCCCA 3960
   CCTCTCCCCT TGCCCAACTG GGGCTCCAGC CTTACTGCAT TTGGCTCTTG GTAACTGTCC 4020
   CAGGGCCTCT CTGACACACA GGGTTGTAGC CCCAGCTCCC TCTCTTCTCC TCCCCCCTTT 4080
   CTCTTTTGCT TCTGAGACTT AATTTTTTTC TTTTTCTTTT TGGCTTTTTG AGACAGGGTT 4140
   TCTCTGTACA GCCCTGGCTG CCCTGGCACT CATTCTGTAG ACCAGGCTAG CCTCAAACTC 4200
   ACAAACCTAC CTGCCTCTGC CTTTCCAGTG CTGGCACTAA AGATGTGGGC CACCACAACT 4260
   AGTAGTTAAG TGTTTTGCTG TGTCTTTATT CCTATAGTGA CCTCAGTTCC TGGCATATTG 4320
   TAGGCGATGG ATGGATGAAT GGATGGATGG ATGGATGGAT GGATGGTTGG ATGGAGCAAG 4380
   CTTGAATCGT CCTGAGTGAA AAAAGAGACC TCAGAGAACT GAATGGAGTT AGGTTCCCAG 4440
   GGCAGCCTGG CCTGCTGGTC TCATGGGAGC TCCCTGTGAA ACTTCCCCCA CACCTCCCAC 4500
   CACCCTGCCA TCCTGTGTGG CTGACAAGAA AGGCCAATGG CCAGATGGGG ACACAGACTC 4560
   AGGGAAGCTT GGAATATGTT CCCCTCCTCA TATCCTAGGC CTTGTTGTCC CCCTGAGGGC 4620
   CCAGCCTATG AGTAGGGCAG CTGTGGGCTG CCCTAAGGTT GGGTAGGCAA GAAGGGGGTG 4680
   GTCCCTCAGG GTGGGTCACA GGATTGAGGT CATTTCCAAA GTGGCCATCA CAGTGGCCCT 4740
   AGGAAATGAT TGTGGAGAGT CAGAACTCCT GTTGGGAGTT GTAGAGGGCC TTGCATGTGG 4800
   GCTTCTGTGG CTGTCCCTTC TCTTGTGGTC CTTTGCACAG TCCCCTCGTG TGTGCTGGGA 4860
   TGTGAGGAGG GCACGGGGAA AATGAAGGCT CAGCCCCTCA GCTTGCCCTT CACGGTTCAC 4920
   CCAACAGGGC TCACCTCTCC TCTGGACAGG CTCTCACTGT ATGCACAGAT TGGCCTCACA 4980
   TTTGATTCCC TTCCTTTGGT CTCCTGGGAT GACAAACATT TACCAGGGTA GGATTTTACA 5040
   TTTTAGATAT GTCCATTCTC CAGAAACACA CTTGTGAGGT TAGGGTATCA GTGAAAGGAC 5100
   ACCACCAGGA CAGACAAAGA ATTGGAGAGG AAGGAAATTG GTAAGCCAGG CCATGCTTGA 5160
   TGGCTTATGT GTAATCCCAG AACTCTGGAC GCTGAGGCAG GAGGATTCCA AGTTTCAAGA 5220
   CAGTGTGTTC TAGGTAATGA GACCCTGTCA AGAAAAGAAA AGAAATAAAG AGACAAGAAA 5280
   ATGTTTATAG GCTGTGAGAC AGCTTGGTGG GTAAGGGGCA CTTGCCTCCA ATCAAGATGA 5340
   CCTCAGCCCC ATCCCTAGGA ATCCATGGTA GAAGGAGAAA GCAAACTCCA GCTGCTGACC 5400
   TCCATACATG TGCTCCAATG TGCACACACA CAGGGAGACA TAATCAATTA ATAGGATGTA 5460
   TTTGCTTAGA TTTGAGTAGG CATTTATGAC TGATGTTTTA AAATTTTTAT TTGATTTTAT 5520
   GAAAATATAC CTGTTTGTAT TTGGTTTGGT TTGGTTTGAG TTTTGTTTAT TTGAGACAGG 5580
   GCTTCTCTGT GTAGTCCTGG CTGTCCTTGG AACTCACTCT GTAGACCAGG CTGGCCTTGA 5640
   ACTCAGAAAT CCGCCTGCTT GTGCTTCCCA AGTGCTTAGA TTAAAGGTGT GCACTGCCAT 5700
   TCAGCAAAAT TGCATACTTT AACCCCAGTA TTTGGGAGGC AGAGGCAGAC TAATGTGTGA 5760
   ATTCCAGGCT AGCCAAGGAT ACAGAGTGAG ACCCTATTCT TACCCTCCCC CCCCAAAACC 5820
   CCAAAATGTA TTTTGTGCTT GTGTATGTAC ATGTGTGTTG CAGCACGTAA ATGTCCAAGG 5880
   ACAACTTGTA GAAGTTCTCT CCGTTCACAG TCTAAGTCCT GAATTCAAAC TAAGGTCCTC 5940
   AGGCTTAGCC ACAGTCTTCT TTATGTACTG AGCCATTTCA CTGGCCCTGG ATTGACTGAT 6000
   GAATTAATTT TTGAGATAAG GTCTCTTGTA GCTCTAGCTA GGCTCAAACT ATGAACTCCC 6060
   AAGGTCATCT TGAGCTGCTG GTACTCTTGC TTCCACCCCA AGTGGTGGAA TGATACTCAG 6120
   GCAGCACTTC TCTGGGGAAG GGGCTGGCCT TGGCCTTGAT TTTGTTGCCT CAGCTTCAAT 6180
   GAGTGCTTGG GTCTCGTTGT TTCTTTTCTT TATCTGTGAA ATGGGTGAAC ACCTGTTCAA 6240
   GACTTCCTGA CTCTTGAAAC ATCCAGGCAG GGTGAGGGAC TTGAAGTGGG CTCATCCCAT 6300
   GCCTAACAAA GTGTCGTCTT TGACCCCAGA CACAGCTGTA ATCAGCCCCC AGGACCCCAC 6360
   CCTTCTCATC GGCTCCTCCC TGCAAGCTAC CTGCTCTATA CATGGAGACA CACCTGGGGC 6420
   CACCGCTGAG GGGCTCTACT GGACCTTCAA TGGTCGCCGC CTGCCCTCTG AGCTGTCCCG 6480
   CCTCCTTAAC ACCTCCACCC TGGCCCTGGC CCTGGCTAAC CTTAATGGGT CCAGGCAGCA 6540
   GTCAGGAGAC AATCTGGTGT GTCACGCCCG AGACGGCAGC ATTCTGGCTG GCTCCTGCCT 6600
   CTATGTTGGC TGTAAGTGGG GCCCCAGACA CTCAGAGATA GATGGGGGTT GGCAATGACA 6660
   GATTTAGAGC CTGGGTCTTC TGTCCTGGGG CAGAGCCATG GGCTCTCACT TGCATGCAGG 6720
   CATGGTCATA CCCAGCACAG GCATTGCAAC TCTAGGGACA GCTGTGGCTG CACTGTCCCC 6780
   TGTGTACCCC ACAGCTTTAG AAAAGCTGTC ATGTTTTCCT TGTAGTGCCC CCTGAGAAGC 6840
   CCTTTAACAT CAGCTGCTGG TCCCGGAACA TGAAGGATCT CACGTGCCGC TGGACACCGG 6900
   GTGCACACGG GGAGACATTC TTACATACCA ACTACTCCCT CAAGTACAAG CTGAGGTTGG 6960
   TACCCAGCCA AGCCTTGCTG TGTGACTTCT GGCAATACTT ACCTTCTCTG ATCAAATATG 7020
   TTCCTGTTTA TGAACTCAAA AGGGACTCTC GCACCTCCAC AGGTGGTACG GTCAGGATAA 7080
   CACATGTGAG GAGTACCACA CTGTGGGCCC TCACTCATGC CATATCCCCA AGGACCTGGC 7140
   CCTCTTCACT CCCTATGAGA TCTGGGTGGA AGCCACCAAT CGCCTAGGCT CAGCAAGATC 7200
   TGATGTCCTC ACACTGGATG TCCTGGACGT GGGTGAGCCC CCAGTGTCCA CCTGTGTTCT 7260
   GCCCTAGACC TTATAGGGCG CCTCCCCCCC ATCCCCCCAG ACTTTTTGGT TCTTCTAGAG 7320
   GTCTTAGCCA CAGCCACGGT GGTTGCAGGA CAGTGGTTGT TCATAACTTA ATGCAAAGAC 7380
   TTTCCCCCAA GACAGTCAAG ATTTTCCCCT CCCCACCCCC AACACACACA TACACACACA 7440
   CTCTGCAGAG AACACCTGGC CTGACCACCC TCCCTCTCTA CAGCCCAGGT GTTCAGAAGG 7500
   GAGTCCTAGG GGACTGAGAG GAGGCGCCCA GGTCTGAAGG CGCCCCAGGA AGCCGAGGCC 7560
   TTGAGCTGGG GGGGGGGGCG AGGGTTGGAG GCACGAACTG GATGATCCCT GAGCACAACT 7620
   GGGCCTAATC TAATTAGGGT GTTCCCAGCC CAAAGCAGCC TGGGCCATTT AACCCTTCAA 7680
   GTGCCTCACT GAAGACTCAG GGGAGAGATC AGCTTGTACT CTCTCCATGG TCCCCCAGGA 7740
   GGGTTCCTGG GTGCCCCTGG CTCATTCCCA CATCCAGAGG TTTTGTGTCT TCCTGGCATC 7800
   TAACCCTCAG TTGTGCTCTG TGGCTGGCAC AGCTGCCCCG TGGAGGCTCT TGGTAATGTA 7860
   CAAGGCATCA GAGGTGGACA TGGGATGGGG ATACATAGGG ATGGAGCCAA ATAGCACCTC 7920
   AAGGTGGGGT GATATACAAT AAAGCTTGTC ACCCTGACGC TCAGAAAGCC TACTCATGAT 7980
   GATCACAATT GTTGACATCA CTCTGGGACA TGTAGTGAGA CCCTAGCTCA AAACACAGAC 8040
   AGTAGCTTTA AGAGTCAGCT TGTGACTTAA TACTGGAACT CAGGGCCTAA TAGGTGCTGG 8100
   GTGATGCTCG CCTCACTCCC TGTTTAGTGA GATCTCTGCG CTAATCTCCA CCCCAGCTGG 8160
   GTGGGCTGCT CTGTCCCCTT GAGGGCAGGA ATGTGTGTCT TCCATCAGAG ATAGGACCCG 8220
   TGGTAGCAGC AACTGCTGCT GGCTGTTTCT GGAATATTAA ATGACAGTAA TCTATCAGGC 8280
   CTGGGTGAGT AGCTAACAGG GGTGGGGGCG TGGTCTGGAA AACGCAGATA GGGTCATAGG 8340
   AGCCACTGCA GCCTAGATTA CACCACTGGG TGTTCTGTCA CTAGGCCATT CTCACCAAGC 8400
   AGTCCTCAGA ACTGGGAGCA CTGTTGCCAG CATTTAATGC CAGCATTTAA TGCCAGCATT 8460
   AGGGGAGGCA GAGGCAGAAG GATCTCTCTG AGTTCAAGGC CATCCTGAAT TTACATAAAG 8520
   AGCTCCAGGC CAGCCAGGGT GCGCAGTAAA ACCTTGTCTC AAAAAACAAA GCATCTTTAG 8580
   TGACCAGGCT TGCTCCACCC CCAGTGACCA CGGACCCCCC ACCCGACGTG CACGTGAGCC 8640
   GCGTTGGGGG CCTGGAGGAC CAGCTGAGTG TGCGCTGGGT CTCACCACCA GCTCTCAAGG 8700
   ATTTCCTCTT CCAAGCCAAG TACCAGATCC GCTACCGCGT GGAGGACAGC GTGGACTGGA 8760
   AGGTGCCCGT CCCGCCCCGG ACCCGCCCCT GACCCCGCCC CCCGCATCTG ACTCCTCCCT 8820
   CACCGTGCAG GTGGTGGATG ACGTCAGCAA CCAGACCTCC TGCCGTCTCG CGGGCCTGAA 8880
   GCCCGGCACC GTTTACTTCG TCCAAGTGCG TTGTAACCCA TTCGGGATCT ATGGGTCGAA 8940
   AAAGGCGGGA ATCTGGAGCG AGTGGAGCCA CCCCACCGCT GCCTCCACCC CTCGAAGTGG 9000
   TGAGCACCTC TCCAGGGCTG GCTGGCCCAT GGAATCCCCA ATCCATCCTG TTCCTTCCCC 9060
   CCCACCCTTT TTTTGAGACA GCGTCTTCAG GTAGCGCATG CTGGCCTTAA ATTCAGTATG 9120
   TAGTCAAGGA TGACCTCGAG CTCCTGGTCT TTTTGTCTCC ACTTAGAGAC AATGGCCAGT 9180
   GGCCATCACC ACCTTTGGGA GACTAGCCAT GGAGTCTATT TAGCCTGTCA TTTGGTGACA 9240
   GATGGAGTAC AACAGTGTGA CCTCTTGTAA GAGAACTGAA GACAGGCTGT TTTTAACCCC 9300
   AATATCCTAG GCTCTCTAGA GGTTAACTTT ATATAAAATA GAGACTATTA CAGCCAGTTA 9360
   TCACATGGTC CCACAGAACC TTTTGTCACA CAACCTATAG ACCACAGTGC CTGTGCCTAC 9420
   CACATAAGGG TCTCTACTGC TGGCCCACCC CTCCAACCCT TAAAAGGTAA CCTAGGCAGC 9480
   CTTAATATTT GCAATCCTCC TACCTCAGCC TCTTGAATGC TCAGAAACCA GGCATTAACC 9540
   CAAGTTTCTC TTCTCTGGGT CCCTTTCTTA AGGTGGGAGG GCCTAAAGAT GACTTCCTTT 9600
   GTCCTGAAGA CTCTCCGAGC CCATGGATCT GCACTCTCTA ATATGAAATA TATTGCATAA 9660
   AATGTCTGGC CTCAGTTTCC CCACCTGTCA GGTTTAGGCA GCACAGTCGG TCCAAGACAC 9720
   TTCATTATTT GCAGGCAGTA TAAGAAGAAG CTCCCATCCC CCACCCGCTT CCTCCGGTCC 9780
   CTAAGACAGA ATACTTCTAC ACTGAAACTG AACTCTCGCA GACGCATATG CTCACTTTAA 9840
   TGATGATGAA ATAATGGGGA AACTGAGGCT CCGAGAGATT CCTGGAGGAA GAGGGTCAAA 9900
   ACCAGCTCCA GGAAGCTCTC CAGCCCCCAT CCGGGCCTCT CCAGGTTCTG GGCTTGGCGG 9960
   GAGTGAACAC AGCTGGGAGG GGCTGGAGCC TGGGAGCTTT GGCCCTTGCT CGTGCCCAGC 10020
   ACCTGCGATT CTTGCACGGG AGCCAGCAGG CGGCTGCGTC CGCCCGAGAG ACTGAAGAAG 10080
   CCGGGGGTAG GGTTGGAGGG AGGTAAGCAG GGGCTGTGGG GGCCGAAGCT TGTGCCAGGG 10140
   CCTGTCAGCG AGTCCCCAGT TTTATTTATG GCGTGAGGCC GATGTCCTTA TCCGCTGGCC 10200
   TGCTGGGGGA TGGCTGCGGC TGGGGATTGG ACCCAAGGGC TGGCTTCCCA CTCAGTCCTC 10260
   CAGCCCACTC CATGTCACAC CCGTGCATTC TCTGAGGCTT ATCTTGGGAA CCCGCCCTTG 10320
   TTCTGTGCTG TCTGTCTCTA TTTCTGTCAT TCACTTTCCC AGAGCCTTTT TTTTATGCTT 10380
   TTAATATAAC TACGTTTTAA AAATTGCTTT TGTATAATGT GTGTGCCTTC GTGAGCGTGC 10440
   GTGCCACAAC ACACACGTGA AGGTTAGAGA ACTTTGTTGA GTAGGCTCCT TCCACCATGT 10500
   GGGACTAGGG CTGGCGACAA GAGCAATTAC TGAGTCATCT CGCCAGCCCC TCACCCCTCA 10560
   CTTCCCATCC TGTTTGGATA GTCATAGGTA ATCGAAGGTA AATCGCTGGC TTTAATTTCG 10620
   TAGCTATCCT GCCTCAGCCT ACCAAGTGCT GTGCTACCAC GTTTGTGGGA GGGGCTCTCC 10680
   TCCCAGTGTC TGGGGGTACA CAGTCCCAAG ATCTCTGCTT TCTAGGTCTT TGTCTTAGTT 10740
   TGCCCCTTGC TTTGTCCGTG TCCCTAGAGT CTCCGGCCCC ACTTAGTCTC CATTGATTTC 10800
   CTTTCTGACC GAATACTCGG TTTTACCTCC CACTGATTTG ACTCCCTCCT TTGCTTGTCT 10860
   CCATCGCCGT GGCATTGCCA TTCCTCTGGG TGACTCTGGG TCCACACCTG ACACCTTTCC 10920
   CAACTTTCCC CAGCCGAAGC TGGTCTGGTA TGGGAGGCCG CCGTCCCGCG CGCGCCTCCT 10980
   GCTGGCCGCG CCCCAACACT GCCGCTCCAT TCTCTTTAGA GCGCCCGGGC CCGGGCGGCG 11040
   GGGTGTGCGA GCCGCGGGGC GGCGAGCCCA GCTCGGGCCC GGTGCGGCGC GAGCTCAAGC 11100
   AGTTCCTCGG CTGGCTCAAG AAGCACGCAT ACTGCTCGAA CCTTAGTTTC CGCCTGTACG 11160
   ACCAGTGGCG TGCTTGGATG CAGAAGTCAC ACAAGACCCG AAACCAGGTA GGAAAGTTGG 11220
   GGGAGGCTTG CGTGGGGGGT AAAGGAGCAG AGGAAGAGAG AGACCCGGGT GAGCAGCCTC 11280
   CACAACACCG CACTCTTCTT TCCAAGCACA GGACGAGGGG ATCCTGCCCT CGGGCAGACG 11340
   GGGTGCGGCG AGAGGTAAGG GGGTCTGGGT GAGTGGGGCC TACAGCAGTC TAGATGAGGC 11400
   CCTTTCCCCT CCTTCGGTGT TGCTCAAAGG GATCTCTTAG TGCTCATTTC ACCCACTGCA 11460
   AAGAGCCCCA GGTTTTACTG CATCATCAAG TTGCTGAAGG GTCCAGGCTT AATGTGGCCT 11520
   CTTTTCTGCC CTCAGGTCCT GCCGGCTAAA CTCTAAGGAT AGGCCATCCT CCTGCTGGGT 11580
   CAGACCTGGA GGCTCACCTG AATTGGAGCC CCTCTGTACC ATCTGGGCAA CAAAGAAACC 11640
   TACCAGAGGC TGGGCACAAT GAGCTCCCAC AACCACAGCT TTGGTCCACA TGATGGTCAC 11700
   ACTTGGATAT ACCCCAGTGT GGGTAGGGTT GGGGTATTGC AGGGCCTCCC AAGAGTCTCT 11760
   TTAAATAAAT AAAGGAGTTG TTCAGGTCCC GATGGCCAGT GTGTTTGGGG CCTATGTGCT 11820
   GGGGTGGGGG GA 11832
(2) INFORMATION FOR SEQ ID NO:39:
   (i) SEQUENCE CHARACTERISTICS:
      (A) LENGTH: 26 amino acids
      (B) TYPE: amino acids
      (C) STRANDEDNESS: single
      (D) TOPOLOGY: linear
   (ii) MOLECULE TYPE: Protein
   (xi) SEQUENCE DESCRIPTION: SEQ ID NO:39:
   Val Ile Ser Pro Gln Asp Pro Thr Leu Leu Ile Gly Ser Ser Leu Gln Ala Thr Cys Ser
   5 10 15 20
   Ile His Gly Asp Thr Pro
   25